# EUROPEAN PATENT APPLICATION

(11) **EP 4 553 074 A1**
(43) Date of publication of application: **14.05.2025**
(21) Application number: 23835581.2
(22) Date of filing: 05.07.2023
(51) Int. Cl.: C07D 413/14, C07D 263/57, C07D 277/66, C07D 413/12, C07D 417/12, C07D 417/14, C07D 471/04, G02B 5/22, H05B 33/02, H10K 50/10

(54) **AMINE COMPOUND, AND ORGANIC ELECTROLUMINESCENCE ELEMENT, ELECTRONIC EQUIPMENT, AND ELECTRONIC ELEMENT CONTAINING SAME**

(30) Priority: 07.07.2022 JP 2022109646
(71) Applicant: Hodogaya Chemical Co., Ltd., Tokyo, 105-0021 (JP)
(72) Inventor: KASE, Kouki, Tokyo 105-0021 (JP); CHA, Hyun-Wook, Tokyo 105-0021 (JP); HIRAYAMA, Yuta, Tokyo 105-0021 (JP); HAYASHI, Shuichi, Tokyo 105-0021 (JP)
(74) Representative: Müller-Boré & Partner Patentanwälte PartG mbB
(86) International application number: PCT/JP2023/025018
(87) International publication number: WO 2024/010050

(57) **Abstract**

[Object] An object of the present invention is to provide a compound that does not affect a material inside an organic EL device by absorbing light at wavelengths from 400 nm to 410 nm in sunlight and has a high refractive index in a wavelength range from 450 nm to 750 nm in order to prevent the inside of the organic EL device from deteriorating and significantly improve light extraction efficiency.

[Solving Means] The present invention has focused on the fact that an arylamine material has excellent stability in a thin-film state and excellent durability, has designed a specific amine compound having a high refractive index and a benzoazole ring structure, and has used the amine compound as a material for forming a capping layer to obtain an organic EL device having excellent light emission efficiency.

## Description

### Technical Field

The present invention relates to a compound and device suitable for a spontaneous light-emitting electronic device suitable for various display devices, particularly, an organic electroluminescent device (hereinafter, abbreviated as an organic EL device), and more specifically to an amine compound having a benzoazole structure and an organic EL device or electronic apparatus using the amine compound.

### Background Art

In 1987, C.W.Tang et al. (Eastman Kodak Company) have developed a stacked-structure device in which various roles are assigned to the materials and put an organic EL device using an organic material to practical use. They have stacked a phosphor capable of transporting electrons and an organic matter capable of transporting holes and caused their charges to emit light by injecting the charges into a layer of the phosphor, making it possible to achieve high luminance of 1000 cd/m² or more at a voltage of 10 V or less (see Patent Literature 1 and Patent Literature 2).

Many improvements have been made for practical use of the organic EL device until now. Various roles of the stacked structure are further subdivided, and high efficiency and durability have been achieved by a light-emitting element having a bottom emission structure that emits light from the bottom portion in an electroluminescent device in which an anode, a hole injection layer, a hole transport layer, a light-emitting layer, an electron transport layer, an electron injection layer, and a cathode are sequentially provided on a substrate (see, for example, Non-Patent Literature 1).

In recent years, a light-emitting element having a top emission structure in which a metal having a high work function is used as an anode and light is emitted from the upper part has been used. While the area of the light-emitting unit is limited in the bottom emission structure in which light is extracted from the bottom portion having a pixel circuit, the light-emitting element having a top emission structure has an advantage that the light-emitting unit can be made wider because light is extracted from the upper part, which does not block the pixel circuit. In the light-emitting element having a top emission structure, a semi-transparent electrode formed of LiF/Al/Ag (see, for example, Non-Patent Literature 2), Ca/Mg (see, for example, Non-Patent Literature 3), LiF/MgAg, or the like, is used as a cathode.

In such a light-emitting element, when light emitted from the light-emitting layer enters a different film at a certain angle or more, the light is totally reflected at the interface between the light-emitting layer and the different film. For this reason, only part of the emitted light could have been used. In recent years, a light-emitting element in which a "capping layer" having a high refractive index is provided outside a semi-transparent electrode having a low refractive index in order to improve the light extraction efficiency has been proposed (see, for example, Non-Patent Literature 2 and Non-Patent Literature 3).

Regarding the effect of the capping layer in the light-emitting element having a top emission structure, the current efficiency was 38 cd/A in the light-emitting element that includes no capping layer and uses Ir(ppy)₃ as a light-emitting material while the current efficiency was improved to be approximately 1.7 times, i.e., 64 cd/A, in the light-emitting element that uses ZnSe with a film thickness of 60 nm as a capping layer. Further, it has been shown that the maximum transmittance and the maximum efficiency of the semi-transparent electrode and capping layer do not necessarily coincide and the maximum light extraction efficiency is determined by the interference effect (see, for example, Non-Patent Literature 3).

In the past, the use of a metal mask with high definition for forming a capping layer has been proposed, but the metal mask is distorted by heat when used under high-temperature conditions, which reduces the alignment accuracy. Therefore, ZnSe has a high melting point of 1100°C or more (see, for example, Non-Patent Literature 3) and cannot be deposited at an accurate position in the metal mask with high definition, which may affect the light-emitting element itself. Further, a capping layer formed of an inorganic matter is not suitable for use because it affects the light-emitting element even if it is deposited by a sputtering method.

In addition, in the case of using tris(8-hydroxyquinoline)aluminum (hereinafter, abbreviated as Alq₃) as a capping layer for adjusting the refractive index (see, for example, Non-Patent Literature 2), Alq₃ is known as an organic EL material that is generally used as a green light-emitting material or an electron transport material, but causes a problem of reduced color purity and reduced light extraction efficiency in the case of a blue light-emitting element because it has weak absorption around 450 nm used in the blue light-emitting material.

Further, devices prepared using an existing capping layer also has a problem of reduced color purity and reduced light extraction efficiency because light at a wavelength from 400 nm to 410 nm in sunlight passes therethrough and affects the materials inside the device.

In order to improve the device properties of an organic EL device and significantly improve the light extraction efficiency, a material having a high refractive index, a low extinction coefficient, and excellent stability in a thin-film state and durability is desired as a material of a capping layer.

### Citation List

### Patent Literature

Patent Literature 1: Japanese Patent Application Laid-open No. 1996-048656
Patent Literature 2: Japanese Patent No. 3194657
Patent Literature 3: WO 2014009310
Patent Literature 4: WO 2013/038627
Patent Literature 5: WO 2021140896

### Non-Patent Literature

Non-Patent Literature 1: The Japan Society of Applied Physics, proceedings of the ninth workshop, pp. 55-61 (2001)
Non-Patent Literature 2:
   Appl.Phys.Let.,78,544(2001)
Non-Patent Literature 3:
   Appl.Phys.Let.,82,466(2003)
Non-Patent Literature 4: J.Org.Chem.,71,1802(2006)
Non-Patent Literature 5: Chem.Rev.2016,116,12564
Non-Patent Literature 6:
   Tetrahedron,58, (2002),9633
Non-Patent Literature 7:
   Appl.Phys.Lett.,98,083302(2011)

### Disclosure of Invention

### Technical Problem

An object of the present invention is to provide an organic EL device that includes a capping layer formed of a material having (1) a high refractive index, (2) favorable stability in a thin-film state, (3) excellent durability, (4) excellent light resistance, and (5) no absorption in blue, green, and red wavelength regions in order to improve the device properties of the organic EL device and significantly improve the light extraction efficiency.

Examples of the physical properties of the material of the capping layer suitable for the present invention include (1) having a high refractive index, (2) being depositable, (3) being stable in a thin-film state, and (4) having a high glass transition temperature. Further, examples of the physical properties of the device suitable for the present invention include (1) having a high light extraction efficiency, (2) having no reduced color purity, (3) causing light to be transmitted therethrough without change over time, and (4) having a long lifetime. Solution to Problem

In this regard, in order to achieve the above-mentioned object, the present inventors have focused on the fact that an arylamine material has excellent stability in a thin-film state and excellent durability, selected a specific amine compound having a high refractive index and a benzoazole structure as a material having high absorbance at a wavelength from 400 nm to 410 nm in the absorption spectrum of 10⁻⁵ mol/L, prepared an organic EL device using the selected compound as a material for forming a capping layer, and intensively evaluated the properties of the device to complete the present invention.

That is, according to the present invention, there are provided an amine compound represented by the following general formula (A), and an organic EL device or electronic apparatus using the amine compound.
1) An amine compound represented by the following general formula (A). 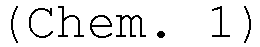 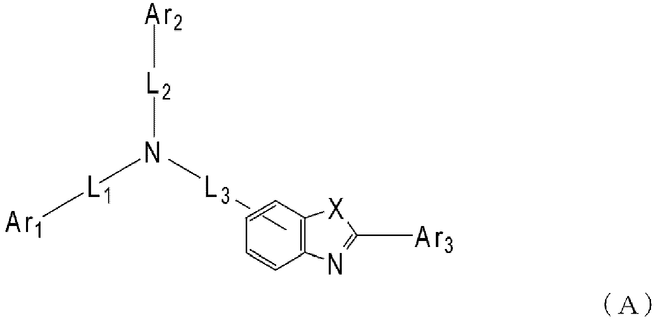 Wherein, Ar₁ represents a substituted or unsubstituted benzoxazolyl group, a substituted or unsubstituted benzothiazolyl group, a substituted or unsubstituted benzofuranyl group, a substituted or unsubstituted benzothienyl group, a substituted or unsubstituted dibenzofuranyl group, or a substituted or unsubstituted dibenzothienyl group, Ar₂ and Ar₃ may be the same as or different from each other and each represent a substituted or unsubstituted aromatic hydrocarbon group, a substituted or unsubstituted aromatic heterocyclic group, or a substituted or unsubstituted fused polycyclic aromatic group, L₁ to L₃ may be the same as or different from each other and each represent a single bond, a divalent group obtained by removing two hydrogen atoms from unsubstituted benzene, a divalent group obtained by removing two hydrogen atoms from unsubstituted biphenyl, or a divalent group obtained by removing two hydrogen atoms from unsubstituted naphthalene, and X represents an oxygen atom or a sulfur atom.
2) The amine compound according to above 1), which is represented by the following general formula (B) or (C). 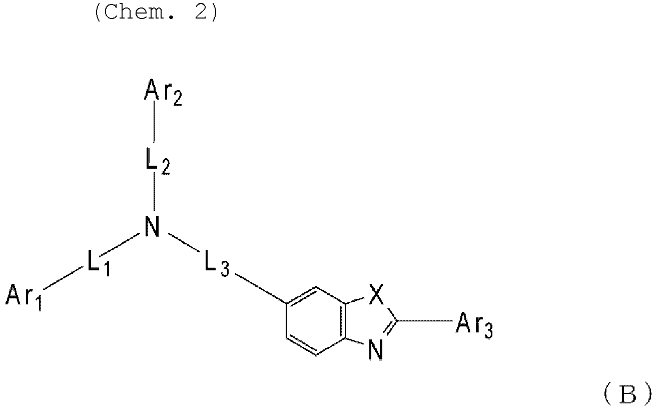 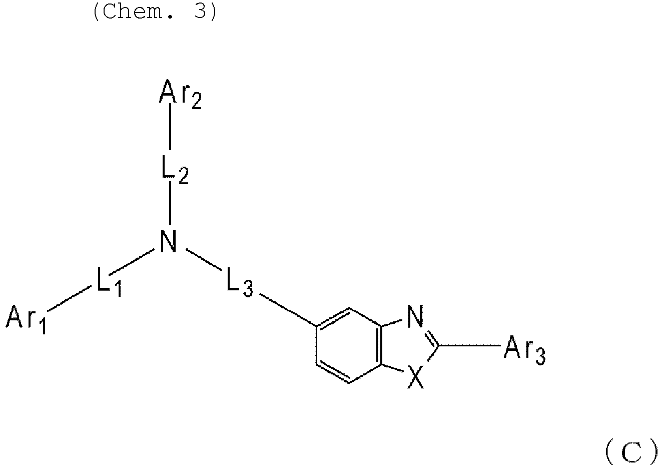 In the general formula (B) or (C), Ar₁ to Ar₃, L₁ to L₃, and X are defined to be the same as those in the general formula (A).
3) The amine compound according to above 2), wherein
   L₁ to L₃ in the general formula (A), (B), or (C) each represent a single bond, an unsubstituted 1,4-phenylene group, an unsubstituted 4,4'-biphenylylene group, an unsubstituted 2,6-naphthylene group, or an unsubstituted 2,7-naphthylene group.
4) The amine compound according to above 3), wherein
   Ar₃ in the general formula (A), (B), or (C) represents a substituted or unsubstituted phenyl group, a substituted or unsubstituted biphenyl group, a substituted or unsubstituted naphthyl group, a substituted or unsubstituted furanyl group, or a substituted or unsubstituted thienyl group.
5) The amine compound according to above 4), wherein
   Ar₃ in the general formula (A), (B), or (C) represents an unsubstituted phenyl group, an unsubstituted biphenyl group, an unsubstituted naphthyl group, an unsubstituted furanyl group, or an unsubstituted thienyl group.
6) The amine compound according to above 3), wherein
   Ar₁ in the general formula (A), (B), or (C) represents a substituted or unsubstituted 2-benzoxazolyl group, a substituted or unsubstituted 2-benzothiazolyl group, a substituted or unsubstituted 2-benzofuranyl group, a substituted or unsubstituted 2-benzothienyl group, a substituted or unsubstituted 2-dibenzofuranyl group, a substituted or unsubstituted 2-dibenzothienyl group, a substituted or unsubstituted 3-dibenzofuranyl group, or a substituted or unsubstituted 3-dibenzothienyl group.
7) The amine compound according to above 6), wherein
   Ar₁ in the general formula (A), (B), or (C) represents an unsubstituted 2-benzoxazolyl group, an unsubstituted 2-benzothiazolyl group, an unsubstituted 2-benzofuranyl group, an unsubstituted 2-benzothienyl group, an unsubstituted 2-dibenzofuranyl group, an unsubstituted 2-dibenzothienyl group, an unsubstituted 3-dibenzofuranyl group, or an unsubstituted 3-dibenzothienyl group.
8) The amine compound according to above 7), wherein
   Ar₃ in the general formula (A), (B), or (C) represents an unsubstituted phenyl group, an unsubstituted biphenyl group, an unsubstituted naphthyl group, an unsubstituted furanyl group, or an unsubstituted thienyl group.
9) An organic EL device, comprising:
   at least an anode electrode, a hole transport layer, a light-emitting layer, an electron transport layer, a cathode electrode, and a capping layer in this order,
   the capping layer including the amine compound according to above 1) or 2).
10) The organic EL device according to above 9), characterized in that
   the capping layer has an extinction coefficient of 0.2 or more in a wavelength range from 400 nm to 410 and absorbance in an absorption spectrum at a concentration of 10⁻⁵ mol/L of 0.2 or more in the wavelength range from 400 nm to 410 nm.
11) The organic EL device according to above 9), characterized in that
   the capping layer has a refractive index of 1.85 or more in a wavelength range of 450 nm to 750 nm of light transmitted through the capping layer.
12) An organic EL device, comprising:
   at least an anode electrode, a hole transport layer, a light-emitting layer, an electron transport layer, a cathode electrode, and a capping layer in this order,
   the capping layer being a stacked layer or a mixed layer formed of two or more types of compounds, at least one of the compounds being the amine compound
   according to above 1) or 2).
13) An electronic apparatus or an electronic device, comprising:
   a pair of electrodes and at least one organic layer sandwiched between the pair of electrodes,
   the organic layer including the compound according to above 1) or 2).

### Advantageous Effects of Invention

The amine compound according to the present invention (1) has a high absorption coefficient, (2) has a high refractive index in the wavelength range of 450 nm to 750 nm, (3) is depositable, (4) is stable in a thin-film state, and (5)has high heat resistance, and using it as a capping layer having a refractive index higher than that of a semi-transparent electrode, which is provided outside a transparent or semi-transparent electrode of the organic EL device, makes it possible to obtain an organic EL device that is capable of significantly improving the light extraction efficiency and prevents the materials inside the device from deteriorating.

### Brief Description of Drawings

[Fig. 1] Fig. 1 is a diagram showing structural formulae of compounds 1 to 12 as examples of the amine compound according to the present invention.
[Fig. 2] Fig. 2 is a diagram showing structural formulae of compounds 13 to 24 as examples of the amine compound according to the present invention.
[Fig. 3] Fig. 3 is a diagram showing structural formulae of compounds 25 to 36 as examples of the amine compound according to the present invention.
[Fig. 4] Fig. 4 is a diagram showing structural formulae of compounds 37 to 48 as examples of the amine compound according to the present invention.
[Fig. 5] Fig. 5 is a diagram showing structural formulae of compounds 49 to 60 as examples of the amine compound according to the present invention.
[Fig. 6] Fig. 6 is a diagram showing structural formulae of compounds 61 to 69 as examples of the amine compound according to the present invention.
[Fig. 7] Fig. 7 is a diagram showing structural formulae of compounds 70 to 79 as examples of the amine compound according to the present invention.
[Fig. 8] Fig. 8 is a diagram showing structural formulae of compounds 80 to 92 as examples of the amine compound according to the present invention.
[Fig. 9] Fig. 9 is a diagram showing structural formulae of compounds 93 to 104 as examples of the amine compound according to the present invention.
[Fig. 10] Fig. 10 is a diagram showing structural formulae of compounds 105 to 116 as examples of the amine compound according to the present invention.
[Fig. 11] Fig. 11 is a diagram showing structural formulae of compounds 117 to 128 as examples of the amine compound according to the present invention.
[Fig. 12] Fig. 12 is a diagram showing structural formulae of compounds 129 to 136 as examples of the amine compound according to the present invention.
[Fig. 13] Fig. 13 is a diagram showing structural formulae of compounds 137 to 144 as examples of the amine compound according to the present invention.
[Fig. 14] Fig. 14 is a diagram showing structural formulae of compounds 145 to 152 as examples of the amine compound according to the present invention.
[Fig. 15] Fig. 15 is a diagram showing structural formulae of compounds 153 to 160 as examples of the amine compound according to the present invention.
[Fig. 16] Fig. 16 is a diagram showing structural formulae of compounds 161 to 168 as examples of the amine compound according to the present invention.
[Fig. 17] Fig. 17 is a diagram showing structural formulae of compounds 169 to 178 as examples of the amine compound according to the present invention.
[Fig. 18] Fig. 18 is a diagram showing structural formulae of compounds 179 to 188 as examples of the amine compound according to the present invention.
[Fig. 19] Fig. 19 is a diagram showing structural formulae of compounds 189 to 200 as examples of the amine compound according to the present invention.
[Fig. 20] Fig. 20 is a diagram showing structural formulae of compounds 201 to 208 as examples of the amine compound according to the present invention.
[Fig. 21] Fig. 21 is a diagram showing an example of a configuration of the organic EL device according to the present invention.

### Mode(s) for Carrying Out the Invention

The amine compound according to the present invention is a novel compound represented by the general formula (A), (B), or (C), and benzoazole derivatives that are the main skeletons of these compounds can be synthesized by known methods (see, for example, Patent Literature 4).

Further, the amine compound according to the present invention can be synthesized by subjecting the synthesized halogenated benzoazole derivative and arylamine to a coupling reaction using a copper catalyst, a palladium catalyst, or the like. In addition, the amine compound according to the present invention can be similarly synthesized by obtaining a boronic acid ester derivative from the halogenated benzoazole derivative and then subjecting the obtained derivative to a coupling reaction with halogenated arylamine (see, for example, Non-Patent Literature 5 and Non-Patent Literature 6).

In the amine compound according to the present invention, Ar₂ and Ar₃ in the general formula (A), (B), or (C) may be the same as or different from each other and each represent a substituted or unsubstituted aromatic hydrocarbon group, a substituted or unsubstituted aromatic heterocyclic group, or a substituted or unsubstituted fused polycyclic aromatic group.

In the amine compound according to the present invention, the "aromatic hydrocarbon group", "aromatic heterocyclic group", or "fused polycyclic aromatic group" in the "substituted or unsubstituted aromatic hydrocarbon group", "substituted or unsubstituted aromatic heterocyclic group", or "substituted or unsubstituted fused polycyclic aromatic group" represented by Ar₂ and Ar₃ in the general formula (A), (B), or (C) is specifically selected from the group consisting of an aryl group having 6 to 30 carbon atoms and a heteroaryl group having 2 to 20 carbon atoms in addition to a phenyl group, a biphenylyl group, a terphenylyl group, a naphthyl group, an anthracenyl group, a phenanthrenyl group, a fluorenyl group, a spirobifluorenyl group, an indenyl group, a pyrenyl group, a perylenyl group, a fluoranethenyl group, a fluoranethenyl group, a triphenylenyl group, a pyridyl group, a pyrimidinyl group, a triazinyl group, a furil group, a pyrrolyl group, a thienyl group, a quinolyl group, an isoquinolyl group, a benzofuranyl group, a benzothienyl group, an indolyl group, a carbazolyl group, an imidazopyridyl group, a benzoxazolyl group, a benzothiazolyl group, a quinoxalinyl group, a benzimidazolyl group, a pyrazolyl group, a dibenzofuranyl group, a dibenzothienyl group, a naphthyridinyl group, a phenanthrolinyl group, an acridinyl group, a carbolinyl group, and the like.

In the amine compound according to the present invention, specific examples of the "substituent group" in the "substituted aromatic hydrocarbon group", "substituted aromatic heterocyclic group" or "substituted fused polycyclic aromatic group" represented by Ar₂ and Ar₃ in the general formula (A), (B), or (C) include an aryl group having 6 to 30 carbon atoms and a heteroaryl group having 2 to 20 carbon atoms in addition to a deuterium atom, a cyano group, a nitro group; a halogen atom such as a fluorine atom, a chlorine atom, a bromine atom, and an iodine atom; a silyl group such as a trimethylsilyl group and a triphenylsilyl group; a linear or branched alkyl group having 1 to 6 carbon atoms such as a methyl group, an ethyl group, and a propyl group; a linear or branched alkyloxy group having 1 to 6 carbon atoms such as a methyloxy group, an ethyloxy group, and a propyloxy group; an alkenyl group such as a vinyl group and an allyl group; an aryloxy group such as a phenyloxy group and a tolyloxy group; an arylalkyloxy group such as a benzyloxy group and a phenethyloxy group; an aromatic hydrocarbon group or a fused polycyclic aromatic group such as a phenyl group, a biphenylyl group, a terphenylyl group, a naphthyl group, an anthracenyl group, a phenanthrenyl group, a fluorenyl group, a spirobifluorenyl group, an indenyl group, a pyrenyl group, a perylenyl group, a fluoranethenyl group, and a triphenylenyl group; a pyridyl group, a thienyl group, a furil group, a pyrrolyl group, a quinolyl group, an isoquinolyl group, a benzofuranyl group, a benzothienyl group, an indolyl group, a carbazolyl group, an imidazopyridyl group, a benzoxazolyl group, a benzothiazolyl group, a quinoxalinyl group, a benzimidazolyl group, a pyrazolyl group, a dibenzofuranyl group, a dibenzothienyl group, a carbolinyl group, and the like. These substituent groups may be further substituted with the exemplified substituted groups. Further, these substituent groups and the substituted benzene ring or a plurality of substituent groups on the same benzene ring may be boded to each other via a single bond, a substituted or unsubstituted methylene group, an oxygen atom, or a sulfur atom to form a ring.

In the amine compound according to the present invention, as Ar₂ in the general formula (A), (B), or (C), an unsubstituted aromatic hydrocarbon group or an unsubstituted aromatic heterocyclic group is favorable, and an unsubstituted phenyl group, an unsubstituted naphthyl group, an unsubstituted dibenzofuranyl group, an unsubstituted dibenzothienyl group, an unsubstituted benzoxazolyl group, an unsubstituted benzothiazolyl group, an unsubstituted benzofuranyl group, an unsubstituted benzothienyl group, an unsubstituted phenanthryl group, or an unsubstituted quinolyl group is more favorable.

In the amine compound according to the present invention, as Ar₃ in the general formula (A), (B), or (C), a substituted or unsubstituted phenyl group, a substituted or unsubstituted biphenyl group, a substituted or unsubstituted naphthyl group, a substituted or unsubstituted furanyl group, or a substituted or unsubstituted thienyl group is favorable, an unsubstituted phenyl group, an unsubstituted biphenyl group, an unsubstituted naphthyl group, an unsubstituted furanyl group, or an unsubstituted thienyl group is more favorable, an unsubstituted phenyl group or an unsubstituted naphthyl group is more favorable, and an unsubstituted phenyl group or an unsubstituted 2-naphthyl group is still more favorable.

In the amine compound according to the present invention, Ar₁ in the general formula (A), (B), or (C) represents a substituted or unsubstituted benzoxazolyl group, a substituted or unsubstituted benzothiazolyl group, a substituted or unsubstituted benzofuranyl group, a substituted or unsubstituted benzothienyl group, a substituted or unsubstituted dibenzofuranyl group, or a substituted or unsubstituted dibenzothienyl group.

Favorably , Ar₁ represents a substituted or unsubstituted 2-benzoxazolyl group, a substituted or unsubstituted 2-benzothiazolyl group, a substituted or unsubstituted 2-benzofuranyl group, a substituted or unsubstituted 2-benzothienyl group, a substituted or unsubstituted 2-dibenzofuranyl group, a substituted or unsubstituted 2-dibenzothienyl group, a substituted or unsubstituted 3-dibenzofuranyl group, or a substituted or unsubstituted 3-dibenzothienyl group.

More favorably , Ar₁ represents an unsubstituted 2-benzoxazolyl group, an unsubstituted 2-benzothiazolyl group, an unsubstituted 2-benzofuranyl group, an unsubstituted 2-benzothienyl group, an unsubstituted 2-dibenzofuranyl group, an unsubstituted 2-dibenzothienyl group, an unsubstituted 3-dibenzofuranyl group, or an unsubstituted 3-dibenzothienyl group.

In the amine compound according to the present invention, examples of the "substituent group" in the "substituted benzoxazolyl group", "substituted benzothiazolyl group", "substituted benzofuranyl group", "substituted benzothienyl group", "substituted dibenzofuranyl group", or "substituted dibenzothienyl group" represented by Ar₁ in the general formula (A), (B), or (C) includes those shown for the "substituent group" in the "substituted aromatic hydrocarbon group", "substituted aromatic heterocyclic group", or "substituted fused polycyclic aromatic group" represented by Ar₂ and Ar₃ in the general formula (A), and aspects similar to those of the "substituent group" in the "substituted aromatic hydrocarbon group", "substituted aromatic heterocyclic group", or "substituted fused polycyclic aromatic group" represented by Ar₂ and Ar₃ in the general formula (A) can also be taken.

In the amine compound according to the present invention, L₁ to L₃ in the general formula (A), (B), or (C) may be the same as or different from each other and each represent a single bond, a divalent group obtained by removing two hydrogen atoms from unsubstituted benzene, a divalent group obtained by removing two hydrogen atoms from unsubstituted biphenyl, or a divalent group obtained by removing two hydrogen atoms from unsubstituted naphthalene, favorably a single bond, an unsubstituted 1,4-phenylene group, an unsubstituted 4,4'-biphenylylene group, an unsubstituted 2,6-naphthylene group, or an unsubstituted 2,7-naphthylene group. In particular, L₃ represents favorably an unsubstituted 1,4-phenylene group or an unsubstituted 4,4'-biphenylylene group, more favorably an unsubstituted 1,4-phenylene group.

In the amine compound according to the present invention, X in the general formula(A), (B), or (C) represents an oxygen atom or a sulfur atom.

The amine compound according to the present invention is suitable for use in an organic EL device, an electronic apparatus, or an electronic device.

Here, the electronic apparatus or the electronic device is an electronic apparatus such as a television set, a mobile phone, a car navigation system, and a digital camera or an electronic device such as a semiconductor device, and includes a pair of electrodes and at least one organic layer sandwiched between the pair of electrodes. The amine compound according to the present invention is included in the organic layer.

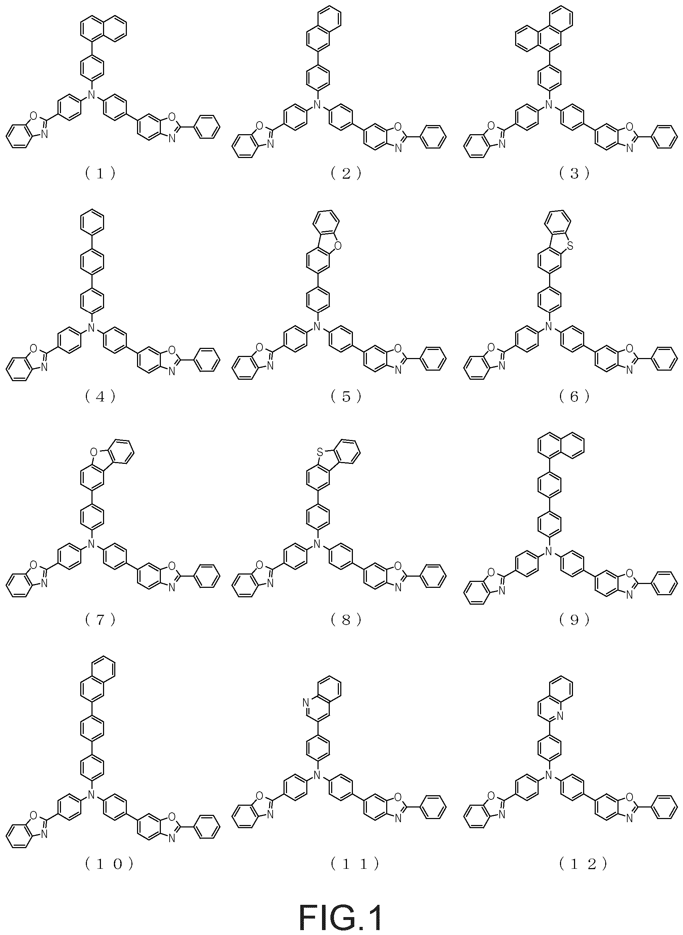

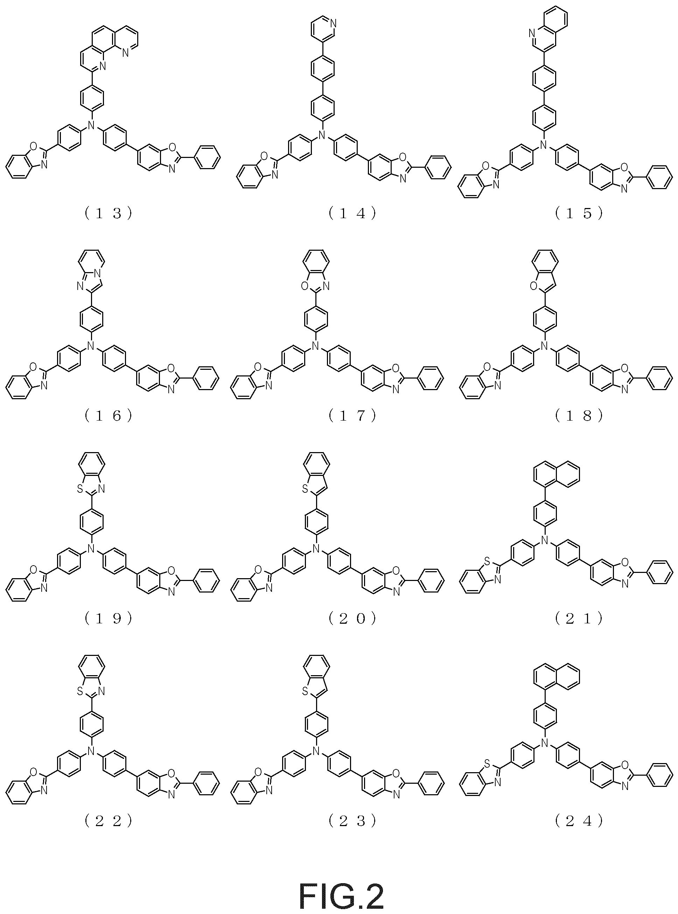

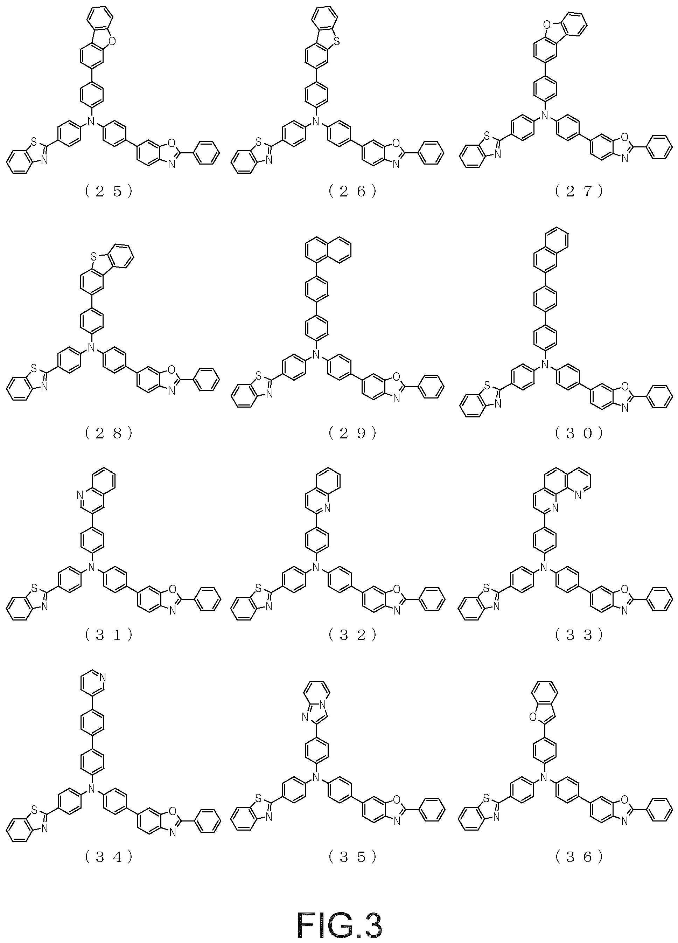

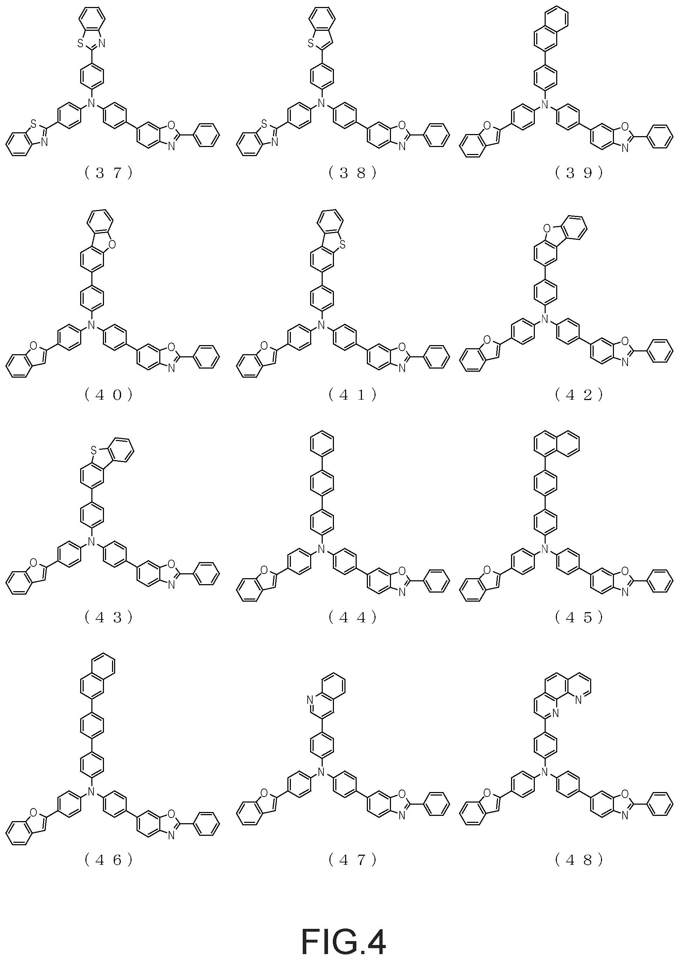

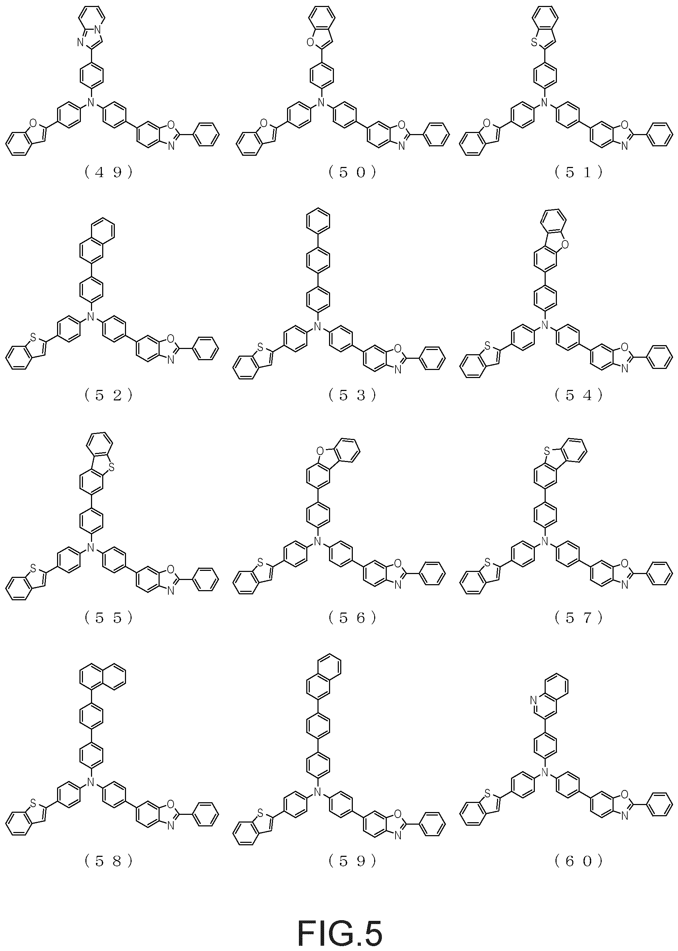

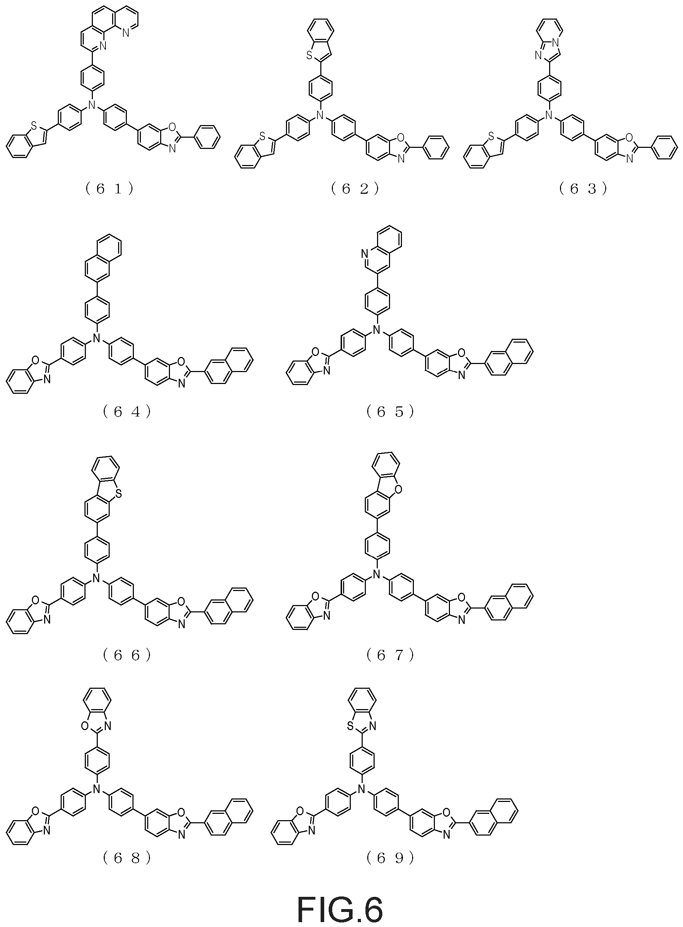

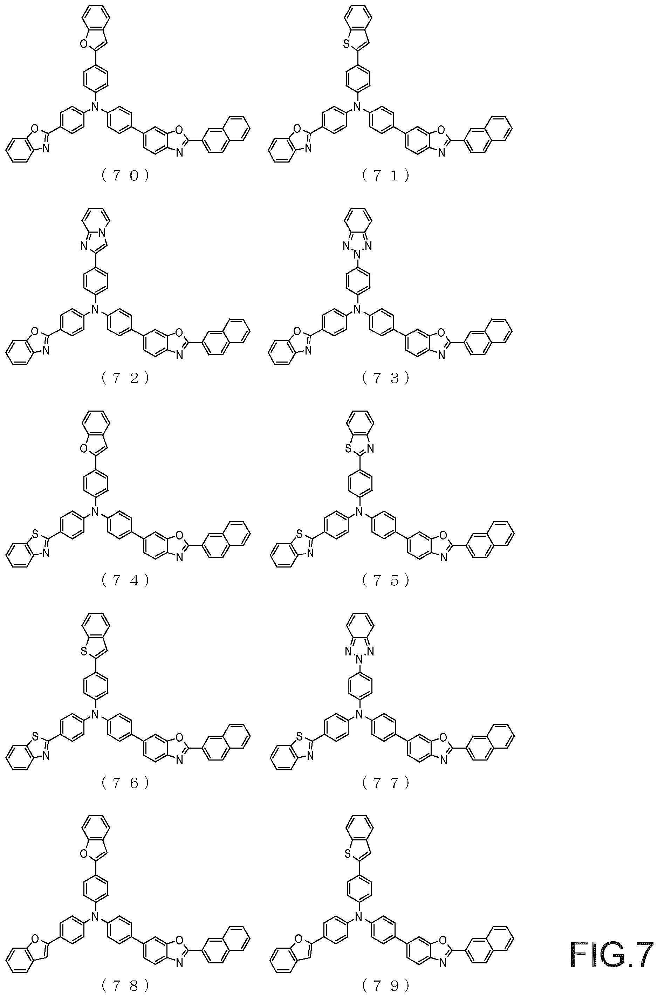

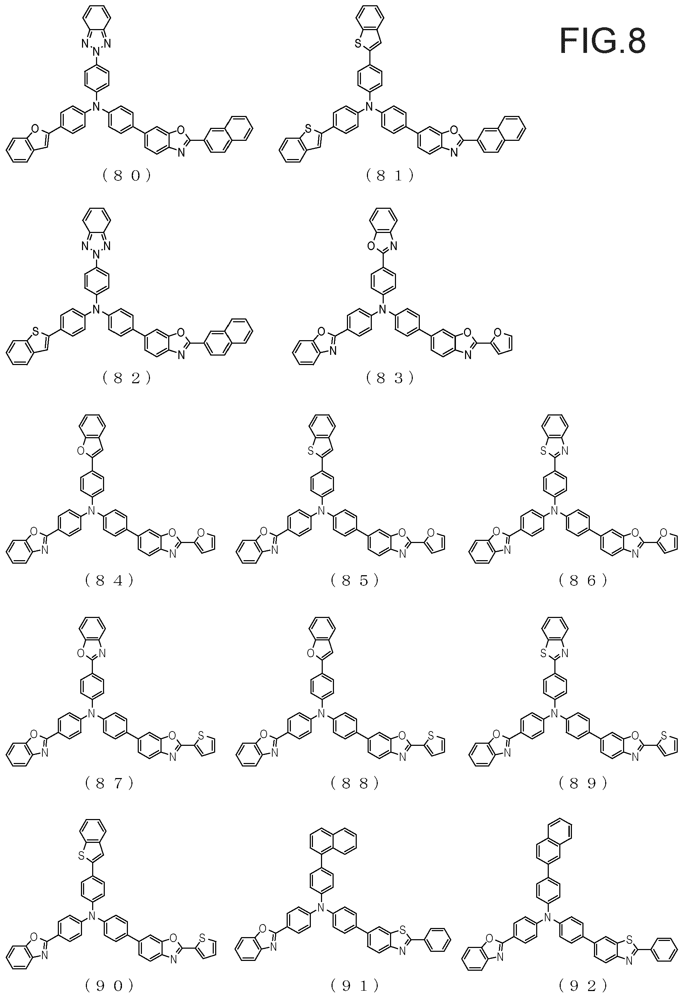

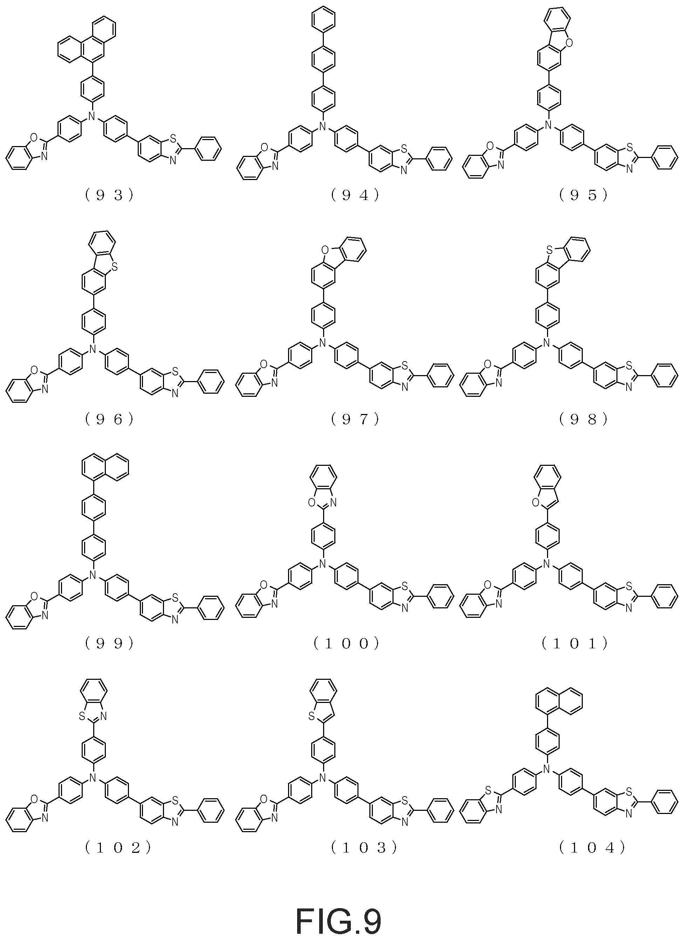

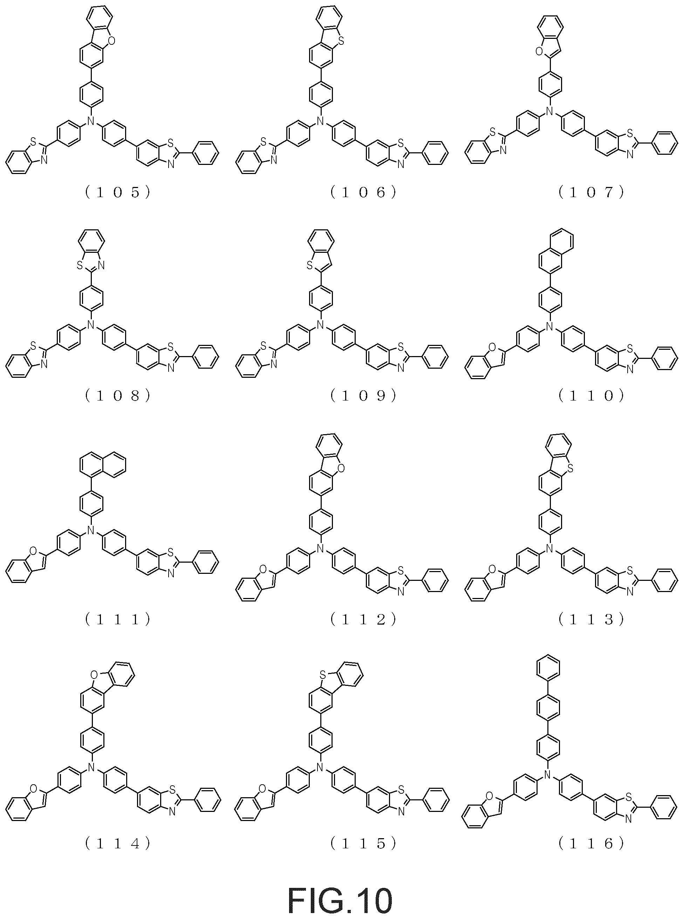

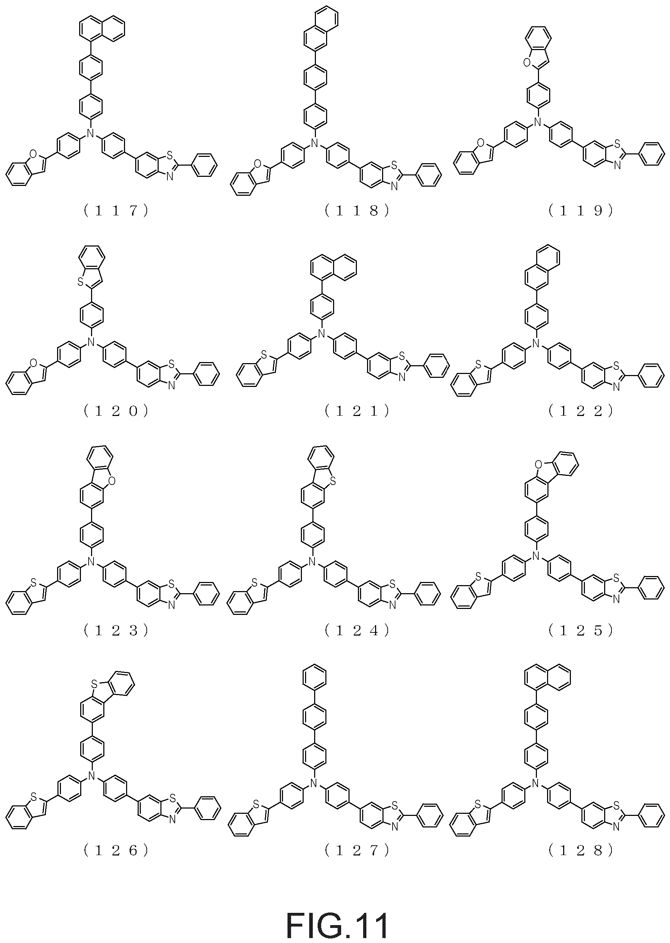

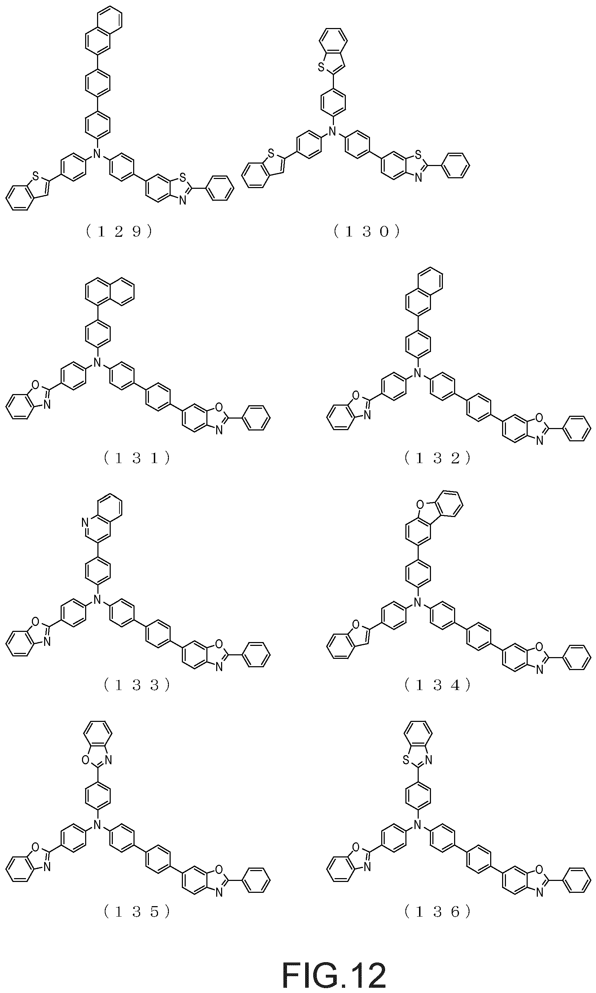

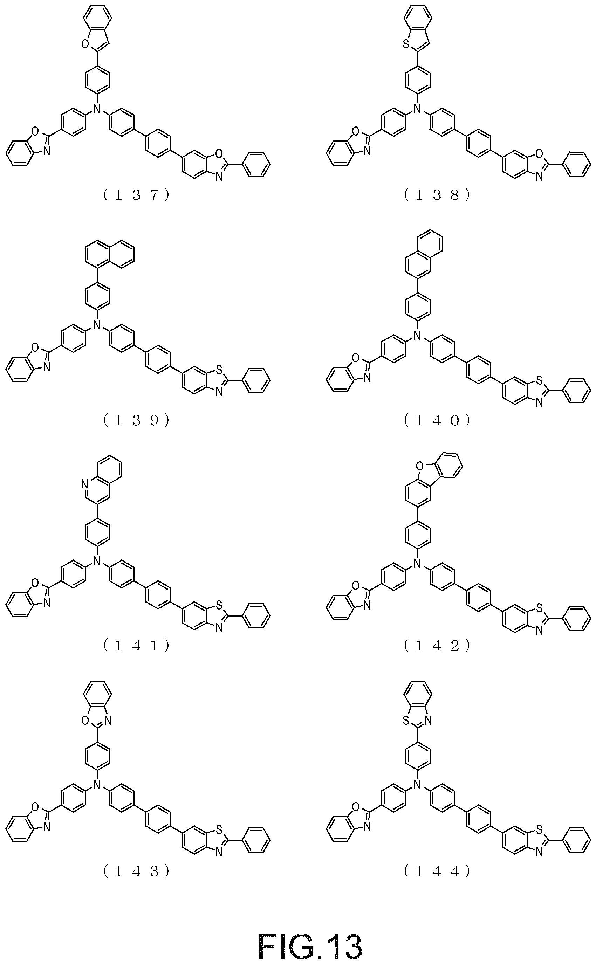

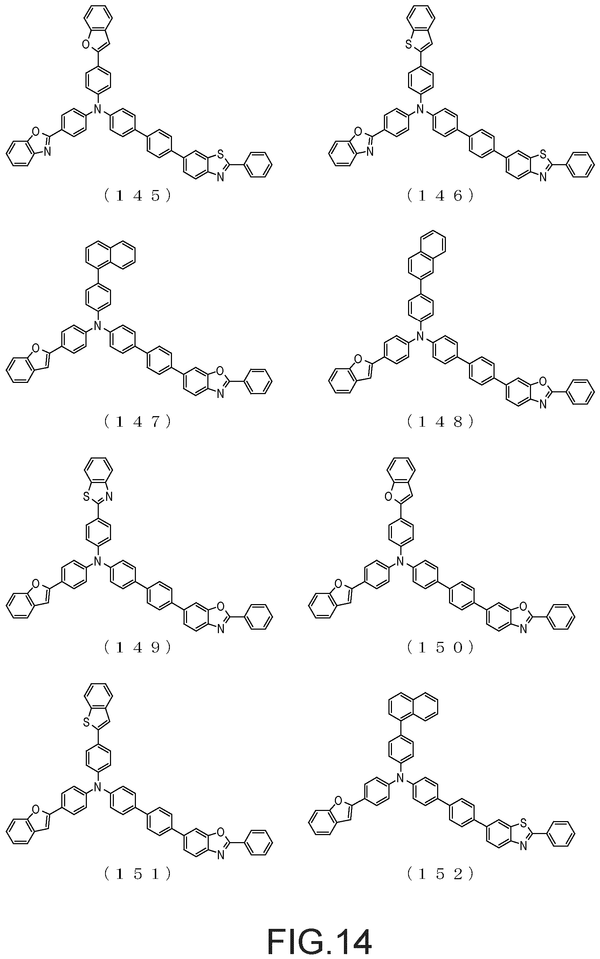

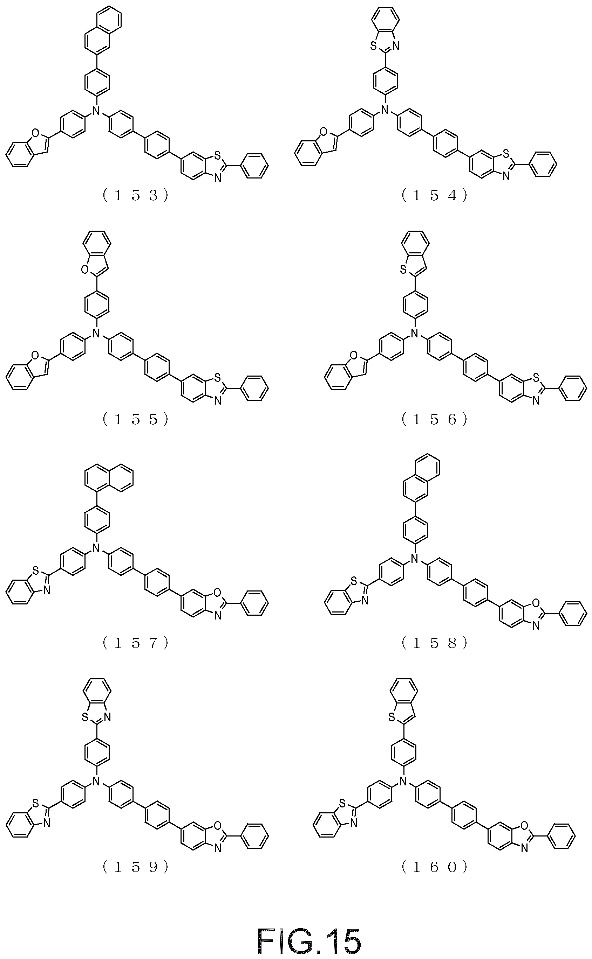

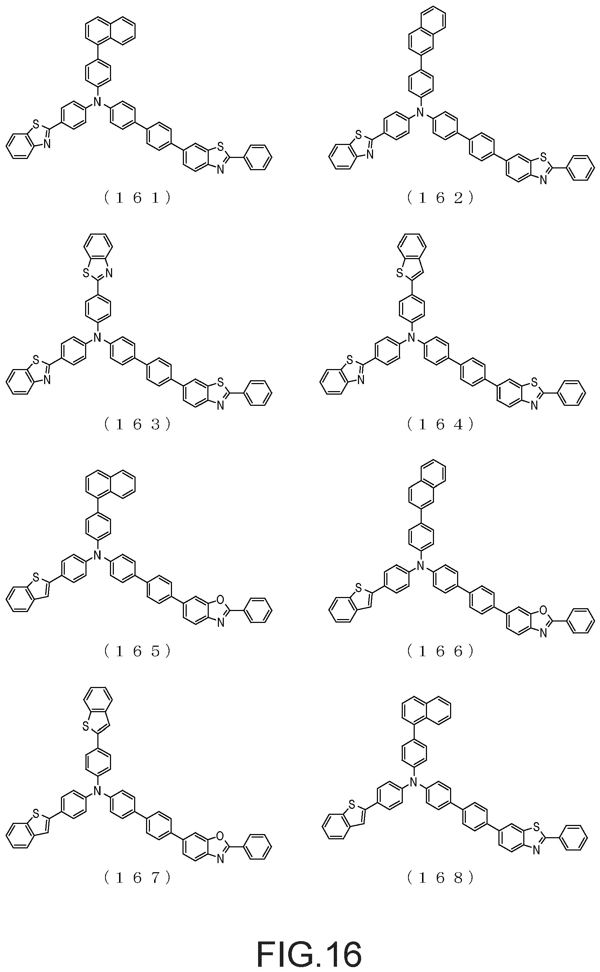

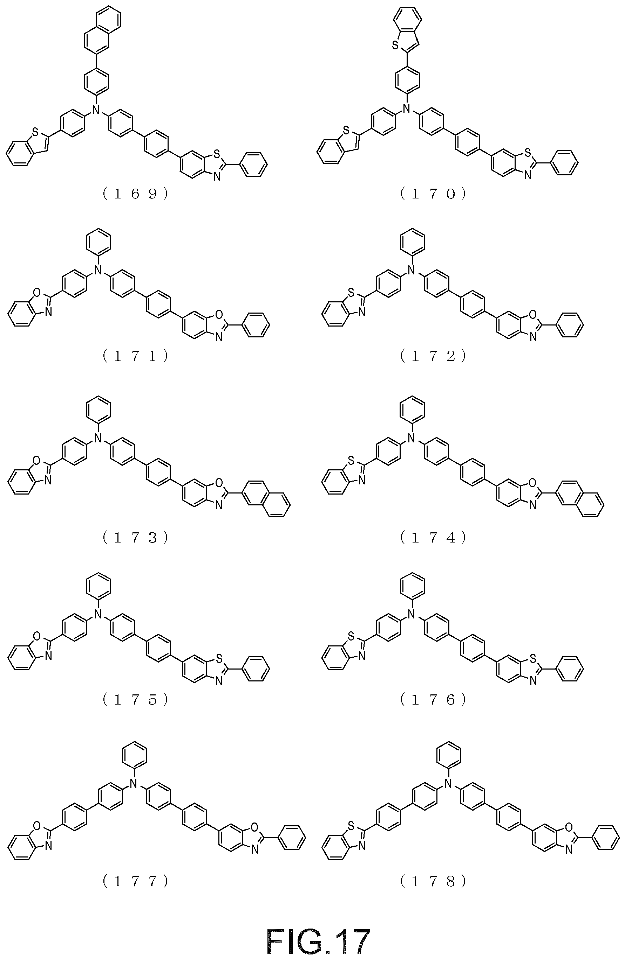

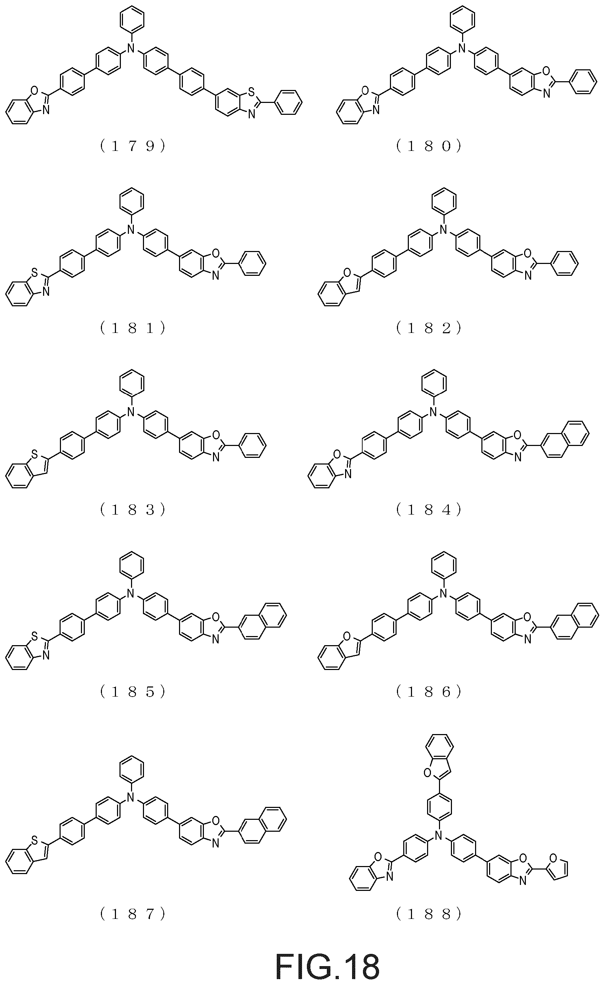

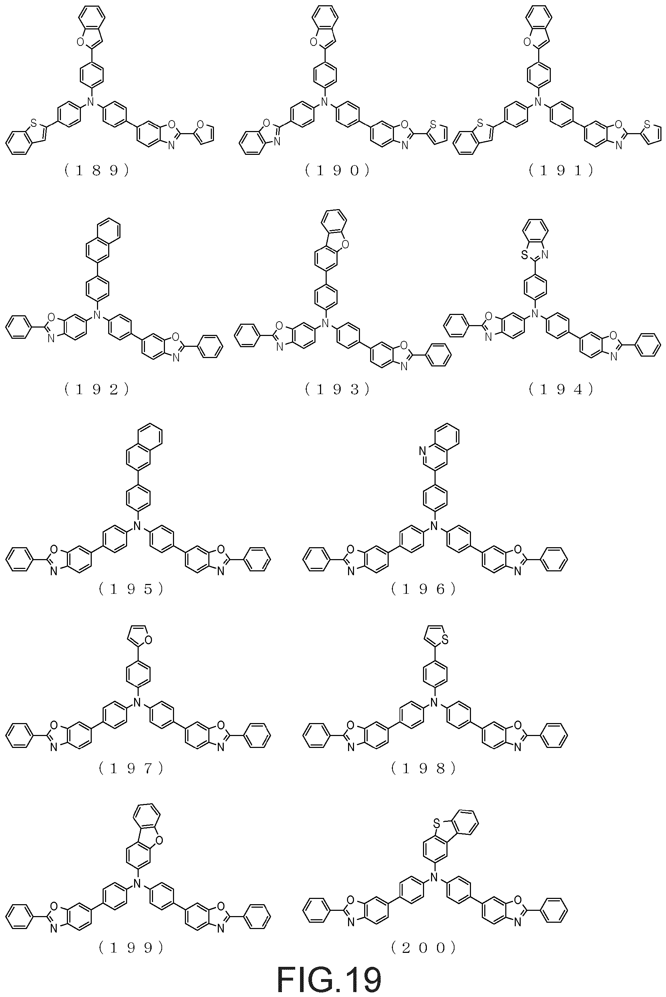

Although specific examples of favorable compounds among the amine compounds according to the present invention, which are suitable for use in the organic EL device, electronic apparatus, and electronic device according to the present invention, are shown in Fig. 1 to Fig. 20, the present invention is not limited to these compounds.

The purification of the amine compound according to the present invention is not particularly limited and may be performed by known methods used for purifying organic compounds, such as purification by column chromatography, adsorption purification with silica gel, activated carbon, activated clay, or the like, a recrystallization purification method with a solvent, a crystallization purification method, and a sublimation purification method, and the compound was identified by NMR analysis. The melting point, the glass transition temperature (Tg), and the refractive index were measured as physical property values. The melting point is an index of a vapor deposition property. The glass transition point (Tg) is an index of stability in a thin film state. The refractive index is an index regarding the improvement of light extraction efficiency.

The melting point and the glass transition point (Tg) were measured with a powder using a high sensitivity differential scanning calorimeter (DSC3100SA manufactured by Bruker AXS GmbH).

The refractive index and the extinction coefficient were measured by preparing a thin film having a thickness of 80 nm on a silicon substrate and using a spectrometer (F10-RT-UV manufactured by Filmetrics, INC.)

The absorbance at wavelengths of 400 nm and 410 nm was calculated by adjusting the concentration to 10⁻⁵ mol/L with a toluene solvent and using the compound according to the present invention, and the absorption coefficient was calculated by adjusting the concentration to four types of concentrations, i.e., 5.0×10⁻⁶ mol/L, 1.0×10⁻⁵ mol/L, 1.5×10⁻⁵ mol/L, and 2.0×10⁻⁵ mol/L, with a toluene solvent, from the calibration curve obtained by measurement using an ultraviolet-visible near-infrared spectrophotometer (V-650 manufactured by JASCO Corporation).

Examples of the structure of the organic EL device according to the present invention include, in the case of a light-emitting element having a top emission structure, those including an anode, a hole transport layer, a light-emitting layer, an electron transport layer, a cathode, and a capping layer in this order on a glass substrate, those including a hole injection layer between the anode and the hole transport layer, those including an electron blocking layer between the hole transport layer and the light-emitting layer, those including a hole blocking layer between the light-emitting layer and the electron transport layer, and those including an electron injection layer between the electron transport layer and the cathode. In the multilayer structures, several organic layers can be omitted or combined. For example, an organic layer may serve as both the hole injection layer and the hole transport layer, both the hole transport layer and the electron blocking layer, both the hole blocking layer and the electron transport layer, or both the electron transport layer and the electron injection layer. Further, two or more organic layers having the same function may be stacked. For example, two hole transport layers may be stacked, two light-emitting layers may be stacked, two electron transport layers may be stacked, and two capping layers may be stacked.

The total film thickness of the respective layers of the organic EL device is favorably approximately 200 nm to 750 nm, more favorably approximately 350 nm to 600 nm. Further, the film thickness of the capping layer is, for example, favorably 30 nm to 120 nm, more favorably 40 nm to 80 nm. In this case, favorable light extraction efficiency can be achieved. Note that the film thickness of the capping layer can be appropriately changed depending on the type of light-emitting material used in the light-emitting element, the thickness of the organic EL device other than the capping layer, and the like.

For the anode of the organic EL device according to the present invention, an electrode material having a large work function, such as ITO and gold, is used.

For a hole injection layer of the organic EL device according to the present invention, an arylamine compound having three or more triphenylamine structures in the molecule linked by a single bond or a divalent group that does not contain a heteroatom, such as a starburst triphenylamine derivative and various triphenylamine tetramers, a porphyrin compound typified by copper phthalocyanine, an acceptor heterocyclic compound such as hexacyanoazatriphenylene, and a coating type polymer material can be used. These may be deposited alone or may be mixed with other materials and deposited to form a single layer. Further, a stacked structure of layers deposited alone, layers mixed and deposited, or layers including a layer deposited alone and a layer mixed and deposited may be provided. These materials can be formed into a thin film by a known method such as a spin coat method and an ink jet method in addition to a vapor deposition method.

For the hole transport layer of the organic EL device according to the present invention, a benzidine derivative such as N,N'-diphenyl-N,N'-di(m-tolyl)benzidine (hereinafter, abbreviated as TPD), N,N'-diphenyl-N,N'-di(α-naphthyl)benzidine, and N,N,N',N'-tetrabiphenylylbenzidine, 1,1-bis[4-(di-4-tolylamino)phenyl]cyclohexane, particularly an arylamine compound having a structure in which two triphenylamine structures are linked by a single bond or a divalent group that does not contain a heteroatom in the molecule, such as N, N, N', N'-tetrabiphenylylbenzidine, or the like is favorably used. Further, an arylamine compound having three or more triphenylamine structures in the molecule linked by a single bond or a divalent group that does not contain a heteroatom, such as various triphenylamine trimers and tetramers, is favorably used. These may be deposited alone or may be mixed with other materials and deposited to form a single layer. Further, a stacked structure of layers deposited alone, layers mixed and deposited, or layers including a layer deposited alone and a layer mixed and deposited may be provided. Further, for the hole injection/transport layer, a coating type polymer material such as poly(3,4-ethylenedioxythiophene)/poly(styrene sulfonate) can be used. These materials can be formed into a thin film by a known method such as a spin coat method and an ink jet method in addition to a vapor deposition method.

Further, for the hole injection layer or the hole transport layer, those obtained by P-doping materials that are normally used in the layer with trisbromophenylaminehexachloroantimony, a radialene derivative (see, for example, Patent Literature 3), or the like, a polymer compound having a benzidine derivative structure such as TPD in its partial structure, or the like can be used.

For the electron blocking layer of the organic EL device according to the present invention, a compound having an electron blocking effect, such as a carbazole derivative such as 4,4',4"-tri(N-carbazolyl)triphenylamine (hereinafter, abbreviated as TCTA), 9,9-bis[4-(carbazole-9-yl)phenyl]fluorene, 1,3-bis(carbazol-9-yl)benzene (hereinafter, abbreviated as mCP), and 2,2-bis(4-carbazol-9-yl-phenyl)adamantane and a compound having a triphenylsilyl group and a triarylamine structure typified by 9-[4-(carbazole-9-yl)phenyl]-9-[4-(triphenylsilyl)phenyl]-9H-fluorene can be used. These may be deposited alone or may be mixed with other materials and deposited to form a single layer. Further, a stacked structure of layers deposited alone, layers mixed and deposited, or layers including a layer deposited alone and a layer mixed and deposited may be provided. These materials can be formed into a thin film by a known method such as a spin coat method and an ink jet method in addition to a vapor deposition method.

For the light-emitting layer of the organic EL device according to the present invention, various metal complexes, an anthracene derivative, a bis-styrylbenzene derivative, a pyrene derivative, an oxazole derivative, a polyparaphenylene vinylene derivative, or the like can be used in addition to a metal complex of a quinolinol derivative including Alq₃. Further, the light-emitting layer may be formed of a host material and a dopant material, and an anthracene derivative is favorably used as the host material. In addition to the light-emitting material, a heterocyclic compound having an indole ring as a partial structure of a fused ring, a heterocyclic compound having a carbazole ring as a partial structure of a fused ring, a carbazole derivative, a thiazole derivative, a benzimidazole derivative, a polydialkylfluorene derivative, or the like can be used. Further, as the dopant material, quinacridone, coumarin, rubrene, perylene, and derivatives thereof, a benzopyran derivative, a rhodamine derivative, an aminostyryl derivative, and the like can be used. These may be deposited alone or may be mixed with other materials and deposited to form a single layer. A stacked structure of layers deposited alone, layers mixed and deposited, or layers including a layer deposited alone and a layer mixed and deposited may be provided.

Further, as the light-emitting material, a phosphorescent emitter can be used. As the phosphorescent emitter, a phosphorescent emitter of a metal complex such as iridium and platinum can be used. A green phosphorescent emitter such as Ir(ppy)₃, a blue phosphorescent emitter such as FIrpic and FIr6, a red phosphorescent emitter such as Btp₂Ir (acac), or the like is used. As the host material at this time, a carbazole derivative such as 4,4'-di(N-carbazolyl)biphenyl, TCTA, and mCP, which are hole injection/transport host materials, can be used. As the electron transport host material, p-bis(triphenylsilyl)benzene, 2,2',2''-(1,3,5-phenylene)-tris(1-phenyl-1H-benzimidazole), or the like can be used, and a high-performance organic EL device can be prepared.

In order to avoid concentration quenching, it is favorable to dope the phosphorescent light-emitting material with the host material by co-deposition in the range of 1 to 30 weight percent with respect to the entire light-emitting layer.

Further, as the light-emitting material, a material that emits delayed fluorescence such as a CDCB derivative including PIC-TRZ, CC2TA, PXZ-TRZ, and 4CzIPN may be used (see, for example, Non-Patent Literature 7). These materials can be formed into a thin film by a known method such as a spin coat method and an ink jet method in addition to a vapor deposition method.

For the hole blocking layer of the organic EL device according to the present invention, a compound having a hole blocking effect, such as a phenanthroline derivative such as bathocuproine, a metal complex of a quinolinol derivative such as aluminum(III)bis(2-methyl-8-quinolinate)-4-phenylphenolate (hereinafter, abbreviated as BAlq), various rare earth complex, a triazole derivative, a triazine derivative, a pyrimidine derivative, an oxadiazole derivative, and a benzoazole derivative, can be used. These materials may also serve as the material of the electron transport layer. These may be deposited alone or may be mixed with other materials and deposited to form a single layer. Further, a stacked structure of layers deposited alone, layers mixed and deposited, or layers including a layer deposited alone and a layer mixed and deposited may be provided. These materials can be formed into a thin film by a known method such as a spin coat method and an ink jet method in addition to a vapor deposition method.

For the electron transport layer of the organic EL device according to the present invention, various metal complexes, a triazole derivative, a triazine derivative, a pyrimidine derivative, an oxadiazole derivative, a pyridine derivative, a benzimidazole derivative, a benzoazole derivative, a thiadiazole derivative, an anthracene derivative, a carbodiimide derivative, a quinoxaline derivative, a pyridoindole derivative, a phenanthroline derivative, a silole derivative, and the like can be used in addition to the metal complex of a quinolinol derivative including Alq₃ and BAlq. These may be deposited alone or may be mixed with other materials and deposited to form a single layer. Further, a stacked structure of layers deposited alone, layers mixed and deposited, or layers including a layer deposited alone and a layer mixed and deposited may be provided. These materials can be formed into a thin film by a known method such as a spin coat method and an ink jet method in addition to a vapor deposition method.

For the electron injection layer of the organic EL device according to the present invention, an alkali metal salt such as lithium fluoride and cesium fluoride, an alkaline earth metal salt such as magnesium fluoride, a metal complex of a quinolinol derivative such as lithium quinolinol, a metal oxide such as aluminum oxide, a metal such as ytterbium (Yb), samarium (Sm), calcium (Ca), strontium (Sr), and cesium (Cs), or the like can be used. However, this can be omitted in the favorable selection of the electron transport layer and the cathode.

Further, in the electron injection layer or the electron transport layer, those obtained by N-doping the material typically used for the respective layers with a metal such as cesium can be used.

For the cathode of the organic EL device according to the present invention, an electrode material having a low work function, such as aluminum, an alloy having a lower work function, such as a magnesium silver alloy, a magnesium calcium alloy, a magnesium indium alloy, and an aluminum magnesium alloy, ITO, IZO, or the like is used as an electrode material.

For the capping layer of the organic EL device according to the present invention, an amine compound represented by the general formula(A), (B), or (C) is used. These may be deposited alone or may be mixed with other materials and deposited to form a single layer. A stacked structure of layers deposited alone, layers mixed and deposited, or layers including a layer deposited alone and a layer mixed and deposited may be provided. These materials can be formed into a thin film by a known method such as a spin coat method and an ink jet method in addition to a vapor deposition method.

Note that although the organic EL device having a top emission structure has been described above, the present invention is not limited thereto and is applicable similarly to an organic EL device having a bottom emission structure and an organic EL device having a dual emission structure in which light is emitted from the upper part and the bottom portion. In these cases, the electrode present in the direction in which light is extracted from the light-emitting element to the outside needs to be transparent or semi-transparent.

The refractive index of the material for forming a capping layer is favorably larger than the refractive index of the adjacent electrode. That is, the capping layer improves the light extraction efficiency of the organic EL device, but this effect is effective when the reflectance at the interface between the capping layer and the material in contact with the capping layer is greater, because the effect of the optical interference is larger. For this reason, the refractive index of the material for forming a capping layer is favorably larger than the refractive index of the adjacent electrode.

In the organic EL device according to the present invention, the refractive index in the wavelength range of 450 nm to 750 nm of light transmitted through the capping layer only needs to be 1.70 or more, and is more favorably 1.80 or more, particularly favorably 1.85 or more, and still more favorably 1.90 or more.

In the organic EL device according to the present invention, the thickness of the capping layer is favorably in the range from 30 nm to 120 nm, more favorably 40 nm to 80 nm.

It is favorable that the capping layer of the organic EL device according to the present invention has an extinction coefficient of 0.2 or more in a wavelength range from 400 nm to 410 and absorbance in an absorption spectrum at a concentration of 10⁻⁵ mol/L of 0.2 or more in the wavelength range from 400 nm to 410 nm.

In the organic EL device according to the present invention, the capping layer may be prepared by forming a stacked layer or a mixed layer of two or more types of different constituent materials. Note that at least one of the constituent materials is the amine compound according to the present invention. It is favorable that the refractive index, the extinction coefficient, and the absorbance of the capping layer are within the above numerical ranges.

Although an embodiment of the present invention will be specifically described by way of Examples, the present invention is not limited to the following Examples as long as it does not exceed the gist of the present invention.

### (Example 1)

### <Synthesis of N-(4-benzofuran-2-yl-phenyl)-N-(4-dibenzofuran-3-yl-phenyl)-N-{4-(2-phenyl-benzooxazole-6-yl)-phenyl}-amine (compound 40)>

N-(4-benzofuran-2-yl-phenyl)-N-(4-dibenzofuran-3-yl-phenyl)-amine: 10.0 g, 6-(4-chloro-phenyl)-2-phenyl-benzooxazole: 7.4 g, tris(dibenzylideneacetone)dipalladium(0): 0.4 g, tri-t-butylphosphine: 0.2 g, and t-butoxy sodium: 3.2 g were placed in a reaction vessel purged with nitrogen and refluxed and stirred overnight in xylene solvent. After the mixture is allowed to cool, methanol was added to the system and the precipitated solid was filtered to obtain a crude product. The obtained crude product was purified by recrystallization using a chlorobenzene solvent to obtain a yellow powder: 8.6 g (yield: 53.9%) of N-(4-benzofuran-2-yl-phenyl)-N-(4-dibenzofuran-3-yl-phenyl)-N-{4-(2-phenyl-benzooxazole-6-yl)-phenyl}-amine (compound 40).

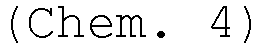

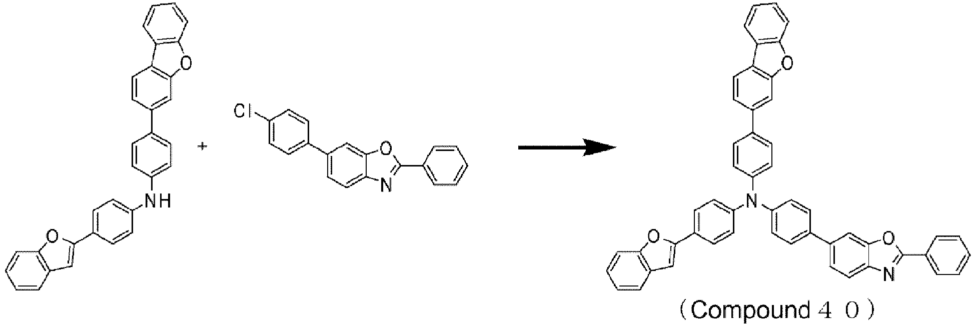

The structure of the obtained yellow powder was identified using NMR. The following 32 hydrogen signals were detected in ¹H-NMR(CDCl₃).
δ(ppm) = 8.29(1H), 8.28(1H), 7.99(1H), 7.96(1H), 7.80(5H), 7.68-7.57(8H), 7.57-7.49(4H), 7.46(1H), 7.36(1H), 7.38-7.26(6H), 7.23(2H), and 6.95(1H)

### (Example 2)

### <Synthesis of N,N-bis(4-benzofuran-2-yl-phenyl)-N-{4-(2-phenyl-benzooxazole-6-yl)-phenyl}-amine (compound 50)>

4-(2-phenyl-benzooxazole-6-yl)-phenyl-amine: 8.7 g, 2-(4-bromo-phenyl)-benzofuran: 17.4 g, tris(dibenzylideneacetone)dipalladium(0): 0.6 g, tri-t-butylphosphine: 0.3 g, and t-butoxy sodium: 4.4 g were placed in a reaction vessel purged with nitrogen and refluxed and stirred overnight in xylene solvent. After the mixture is allowed to cool, methanol was added to the system and the precipitated solid was filtered to obtain a crude product. The obtained crude product was purified by recrystallization using a chlorobenzene solvent to obtain a yellow powder: 14.3 g (yield: 70.1%) of N,N-bis(4-benzofuran-2-yl-phenyl)-N-{4-(2-phenyl-benzooxazole-6-yl)-phenyl}-amine (compound 50).

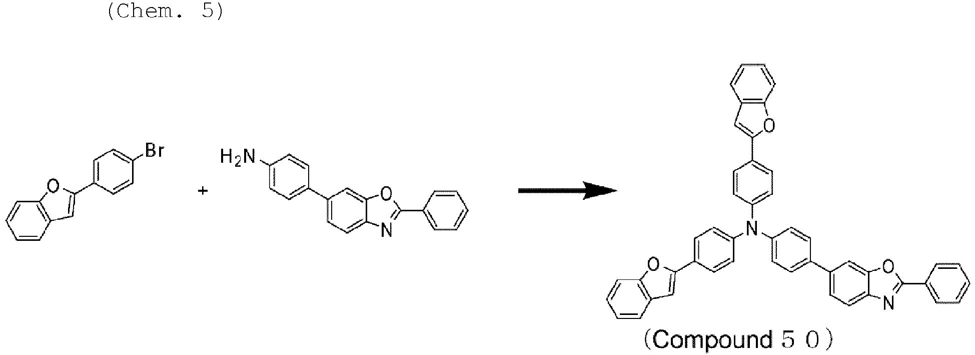

The structure of the obtained yellow powder was identified using NMR. The following 30 hydrogen signals were detected in ¹H-NMR(CDCl₃).

δ(ppm) = 8.28(2H), 7.80(6H), 7.65-7.54(8H), 7.52(2H), 7.31-7.20(10H), and 6.96(2H).

### (Example 3)

### <Synthesis of N-(4-benzooxazole-2-yl-phenyl)-N-(4-benzothiazole-2-yl-phenyl)-N-{4-(2-naphthalene-2-yl-benzooxazole-6-yl)-phenyl}-amine (compound 69)>

N-(4-benzooxazole-2-yl-phenyl)-N-(4-benzothiazole-2-yl-phenyl)-amine: 7.0 g, 6-(4-chloro-phenyl)-2-naphthalene-2-yl-benzooxazole: 6.5 g, tris(dibenzylideneacetone)dipalladium(0): 0.3 g, tri-t-butylphosphine: 0.1 g, and t-butoxy sodium: 2.4 g were placed in a reaction vessel purged with nitrogen and refluxed and stirred overnight in xylene solvent. After the mixture is allowed to cool, methanol was added to the system and the precipitated solid was filtered to obtain a crude product. The obtained crude product was purified by crystallization using a mixed solvent of chlorobenzene and acetone to obtain a yellow powder: 3.6 g (yield: 28.9%) of N-(4-benzooxazole-2-yl-phenyl)-N-(4-benzothiazole-2-yl-phenyl)-N-{4-(2-naphthalene-2-yl-benzooxazole-6-yl)-phenyl}-amine (compound 69).

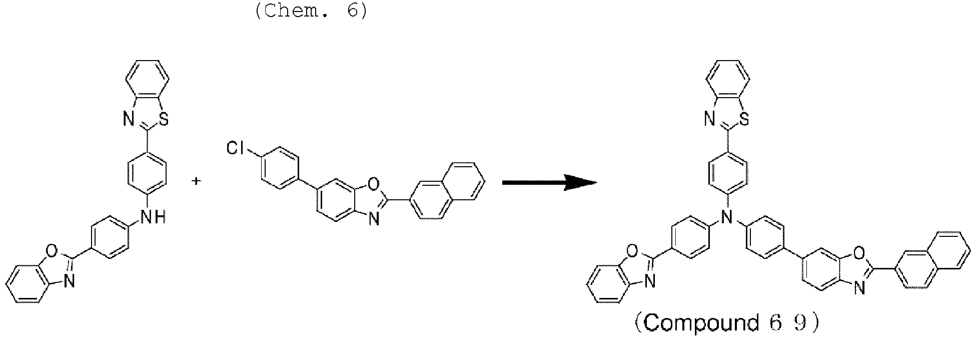

The structure of the obtained yellow powder was identified using NMR. The following 30 hydrogen signals were detected in ¹H-NMR(CDCl₃).

(ppm) = 8.80(1H), 8.34(1H), 8.18(2H), 8.05(3H), 8.00(2H), 7.90(2H), 7.86(1H), 7.85(1H), 7.76(1H), 7.65(3H), 7.58(3H), 7.49(1H), and 7.41-7.27(9H).

### (Example 4)

### <Synthesis of N-(4-benzofuran-2-yl-phenyl)-N-(4-benzooxazole-2-yl-phenyl)-N-{4-(2-naphthalene-2-yl-benzooxazole-6-yl)-phenyl}-amine (compound 70)>

N-(4-benzofuran-2-yl-phenyl)-N-(4-benzooxazole-2-yl-phenyl)-amine: 6.4 g, 6-(4-chloro-phenyl)-2-naphthalene-2-yl-benzooxazole: 6.2 g, tris(dibenzylideneacetone)dipalladium(0): 0.3 g, tri-t-butylphosphine: 0.1 g, and t-butoxy sodium: 2.3 g were placed in a reaction vessel purged with nitrogen and refluxed and stirred overnight in xylene solvent. After the mixture is allowed to cool, methanol was added to the system and the precipitated solid was filtered to obtain a crude product. The obtained crude product was purified by crystallization using a mixed solvent of chlorobenzene and acetone to obtain a yellow powder: 4.3 g (yield: 37.3%) of N-(4-benzofuran-2-yl-phenyl)-N-(4-benzooxazole-2-yl-phenyl)-N-{4-(2-naphthalene-2-yl-benzooxazole-6-yl)-phenyl}-amine (compound 70).

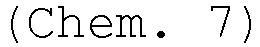

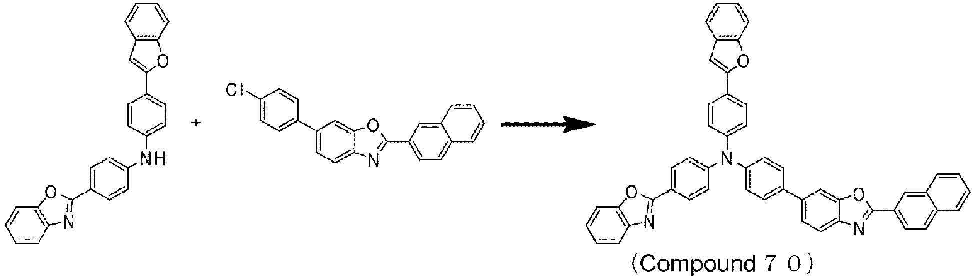

The structure of the obtained yellow powder was identified using NMR. The following 31 hydrogen signals were detected in ¹H-NMR(CDCl₃).

δ(ppm) = 8.81(1H), 8.34(1H), 8.16(2H), 8.01(1H), 7.99(1H), 7.91(1H), 7.84(4H), 7.75(1H), 7.64(3H), 7.58(4H), 7.52(1H), 7.32-7.26(9H), 7.24(1H), and 6.98(1H).

### (Example 5)

### <Synthesis of N,N-bis(4-benzofuran-2-yl-phenyl)-N-{4-(2-naphthalene-2-yl-benzooxazole-6-yl)-phenyl}-amine (compound 78)>

4-(2-naphthalene-2-yl-benzooxazole-6-yl)-phenyl-amine: 5.7 g, 2-(4-bromo-phenyl)-benzofuran: 10.2 g, tris(dibenzylideneacetone)dipalladium(0): 0.6 g, tri-t-butylphosphine: 0.3 g, and t-butoxy sodium: 4.9 g were placed in a reaction vessel purged with nitrogen and refluxed and stirred overnight in toluene solvent. After the mixture is allowed to cool, methanol was added to the system and the precipitated solid was filtered to obtain a crude product. The obtained crude product was purified by recrystallization using a chlorobenzene solvent to obtain a yellow powder: 8.9 g (yield: 72.9%) of N,N-bis(4-benzofuran-2-yl-phenyl)-N-{4-(2-naphthalene-2-yl-benzooxazole-6-yl)-phenyl}-amine (compound 78).

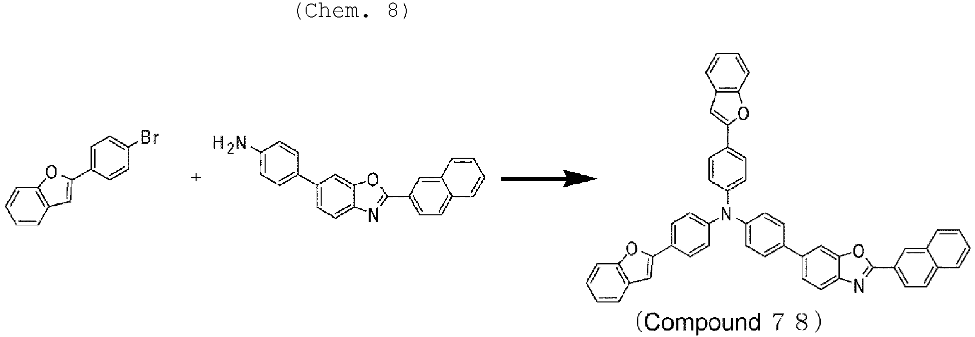

The structure of the obtained yellow powder was identified using NMR. The following 32 hydrogen signals were detected in ¹H-NMR(CDCl₃).

(ppm) = 8.80(1H), 8.34(1H), 8.00(1H), 7.99(1H), 7.90(1H), 7.81(6H), 7.66-7.54(7H), 7.51(2H), 7.32-7.20(10H), and 6.95(2H).

### (Example 6)

### <Synthesis of N,N-bis(4-benzofuran-2-yl-phenyl)-N-{4-(2-phenyl-benzothiazole-6-yl)-phenyl}-amine (compound 119)>

4-(2-phenyl-benzothiazole-6-yl)-phenyl-amine: 4.2 g, 2-(4-bromo-phenyl)-benzofuran: 8.0 g, tris(dibenzylideneacetone)dipalladium(0): 0.5 g, tri-t-butylphosphine: 0.2 g, and t-butoxy sodium: 4.0 g were placed in a reaction vessel purged with nitrogen and refluxed and stirred overnight in toluene solvent. After the mixture is allowed to cool, methanol was added to the system and the precipitated solid was filtered to obtain a crude product. The obtained crude product was purified by recrystallization using a toluene solvent to obtain a yellow powder: 7.6 g (yield: 79.7%) of N,N-bis(4-benzofuran-2-yl-phenyl)-N-{4-(2-phenyl-benzothiazole-6-yl)-phenyl}-amine (compound119).

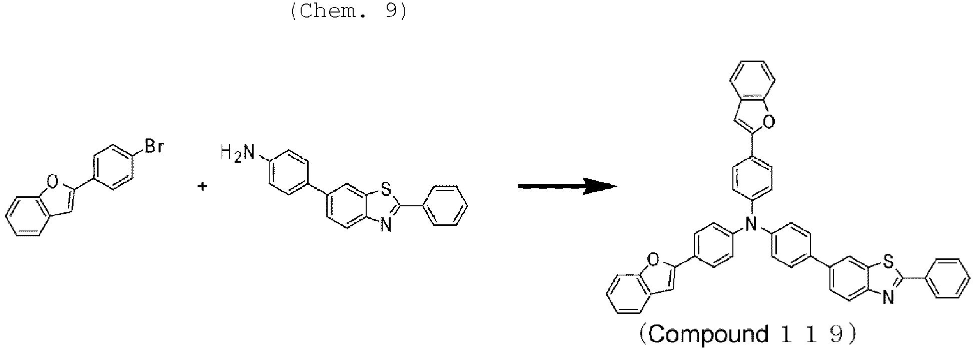

The structure of the obtained yellow powder was identified using NMR. The following 30 hydrogen signals were detected in ¹H-NMR(CDCl₃).

δ(ppm) = 8.12(4H), 7.79(4H), 7.73(1H), 7.61(2H), 7.57(2H), 7.51(5H), 7.32-7.19(10H), and 6.95(2H).

### (Example 7)

### <Synthesis of N, N-(bis(4-benzofuran-2-yl-phenyl)-N-{4'-(2-phenyl-benzooxazole-6-yl)-[1,1']biphenyl-4-yl}-amine (compound 150)>

4'-(2-phenyl-benzooxazole-6-yl)-[1,1']biphenyl-4-yl-amine: 5.0 g, 2-(4-bromo-phenyl)-benzofuran: 7.9 g, tris(dibenzylideneacetone)dipalladium(0): 0.5 g, tri-t-butylphosphine: 0.2 g, and t-butoxy sodium: 4.0 g were placed in a reaction vessel purged with nitrogen and refluxed and stirred overnight in xylene solvent. After the mixture is allowed to cool, methanol was added to the system and the precipitated solid was filtered to obtain a crude product. The obtained crude product was purified by recrystallization using a chlorobenzene solvent to obtain a yellow powder: 5.4 g (yield: 52.6%) of N,N-bis(4-benzofuran-2-yl-phenyl)-N-{4'-(2-phenyl-benzooxazole-6-yl)-[1,1']biphenyl-4-yl}-amine (compound 150).

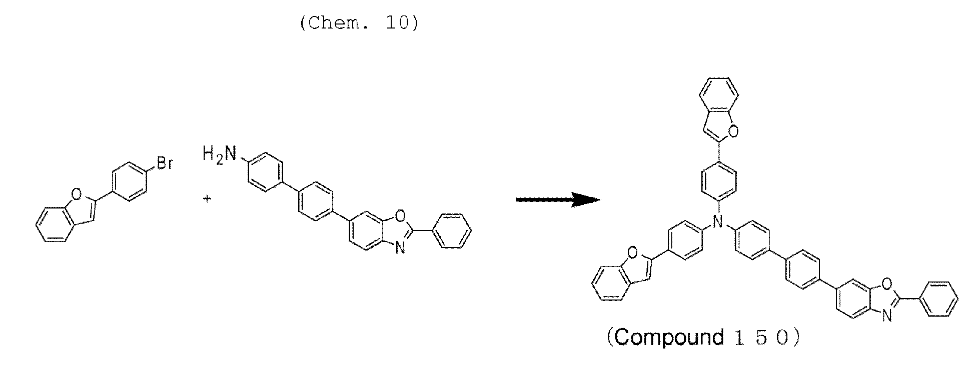

The structure of the obtained yellow powder was identified using NMR. The following 34 hydrogen signals were detected in ¹H-NMR(CDCl₃).

δ(ppm) = 8.29(2H), 7.84(2H), 7.79(4H), 7.72(4H), 7.66(1H), 7.61(2H), 7.56(5H), 7.52(2H), 7.32-7.20(10H), and 6.95(2H).

### (Example 8)

### <Synthesis of N-(4-benzooxazole-2-yl-phenyl)-N-(4-naphthalene-2-yl-phenyl)-N-{4-(2-phenyl-benzooxazole-6-yl)-phenyl}-amine (compound 2)>

N-(4-benzooxazole-2-yl-phenyl)-N-(4-naphthalene-2-yl-phenyl)-amine: 5.8 g, 6-(4-chloro-phenyl)-2-phenyl-benzooxazole: 4.7 g, tris(dibenzylideneacetone)dipalladium(0): 0.3 g, tri-t-butylphosphine: 0.1 g, and t-butoxy sodium: 2.7 g were placed in a reaction vessel purged with nitrogen and refluxed and stirred overnight in toluene solvent. After the mixture is allowed to cool, methanol was added to the system and the precipitated solid was filtered to obtain a crude product. The obtained crude product was purified by crystallization using a mixed solvent of chlorobenzene and acetone to obtain a yellow powder: 5.3 g (yield: 55.8%) of N-(4-benzooxazole-2-yl-phenyl)-N-(4-naphthalene-2-yl-phenyl)-N-{4-(2-phenyl-benzooxazole-6-yl)-phenyl}-amine (compound 2).

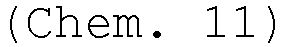

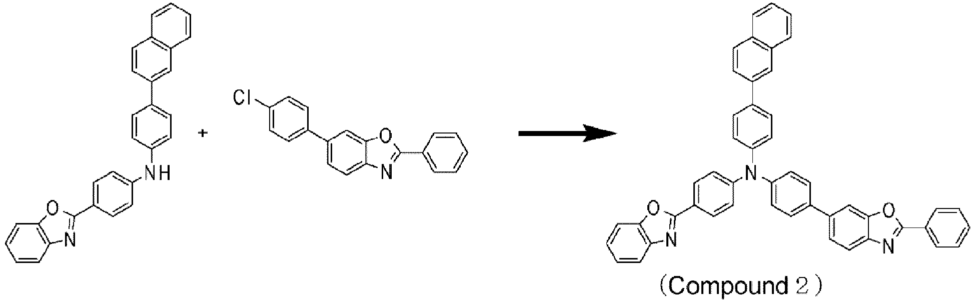

The structure of the obtained yellow powder was identified using NMR. The following 31 hydrogen signals were detected in ¹H-NMR(CDCl₃).

δ(ppm) = 8.32(2H), 8.18(2H), 8.09(1H), 7.94(2H), 7.90(1H), 7.86(1H), 7.84(1H), 7.80(1H), 7.78(1H), 7.75(2H), 7.66(3H), 7.62-7.48(6H), 7.40-7.33(6H), and 7.31(2H).

### (Example 9)

### <Synthesis of N,N-bis(4-benzooxazole-2-yl-phenyl)-N-{4-(2-phenyl-benzooxazole-6-yl)-phenyl}-amine (compound 17)>

N,N-bis(4-benzooxazole-2-yl-phenyl)-amine: 7.1 g, 6-(4-chloro-phenyl)-2-phenyl-benzooxazole: 5.9 g, tris(dibenzylideneacetone)dipalladium(0): 0.3 g, tri-t-butylphosphine: 0.2 g, and t-butoxy sodium: 3.4 g were placed in a reaction vessel purged with nitrogen and refluxed and stirred overnight in toluene solvent. After the mixture is allowed to cool, methanol was added to the system and the precipitated solid was filtered to obtain a crude product. The obtained crude product was purified by crystallization using a mixed solvent of dichloromethane and acetone to obtain a yellow powder: 3.1 g (yield: 26.3%) of N,N-bis(4-benzooxazole-2-yl-phenyl)-N-{4-(2-phenyl-benzooxazole-6-yl)-phenyl}-amine (compound 17).

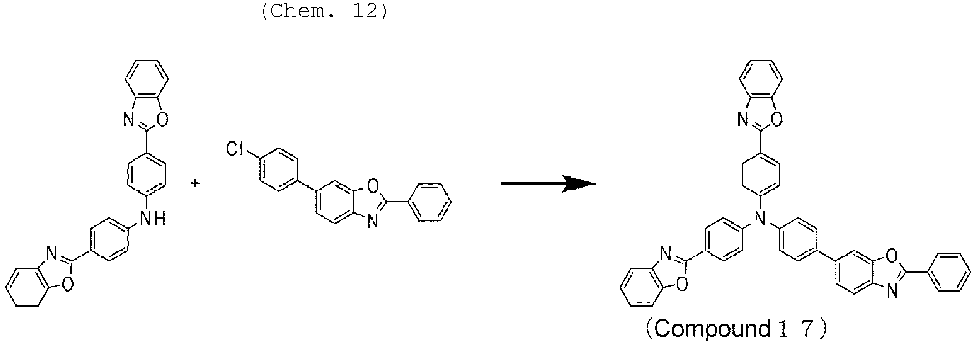

The structure of the obtained yellow powder was identified using NMR. The following 28 hydrogen signals were detected in ¹H-NMR(CDCl₃).

δ(ppm) = 8.31(2H), 8.21(4H), 7.85(1H), 7.84(1H), 7.79(2H), 7.68(2H), 7.65(1H), 7.62-7.53(5H), 7.37(4H), and 7.33(6H).

### (Example 10)

### <Synthesis of N-(4-benzothiazole-2-yl-phenyl)-N-(4-benzothiophene-2-yl-phenyl)-N-{4-(2-phenyl-benzooxazole-6-yl)-phenyl}-amine (compound 38)>

N-(4-benzothiophene-2-yl-phenyl)-N-{4-(2-phenyl-benzooxazole-6-yl)-phenyl}-amine: 6.1 g, 2-(4-bromophenyl)-benzothiazole: 3.9 g, tris(dibenzylideneacetone)dipalladium(0): 0.2 g, tri-t-butylphosphine: 0.1 g, and t-butoxy sodium: 1.8 g were placed in a reaction vessel purged with nitrogen and refluxed and stirred overnight in xylene solvent. After the mixture is allowed to cool, methanol was added to the system and the precipitated solid was filtered to obtain a crude product. The obtained crude product was purified by recrystallization using a chlorobenzene solvent to obtain a bright yellow powder: 6.8 g (yield: 78.3%) of N-(4-benzothiazole-2-yl-phenyl)-N-(4-benzothiophene-2-yl-phenyl)-N-{4-(2-phenyl-benzooxazole-6-yl)-phenyl}-amine (compound 38).

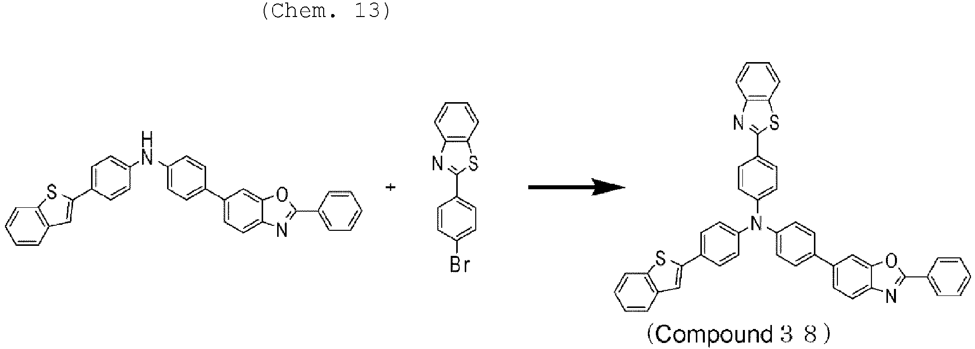

The structure of the obtained bright yellow powder was identified using NMR. The following 29 hydrogen signals were detected in ¹H-NMR(CDCl₃).

δ(ppm) = 8.31(2H), 8.08(1H), 8.03(2H), 7.91(1H), 7.85(2H), 7.83(1H), 7.80(1H), 7.70(2H), 7.65(3H), 7.58 (3H), 7.54(1H), 7.51(1H), 7.43-7.30(5H), and 7.28(4H).

### (Example 11)

### <Synthesis of N-(4-benzothiophene-2-yl-phenyl)-N-(4-naphthalene-2-yl-phenyl)-N-{4-(2-phenyl-benzooxazole-6-yl)-phenyl}-amine (compound 52)>

N-(4-benzooxazole-2-yl-phenyl)-N-(4-naphthalene-2-yl-phenyl)-amine: 10.0 g, 6-(4-chloro-phenyl)-2-phenyl-benzooxazole: 7.9 g, tris(dibenzylideneacetone)dipalladium(0): 0.4 g, tri-t-butylphosphine: 0.2 g, and t-butoxy sodium: 4.5 g were placed in a reaction vessel purged with nitrogen and refluxed and stirred overnight in toluene solvent. After the mixture is allowed to cool, methanol was added to the system and the precipitated solid was filtered to obtain a crude product. The obtained crude product was purified by crystallization using a mixed solvent of chlorobenzene and acetone to obtain a pale yellow powder: 12.1 g (yield: 74.2%) of N-(4-benzothiophene-2-yl-phenyl)-N-(4-naphthalene-2-yl-phenyl)-N-{4-(2-phenyl-benzooxazole-6-yl)-phenyl}-amine (compound 52).

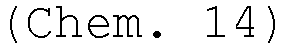

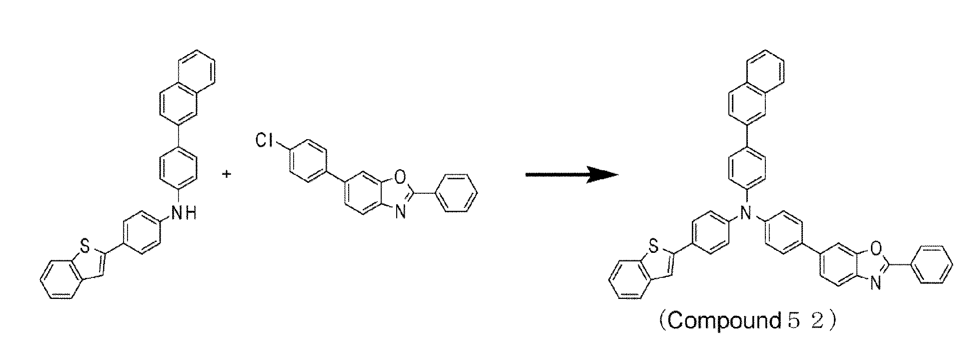

The structure of the obtained pale yellow powder was identified using NMR. The following 32 hydrogen signals were detected in ¹H-NMR(CDCl₃).

δ (ppm) = 8.55(1H), 8.26(1H), 8.09(1H), 7.94(2H), 7.90(1H), 7.85(2H), 7.80(3H), 7.72(2H), 7.68(4H), 7.63(2H), 7.52(3H), 7.41(2H), 7.37 (1H), 7.34(5H), and 7.27(2H).

### (Example 12)

### <Synthesis of N-(4-benzothiophene-2-yl-phenyl)-N-(4-[1,10]phenanthroline-2-yl-phenyl)-N-{4-(2-phenyl-benzooxazole-6-yl)-phenyl}-amine (compound 61)>

N-(4-benzothiophene-2-yl-phenyl)-N-{4-(2-phenyl-benzooxazole-6-yl)-phenyl}-amine: 12.6 g, 2-(4-bromophenyl)-[1,10]phenanthroline: 16.7 g, tris(dibenzylideneacetone)dipalladium(0): 0.9 g, tri-t-butylphosphine: 0.4 g, and t-butoxy sodium: 9.5 g were placed in a reaction vessel purged with nitrogen and refluxed and stirred overnight in toluene solvent. After the mixture is allowed to cool, methanol was added to the system and the precipitated solid was filtered to obtain a crude product. The obtained crude product was purified by crystallization using a mixed solvent of chlorobenzene and acetone to obtain a brown powder: 12.4 g (yield: 64.9%) of N-(4-benzothiophene-2-yl-phenyl)-N-(4-[1,10]phenanthroline-2-yl-phenyl)-N-{4-(2-phenyl-benzooxazole-6-yl)-phenyl}-amine (compound 61).

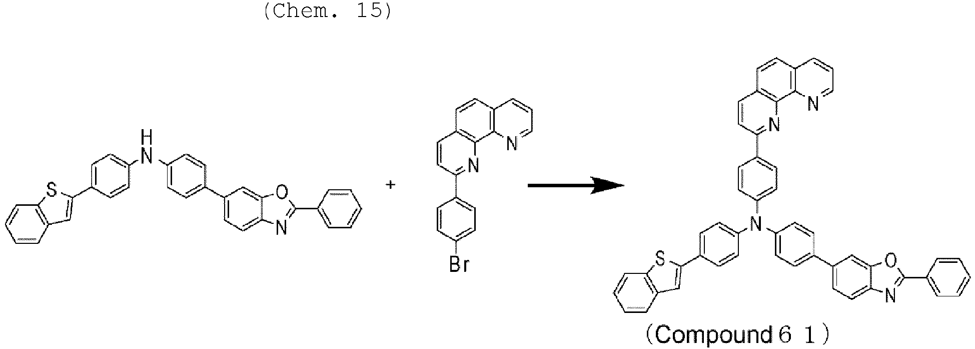

The structure of the obtained brown powder was identified using NMR. The following 32 hydrogen signals were detected in ¹H-NMR(CDCl₃).

δ(ppm) = 9.26(1H), 8.35-8.26(6H), 8.10(1H), 7.85(4H), 7.79(2H), 7.71-7.61(6H), 7.57(3H), 7.53(1H), 7.37(3H), 7.33(3H), and 7.28(2H).

### (Example 13)

### <Synthesis of N,N-bis(4-benzothiophene-2-yl-phenyl)-N-{4-(2-phenyl-benzooxazole-6-yl)-phenyl}-amine (compound 62)>

N,N-bis(4-benzothiophene-2-yl-phenyl)-amine: 7.6 g, 6-(4-chloro-phenyl)-2-phenyl-benzooxazole: 5.9 g, tris(dibenzylideneacetone)dipalladium(0): 0.3 g, tri-t-butylphosphine: 0.2 g, and t-butoxy sodium: 3.4 g were placed in a reaction vessel purged with nitrogen and refluxed and stirred overnight in toluene solvent. After the mixture is allowed to cool, methanol was added to the system and the precipitated solid was filtered to obtain a crude product. The obtained crude product was purified by crystallization using a mixed solution of toluene and acetone to obtain a yellow powder: 9.5 g (yield: 77.2%) of N,N-bis(4-benzothiophene-2-yl-phenyl)-N-{4-(2-phenyl-benzooxazole-6-yl)-phenyl}-amine (compound 62).

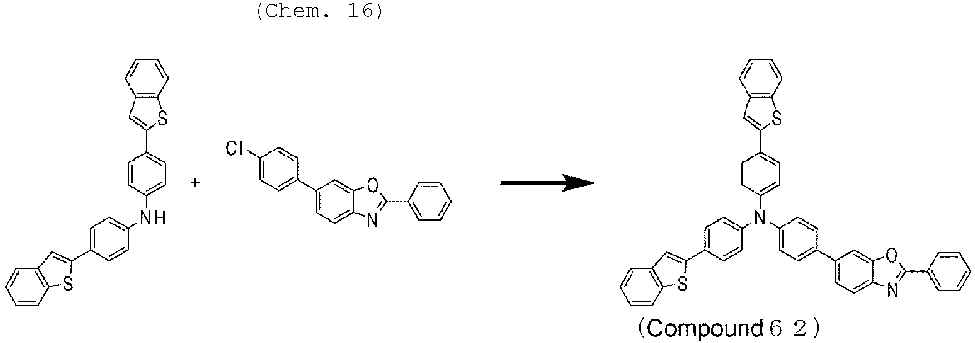

The structure of the obtained yellow powder was identified using NMR. The following 30 hydrogen signals were detected in ¹H-NMR(CDCl₃).

δ (ppm) = 8.31(2H), 7.85(3H), 7.82(1H), 7.79(2H), 7.68(4H), 7.65-7.60(3H), 7.60-7.55(3H), 7.52(2H), 7.35(4H), 7.30(2H), and 7.25(4H).

### (Example 14)

### <Synthesis of N-(4-benzooxazole-2-yl-phenyl)-N-(4-benzothiophene-2-yl-phenyl)-N-{4-(2-naphthalene-2-yl-benzooxazole-6-yl)-phenyl}-amine (compound 71)>

N-(4-benzooxazole-2-yl-phenyl)-N-(4-benzothiophene-2-yl-phenyl)-amine: 10.0 g, 6-(4-chlorophenyl)-2-naphthalene-2-yl-benzooxazole: 9.4 g, tris(dibenzylideneacetone)dipalladium(0): 0.4 g, tri-t-butylphosphine: 0.2 g, and t-butoxy sodium: 3.4 g were placed in a reaction vessel purged with nitrogen and refluxed and stirred overnight in xylene solvent. After the mixture is allowed to cool, methanol was added to the system and the precipitated solid was filtered to obtain a crude product. The obtained crude product was purified by crystallization using a mixed solvent of chlorobenzene and acetone to obtain a yellow powder: 16.5 g (yield: 93.6%) of N-(4-benzooxazole-2-yl-phenyl)-N-(4-benzothiophene-2-yl-phenyl)-N-{4-(2-naphthalene-2-yl-benzooxazole-6-yl)-phenyl}-amine (compound 71).

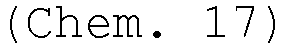

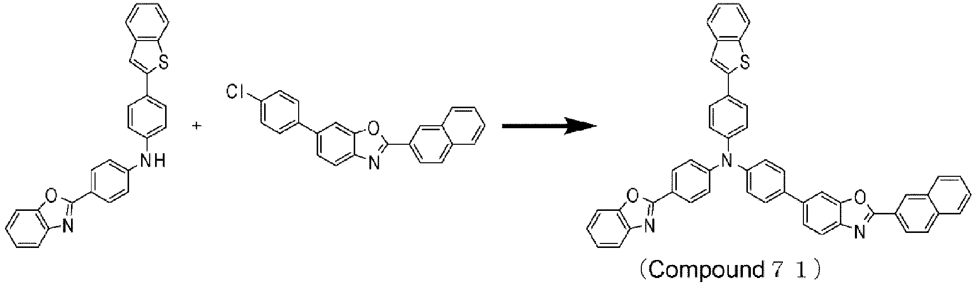

The structure of the obtained yellow powder was identified using NMR. The following 31 hydrogen signals were detected in ¹H-NMR(CDCl₃).

δ(ppm) = 8.83(1H), 8.37(1H), 8.19(2H), 8.03(1H), 8.02(1H), 7.93(1H), 7.88(1H), 7.87(1H), 7.85(1H), 7.79(2H), 7.71(2H), 7.69-7.57(6H), 7.54(1H), and 7.41-7.28(10H).

### (Example 15)

### <Synthesis of N-(4-benzofuran-2-yl-phenyl)-N-(4-benzothiophene-2-yl-phenyl)-N-{4-(2-naphthalene-2-yl-benzooxazole-6-yl)-phenyl}-amine (compound 79)>

N-(4-benzofuran-2-yl-phenyl)-N-(4-benzothiophene-2-yl-phenyl)-amine: 10.0 g, 6-(4-chloro-phenyl)-2-naphthalene-2-yl-benzooxazole: 9.4 g, tris(dibenzylideneacetone)dipalladium(0): 0.4 g, tri-t-butylphosphine: 0.2 g, and t-butoxy sodium: 3.5 g were placed in a reaction vessel purged with nitrogen and refluxed and stirred overnight in xylene solvent. After the mixture is allowed to cool, methanol was added to the system and the precipitated solid was filtered to obtain a crude product. The obtained crude product was purified by recrystallization using a chlorobenzene solvent to obtain a yellow powder: 16.1 g (yield: 91.2%) of N-(4-benzofuran-2-yl-phenyl)-N-(4-benzothiophene-2-yl-phenyl)-N-{4-(2-naphthalene-2-yl-benzooxazole-6-yl)-phenyl}-amine (compound 79).

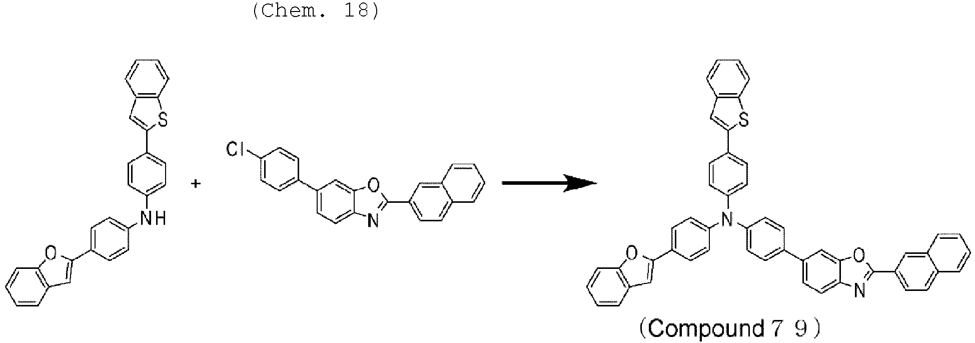

The structure of the obtained yellow powder was identified using NMR. The following 32 hydrogen signals were detected in ¹H-NMR(CDCl₃).

δ(ppm) = 8.83(1H), 8.37(1H), 8.04(1H), 8.02(1H), 7.94(1H), 7.88(1H), 7.85(2H), 7.83(2H), 7.79(1H), 7.71-7.58(8H), 7.55(1H), 7.53(1H), 7.42-7.22(10H), and 6.98(1H).

### (Example 16)

### <Synthesis of N-(4-benzofuran-2-yl-phenyl)-N-(4-benzooxazole-2-yl-phenyl)-N-{4-(2-phenyl-benzothiazole-6-yl)-phenyl}-amine (compound 101)>

N-(4-benzofuran-2-yl-phenyl)-N-(4-benzooxazole-2-yl-phenyl)-amine: 9.8 g, 6-(4-chloro-phenyl)-2-phenyl-benzothiazole: 8.6 g, tris(dibenzylideneacetone)dipalladium(0): 0.5 g, tri-t-butylphosphine: 0.2 g, and t-butoxy sodium: 3.5 g were placed in a reaction vessel purged with nitrogen refluxed and stirred overnight in xylene solvent. After the mixture is allowed to cool, the filtrate obtained by filtration was concentrated to obtain a crude product. The obtained crude product was purified by crystallization using a mixed solution of toluene and acetone to obtain a yellow powder: 3.1 g (yield: 18.5%) of N-(4-benzofuran-2-yl-phenyl)-N-(4-benzooxazole-2-yl-phenyl)-N-{4-(2-phenyl-benzothiazole-6-yl)-phenyl}-amine (compound 101).

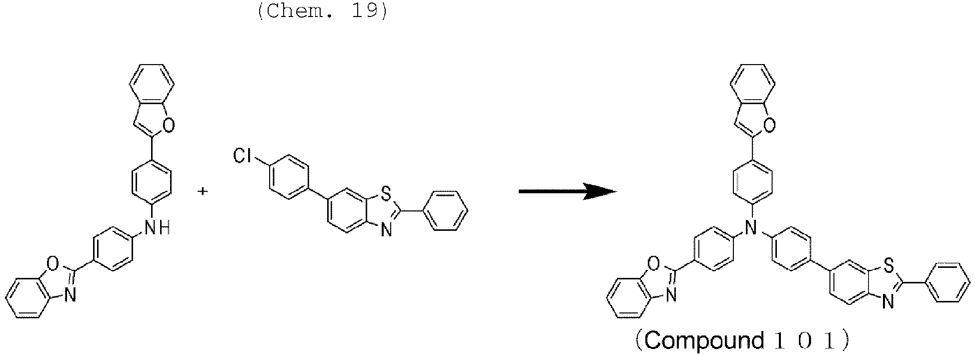

The structure of the obtained yellow powder was identified using NMR. The following 29 hydrogen signals were detected in ¹H-NMR(CDCl₃).

δ (ppm) = 8.21-8.12(6H), 7.86(2H), 7.78(2H), 7.68(2H), 7.60(2H), 7.54(4H), 7.41-7.23(10H), and 7.01(1H).

### (Example 17)

### <Synthesis of N-(4-benzofuran-2-yl-phenyl)-N-(4-benzooxazole-2-yl-phenyl)-N-{4'-(2-phenyl-benzooxazole-6-yl)-[1,1']biphenyl-4-yl}-amine (compound 137)>

N-(4-benzofuran-2-yl-phenyl)-N-(4-benzooxazole-2-yl-phenyl)-amine: 10.0 g, 6-(4'-bromo-[1,1']biphenyl-4-yl)-2-phenyl-benzooxazole: 11.7 g, tris(dibenzylideneacetone)dipalladium(0): 0.5 g, tri-t-butylphosphine: 0.2 g, and t-butoxy sodium: 3.6 g were placed in a reaction vessel purged with nitrogen and refluxed and stirred overnight in toluene solvent. After the mixture is allowed to cool, methanol was added to the system and the precipitated solid was filtered to obtain a crude product. The obtained crude product was purified by crystallization using a mixed solvent of chlorobenzene and acetone to obtain a yellow powder: 14.3 g (yield: 76.4%) of N-(4-benzofuran-2-yl-phenyl)-N-(4-benzooxazole-2-yl-phenyl)-N-{4'-(2-phenyl-benzooxazole-6-yl)-[1,1']biphenyl-4-yl}-amine (compound 137).

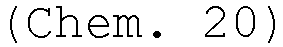

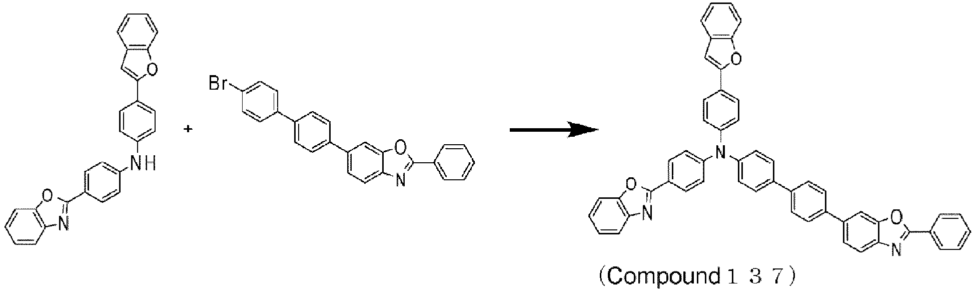

The structure of the obtained yellow powder was identified using NMR. The following 34 hydrogen signals were detected in ¹H-NMR(CDCl₃).

δ (ppm) = 8.32(2H), 8.18(2H), 7.87(2H), 7.85(2H), 7.80-7.73(5H), 7.69(1H), 7.67(2H), 7.64-7.53(6H), 7.37(2H), 7.35-7.28(8H), 7.27(1H), and 7.01(1H).

### (Example 18)

### <Synthesis of N-(4-benzooxazole-2-yl-phenyl)-N-(4-benzothiophene-2-yl-phenyl)-N-{4'-(2-phenyl-benzooxazole-6-yl)-[1,1']biphenyl-4-yl}-amine (compound 138)>

N-(4-benzooxazole-2-yl-phenyl)-N-(4-benzothiophene-2-yl-phenyl)-amine: 10.0 g, 6-(4'-bromo-[1,1']biphenyl-4-yl)-2-phenyl-benzooxazole: 11.2 g, tris(dibenzylideneacetone)dipalladium(0): 0.4 g, tri-t-butylphosphine: 0.2 g, and t-butoxy sodium: 3.4 g were placed in a reaction vessel purged with nitrogen and refluxed and stirred overnight in toluene solvent. After the mixture is allowed to cool, methanol was added to the system and the precipitated solid was filtered to obtain a crude product. The obtained crude product was purified by crystallization using a mixed solvent of chlorobenzene and acetone to obtain a yellow powder: 15.6 g (yield: 85.5%) of N-(4-benzooxazole-2-yl-phenyl)-N-(4-benzothiophene-2-yl-phenyl)-N-{4'-(2-phenyl-benzooxazole-6-yl)-[1,1']biphenyl-4-yl}-amine (compound 138).

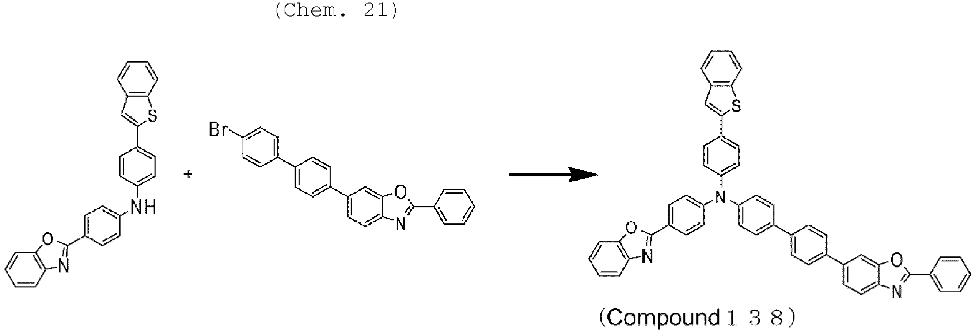

The structure of the obtained yellow powder was identified using NMR. The following 34 hydrogen signals were detected in ¹H-NMR(CDCl₃).

δ(ppm) = 8.32(2H), 8.18(2H), 7.90-7.83(3H), 7.82-7.73(6H), 7.70(3H), 7.67(2H), 7.58(4H), 7.54(1H), and 7.41-7.25(11H).

### (Example 19)

### <Synthesis of N,N-(bis(4-benzofuran-2-yl-phenyl)-N-{4'-(2-phenyl-benzothiazole-6-yl)-[1,1']biphenyl-4-yl}-amine (compound 155)>

4'-(2-phenyl-benzothiazole-6-yl)-[1,1']biphenyl-4-yl-amine: 8.0 g, 2-(4-bromo-phenyl)-benzofuran: 12.7 g, tris(dibenzylideneacetone)dipalladium(0): 0.8 g, tri-t-butylphosphine: 0.3 g, and t-butoxy sodium: 6.1 g were placed in a reaction vessel purged with nitrogen and refluxed and stirred overnight in toluene solvent. After the mixture is allowed to cool, methanol was added to the system and the precipitated solid was filtered to obtain a crude product. The obtained crude product was purified by recrystallization using a chlorobenzene solvent to obtain a yellow powder: 10.9 g (yield: 67.3%) of N,N-(bis(4-benzofuran-2-yl-phenyl)-N-{4'-(2-phenyl-benzothiazole-6-yl)-[1,1'Jbiphenyl-4-yl}-amine (compound 155).

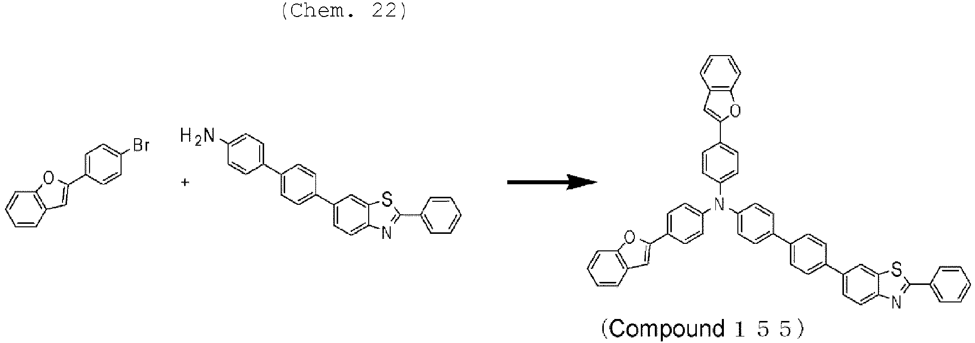

The structure of the obtained yellow powder was identified using NMR. The following 34 hydrogen signals were detected in ¹H-NMR(CDCl₃).

δ(ppm) = 8.17(3H), 8.14(1H), 7.82(5H), 7.76(4H), 7.64(2H), 7.60(2H), 7.57-7.51(5H), 7.35-7.22(10H), and 6.98(2H).

### (Example 20)

### <Synthesis of N-(4-benzooxazole-2-yl-phenyl)-N-phenyl-N-{4'-(2-phenyl-benzooxazole-6-yl)-[1,1']biphenyl-4-yl}-amine (compound 171)>

N-phenyl-N-{4'-(2-phenyl-benzooxazole-6-yl)-[1,1']biphenyl-4-yl}-amine: 12.0 g, 2-(4-bromo-phenyl)-benzooxazole: 8.3 g, tris(dibenzylideneacetone)dipalladium(0): 0.5 g, tri-t-butylphosphine: 0.2 g, and t-butoxy sodium: 5.3 g were placed in a reaction vessel purged with nitrogen and refluxed and stirred overnight in toluene solvent. After the mixture is allowed to cool, methanol was added to the system and the precipitated solid was filtered to obtain a crude product. The obtained crude product was purified by crystallization using a mixed solvent of dichloromethane and acetone to obtain a yellow powder: 9.5 g (yield: 54.9%) of N-(4-benzooxazole-2-yl-phenyl)-N-phenyl-N-{4'-(2-phenyl-benzooxazole-6-yl)-[1,1']biphenyl-4-yl}-amine (compound 171).

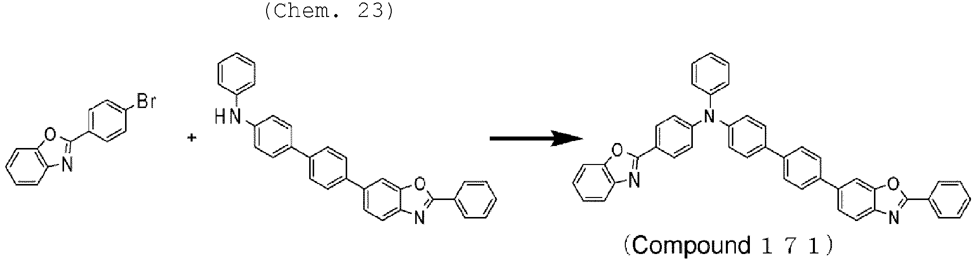

The structure of the obtained yellow powder was identified using NMR. The following 29 hydrogen signals were detected in ¹H-NMR(CDCl₃).

δ(ppm) = 8.32(2H), 8.14(2H), 7.88(1H), 7.86(1H), 7.80-7.71(5H), 7.69(1H), 7.64 (2H), 7.61-7.55(4H), 7.38(3H), 7.35(1H), 7.31-7.24(4H), 7.22(2H), and 7.19(1H).

### (Example 21)

### <Synthesis of N-(4-benzooxazole-2-yl-phenyl)-N-phenyl-N-{4'-(2-naphthalene-2-yl-benzooxazole-6-yl)-[1,1']biphenyl-4-yl}-amine (compound 173)>

N-phenyl-N-{4'-(2-naphthalene-2-yl-benzooxazole-6-yl)-[1,1']biphenyl-4-yl}-amine: 11.0 g, 2-(4-bromophenyl)-benzooxazole: 6.8 g, tris(dibenzylideneacetone)dipalladium(0): 0.4 g, tri-t-butylphosphine: 0.2 g, t-butoxy sodium: 4.3 g were placed in a reaction vessel purged with nitrogen and refluxed and stirred overnight in toluene solvent. After the mixture is allowed to cool, methanol was added to the system and the precipitated solid was filtered to obtain a crude product. The obtained crude product was purified by crystallization using a mixed solution of toluene and acetone to obtain a yellow powder: 12.8 g (yield: 83.6%) of N-(4-benzooxazole-2-yl-phenyl)-N-phenyl-N-{4'-(2-naphthalene-2-yl-benzooxazole-6-yl)-[1,1']biphenyl-4-yl}-amine (compound 173).

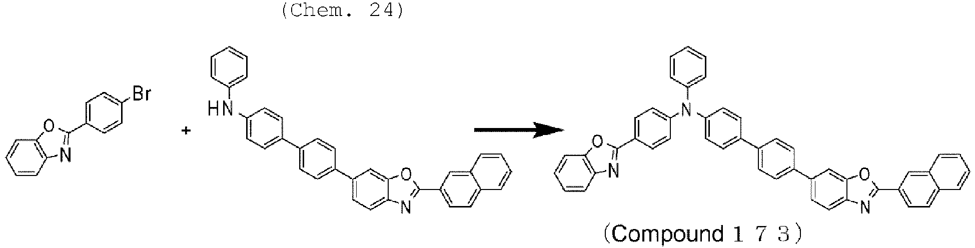

The structure of the obtained yellow powder was identified using NMR. The following 31 hydrogen signals were detected in ¹H-NMR(CDCl₃).

δ(ppm) = 8.83(1H), 8.37 (1H), 8.14(2H), 8.04(1H), 8.02(1H), 7.94(1H), 7.90(1H), 7.89(1H), 7.80-7.72(5H), 7.70(1H), 7.67-7.55(5H), 7.43-7.31(4H), 7.31-7.25(4H), 7.23(2H), and 7.20(1H).

### (Example 22)

### <Synthesis of N-(4'-benzooxazole-2-yl-[1,1']biphenyl-4-yl)-N-phenyl-N-{4'-(2-phenyl-benzooxazole-6-yl)-[1,1']biphenyl-4-yl}-amine (compound 177)>

N-phenyl-N-{4'-(2-phenyl-benzooxazole-6-yl)-[1,1']biphenyl-4-yl}-amine: 10.0 g, 2-(4'-chloro-[1,1']biphenyl-4-yl)-benzooxazole: 7.7 g, tris(dibenzylideneacetone)dipalladium(0): 0.4 g, tri-t-butylphosphine: 0.2 g, and t-butoxy sodium: 4.4 g were placed in a reaction vessel purged with nitrogen and refluxed and stirred overnight in toluene solvent. After the mixture is allowed to cool, methanol was added to the system and the precipitated solid was filtered to obtain a crude product. The obtained crude product was purified by crystallization using a mixed solvent of dichloromethane and acetone to obtain a yellow powder: 9.9 g (yield: 61.5%) of N-(4'-benzooxazole-2-yl-[1,1']biphenyl-4-yl)-N-phenyl-N-{4'-(2-phenyl-benzooxazole-6-yl)-[1,1']biphenyl-4-yl}-amine (compound 177).

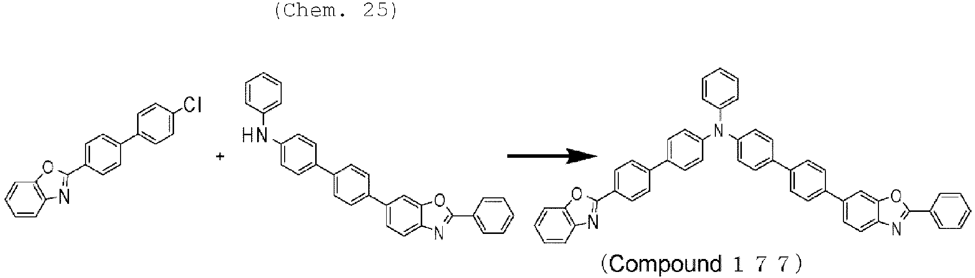

The structure of the obtained yellow powder was identified using NMR. The following 33 hydrogen signals were detected in ¹H-NMR(CDCl₃).

δ(ppm) = 8.34(2H), 8.32(2H), 7.87(1H), 7.86(1H), 7.82(1H), 7.78(2H), 7.74(4H), 7.68(1H), 7.65-7.55(8H), 7.42-7.32(4H), 7.28-7.23(6H), and 7.14(1H).

### (Example 23)

### <Synthesis of N-(4'-benzooxazole-2-yl-[1,1']biphenyl-4-yl)-N-phenyl-N-{4-(2-phenyl-benzooxazole-6-yl)-phenyl}-amine (compound 180)>

N-phenyl-N-{4-(2-phenyl-benzooxazole-6-yl)-phenyl}-amine: 6.5 g, 2-(4'-chloro-[1,1']biphenyl-4-yl)-benzooxazole: 6.0 g, tris(dibenzylideneacetone)dipalladium(0): 0.3 g, tri-t-butylphosphine: 0.2 g, and t-butoxy sodium: 3.4 g were placed in a reaction vessel purged with nitrogen and refluxed and stirred overnight in xylene solvent. After the mixture is allowed to cool, methanol was added to the system and the precipitated solid was filtered to obtain a crude product. The obtained crude product was purified by crystallization using a mixed solution of toluene and acetone to obtain a yellow powder: 6.1 g (yield: 53.7%) of N-(4'-benzooxazole-2-yl-[1,1']biphenyl-4-yl)-N-phenyl-N-{4-(2-phenyl-benzooxazole-6-yl)-phenyl}-amine (compound 180).

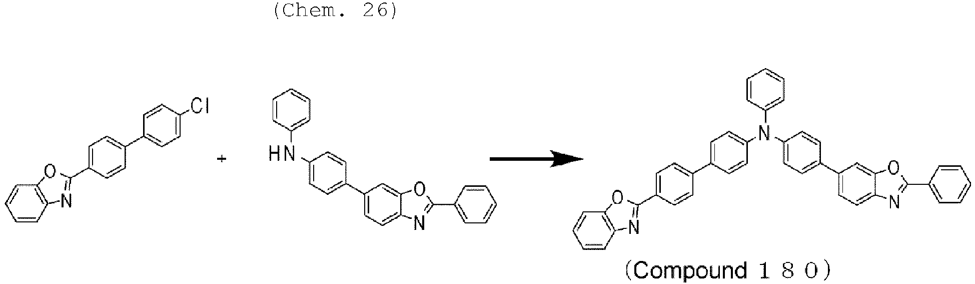

The structure of the obtained yellow powder was identified using NMR. The following 29 hydrogen signals were detected in ¹H-NMR(CDCl₃).

δ(ppm) = 8.34(2H), 8.31(2H), 7.83(3H), 7.77(2H), 7.65-7.59(5H), 7.59-7.53(4H), 7.42-7.33(4H), 7.28-7.22(6H), and 7.14(1H).

### (Example 24)

### <Synthesis of N-(4'-benzofuran-2-yl-[1,1']biphenyl-4-yl)-N-phenyl-N-{4-(2-phenyl-benzooxazole-6-yl)-phenyl}-amine (compound 182)>

N-phenyl-N-{4-(2-phenyl-benzooxazole-6-yl)-phenyl}-amine: 6.5 g, 2-(4'-chloro-[1,1']biphenyl-4-yl)-benzofuran: 6.0 g, tris(dibenzylideneacetone)dipalladium(0): 0.3 g, tri-t-butylphosphine: 0.2 g, and t-butoxy sodium: 3.4 g were placed in a reaction vessel purged with nitrogen and refluxed and stirred overnight in xylene solvent. After the mixture is allowed to cool, methanol was added to the system and the precipitated solid was filtered to obtain a crude product. The obtained crude product was purified by crystallization using a mixed solution of toluene and acetone to obtain a pale yellow powder: 5.4 g (yield: 47.7%) of N-(4'-benzofuran-2-yl-[1,1']biphenyl-4-yl)-N-phenyl-N-{4-(2-phenyl-benzooxazole-6-yl)-phenyl}-amine (compound 182).

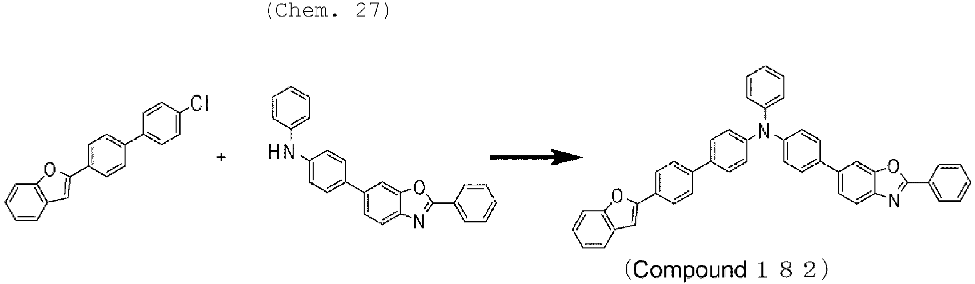

The structure of the obtained pale yellow powder was identified using NMR. The following 30 hydrogen signals were detected in ¹H-NMR(CDCl₃).

δ (ppm) = 8.31(2H), 7.96(2H), 7.84(1H), 7.81(1H), 7.71(2H), 7.66-7.54(10H), 7.39-7.29(3H), 7.28-7.22(7H), 7.13(1H), and 7.08(1H).

### (Example 25)

### <Synthesis of N-(4'-benzooxazole-2-yl-[1,1']biphenyl-4-yl)-N-phenyl-N-{4-(2-naphthalene-2-yl-benzooxazole-6-yl)-phenyl}-amine (compound 184)>

N-phenyl-N-{4-(2-naphthalene-2-yl-benzooxazole-6-yl)-phenyl}-amine: 6.3 g, 2-(4'-chloro-[1,1']biphenyl-4-yl)-benzooxazole: 5.1 g, tris(dibenzylideneacetone)dipalladium(0): 0.3 g, tri-t-butylphosphine: 0.1 g, and t-butoxy sodium: 2.9 g were placed in a reaction vessel purged with nitrogen and refluxed and stirred overnight in xylene solvent. After the mixture is allowed to cool, methanol was added to the system and the precipitated solid was filtered to obtain a crude product. The obtained crude product was purified by crystallization using a mixed solution of toluene and acetone to obtain a yellow powder: 3.4 g (yield: 32.7%) of N-(4'-benzooxazole-2-yl-[1,1']biphenyl-4-yl)-N-phenyl-N-{4-(2-naphthalene-2-yl-benzooxazole-6-yl)-phenyl}-amine (compound 184).

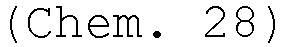

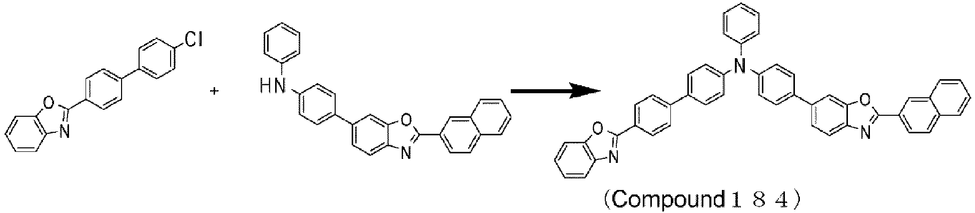

The structure of the obtained yellow powder was identified using NMR. The following 31 hydrogen signals were detected in ¹H-NMR(CDCl₃).

δ(ppm) = 8.81(1H), 8.36(1H), 8.34(2H), 8.02(1H), 8.01(1H), 7.93(1H), 7.86(1H)7.84 (1H), 7.82(1H), 7.78(2H), 7.66-7.56(8H), 7.42-7.33(4H), 7.36-7.23(6H), and 7.15(1H).

### (Example 26)

### <Synthesis of N-(4-benzofuran-2-yl-phenyl)-N-(4-benzooxazole-2-yl-phenyl)-N-{4-(2-furan-2-yl-benzooxazole-6-yl)-phenyl}-amine (compound 188)>

N-(4-benzofuran-2-yl-phenyl)-N-(4-benzooxazole-2-yl-phenyl)-amine: 8.0 g, 6-(4-chloro-phenyl)-2-(furan-2-yl)-benzooxazole: 6.5 g, tris(dibenzylideneacetone)dipalladium(0): 0.4 g, tri-t-butylphosphine: 0.2 g, and t-butoxy sodium: 2.9 g were placed in a reaction vessel purged with nitrogen and refluxed and stirred overnight in xylene solvent. After the mixture is allowed to cool, the filtrate obtained by filtration was concentrated to obtain a crude product. The obtained crude product was purified by crystallization using a mixed solution of toluene and acetone to obtain a yellow powder: 8.4 g (yield: 63.9%) of N-(4-benzofuran-2-yl-phenyl)-N-(4-benzooxazole-2-yl-phenyl)-N-{4-(2-furan-2-yl-benzooxazole-6-yl)-phenyl}-amine (compound 188).

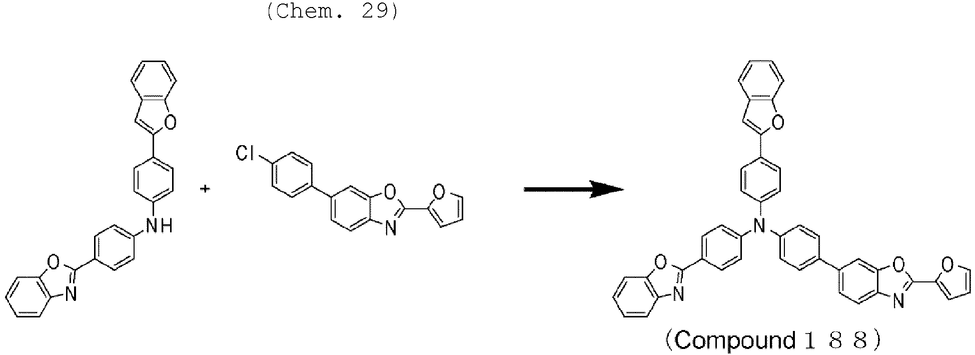

The structure of the obtained yellow powder was identified using NMR. The following 27 hydrogen signals were detected in ¹H-NMR(CDCl₃).

δ(ppm) = 8.18(2H), 7.85(1H), 7.83(1H), 7.80(1H), 7.78(1H), 7.71(1H), 7.67-7.57(5H), 7.55(1H), 7.36(2H), 7.35-7.28(9H), 7.26(1H), 7.01(1H), and 6.66(1H).

### (Example 27)

### <Synthesis of N-(4-benzofuran-2-yl-phenyl)-N-(4-benzothiophene-2-yl-phenyl)-N-{4-(2-furan-2-yl-benzooxazole-6-yl)-phenyl}-amine (compound 189)>

N-(4-benzofuran-2-yl-phenyl)-N-(4-benzothiophene-2-yl-phenyl)-amine: 7.2 g, 6-(4-chloro-phenyl)-2-(furan-2-yl)-benzooxazole: 5.6 g, tris(dibenzylideneacetone)dipalladium(0): 0.3 g, tri-t-butylphosphine: 0.1 g, and t-butoxy sodium: 2.5 g were placed in a reaction vessel purged with nitrogen and refluxed and stirred overnight in xylene solvent. After the mixture is allowed to cool, methanol was added to the system and the precipitated solid was filtered to obtain a crude product. The obtained crude product was purified by crystallization using a mixed solvent of chlorobenzene and acetone to obtain a yellow powder: 5.4 g (yield: 45.9%) of N-(4-benzofuran-2-yl-phenyl)-N-(4-benzothiophene-2-yl-phenyl)-N-{4-(2-furan-2-yl-benzooxazole-6-yl)-phenyl}-amine (compound 189).

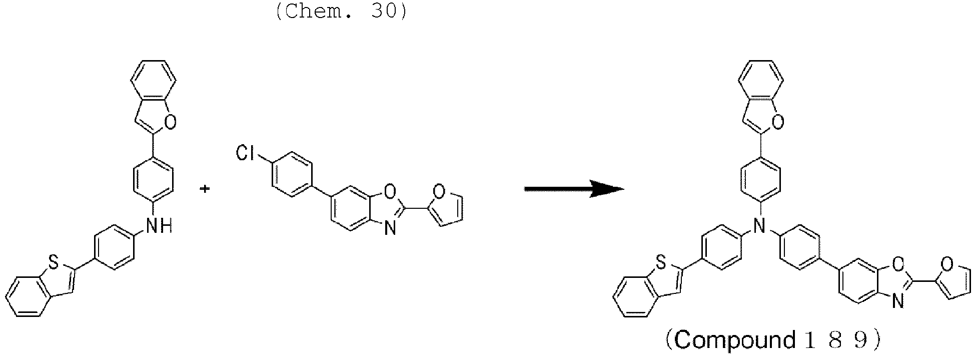

The structure of the obtained yellow powder was identified using NMR. The following 28 hydrogen signals were detected in ¹H-NMR(CDCl₃).

δ(ppm) = 7.88-7.76(6H), 7.72(1H), 7.68(2H), 7.64(1H), 7.62-7.58(3H), 7.54(1H), 7.52(1H), 7.41-7.23(11H), 6.98(1H), and 6.66(1H).

### (Example 28)

### <Synthesis of N-(4-benzofuran-2-yl-phenyl)-N-(4-benzooxazole-2-yl-phenyl)-N-{4-(2-thiophene-2-yl-benzooxazole-6-yl)-phenyl}-amine (compound 190)>

N-(4-benzofuran-2-yl-phenyl)-N-(4-benzooxazole-2-yl-phenyl)-amine: 9.0 g, 6-(4-chloro-phenyl)-2-(thiophene-2-yl)-benzooxazole: 7.7 g, tris(dibenzylideneacetone)dipalladium(0): 0.4 g, tri-t-butylphosphine: 0.2 g, and t-butoxy sodium: 3.2 g were placed in a reaction vessel purged with nitrogen and refluxed and stirred overnight in xylene solvent. After the mixture is allowed to cool, the filtrate obtained by filtration was concentrated to obtain a crude product. The obtained crude product was purified by crystallization using a mixed solvent of chlorobenzene and acetone to obtain a yellow powder: 10.3 g (yield: 68.0%) of N-(4-benzofuran-2-yl-phenyl)-N-(4-benzooxazole-2-yl-phenyl)-N-{4-(2-thiophene-2-yl-benzooxazole-6-yl)-phenyl}-amine (compound 190).

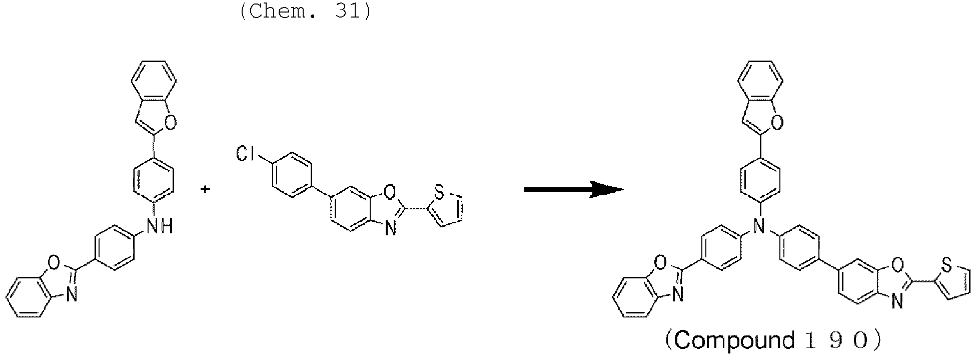

The structure of the obtained yellow powder was identified using NMR. The following 27 hydrogen signals were detected in ¹H-NMR(CDCl₃).

δ(ppm) = 8.18(2H), 7.96(1H), 7.86(1H), 7.81(1H), 7.79(1H), 7.78(1H), 7.67-7.57(6H), 7.55(1H), 7.37(2H), 7.35-7.25(9H), 7.23(1H), and 7.01(1H).

### (Example 29)

### <Synthesis of N-(4-benzofuran-2-yl-phenyl)-N-(4-benzothiophene-2-yl-phenyl)-N-{4-(2-thiophene-2-yl-benzooxazole-6-yl)-phenyl}-amine (compound 191)>

N-(4-benzofuran-2-yl-phenyl)-N-(4-benzothiophene-2-yl-phenyl)-amine: 7.2 g, 6-(4-chloro-phenyl)-2-(furan-2-yl)-benzooxazole: 5.9 g, tris(dibenzylideneacetone)dipalladium(0): 0.3 g, tri-t-butylphosphine: 0.1 g, and t-butoxy sodium: 2.5 g were placed in a reaction vessel purged with nitrogen and refluxed and stirred overnight in xylene solvent. After the mixture is allowed to cool, methanol was added to the system and the precipitated solid was filtered to obtain a crude product. The obtained crude product was purified by crystallization using a mixed solvent of chlorobenzene and acetone to obtain a yellow powder: 7.5 g (yield: 62.8%) of N-(4-benzofuran-2-yl-phenyl)-N-(4-benzothiophene-2-yl-phenyl)-N-{4-(2-thiophene-2-yl-benzooxazole-6-yl)-phenyl}-amine (compound 191).

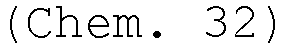

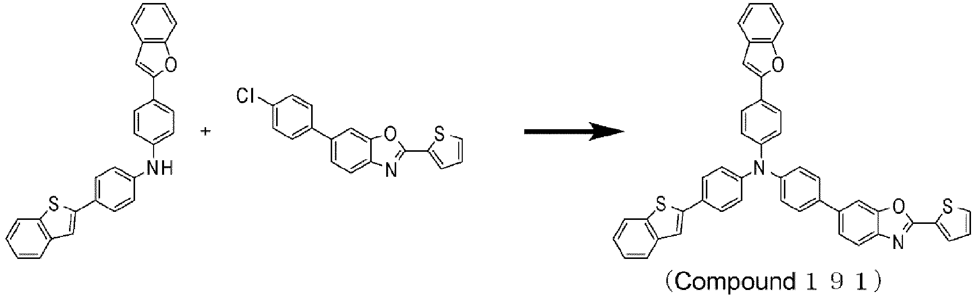

The structure of the obtained yellow powder was identified using NMR. The following 28 hydrogen signals were detected in ¹H-NMR(CDCl₃).

δ (ppm) = 7.96(1H), 7.87-7.75(6H), 7.68(2H), 7.61(5H), 7.55(1H), 7.52(1H), 7.38(1H), 7.36-7.21(10H), and 6.97(1H).

### (Example 30)

### <Synthesis of N-(4-naphthalene-2-yl-phenyl)-N-(2-phenyl-benzooxazole-6-yl)-N-{4-(2-phenyl-benzooxazole-6-yl)-phenyl}-amine (compound 192)>

N-(4-naphthalene-2-yl-phenyl)-N-(2-phenyl-benzooxazole-6-yl)-amine: 6.2 g, 6-(4-chloro-phenyl)-2-phenyl-benzooxazole: 5.1 g, tris(dibenzylideneacetone)dipalladium(0): 0.3 g, tri-t-butylphosphine: 0.1 g, and t-butoxy sodium: 2.9 g were placed in a reaction vessel purged with nitrogen and refluxed and stirred overnight in toluene solvent. After the mixture is allowed to cool, methanol was added to the system and the precipitated solid was filtered to obtain a crude product. The obtained crude product was purified by crystallization using a mixed solution of toluene and acetone to obtain a yellow powder: 6.4 g (yield: 62.7%) of N-(4-naphthalene-2-yl-phenyl)-N-(2-phenyl-benzooxazole-6-yl)-N-{4-(2-phenyl-benzooxazole-6-yl)-phenyl}-amine (compound 192).

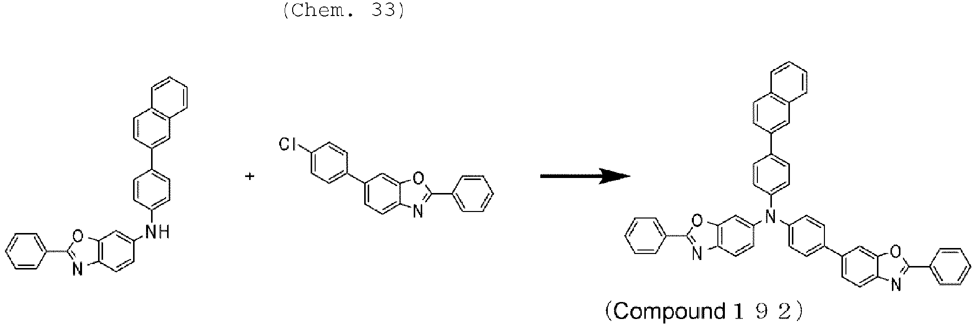

The structure of the obtained yellow powder was identified using NMR. The following 31 hydrogen signals were detected in ¹H-NMR(CDCl₃).

δ(ppm) = 8.31(2H), 8.25(2H), 8.08(1H), 7.93(2H), 7.89(1H), 7.84 (1H), 7.82(1H), 7.79(1H), 7.72(3H), 7.64(1H), 7.62(2H), 7.60-7.47(8H), 7.46(1H), and 7.35-7.25(5H).

### (Example 31)

### <Synthesis of N-(4-dibenzofuran-3-yl-phenyl)-N-(2-phenyl-benzooxazole-6-yl)-N-{4-(2-phenyl-benzooxazole-6-yl)-phenyl}-amine (compound 193)>

N-(4-dibenzofuran-3-yl-phenyl)-N-(2-phenyl-benzooxazole-6-yl)-amine: 8.8 g, 6-(4-chloro-phenyl)-2-phenyl-benzooxazole: 6.5 g, tris(dibenzylideneacetone)dipalladium(0): 0.4 g, tri-t-butylphosphine: 0.2 g, and t-butoxy sodium: 3.7 g were placed in a reaction vessel purged with nitrogen and refluxed and stirred overnight in toluene solvent. After the mixture is allowed to cool, methanol was added to the system and the precipitated solid was filtered to obtain a crude product. The obtained crude product was purified by crystallization using a mixed solution of toluene and acetone to obtain a yellow powder: 10.2 g (yield: 72.8%) of N-(4-dibenzofuran-3-yl-phenyl)-N-(2-phenyl-benzooxazole-6-yl)-N-{4-(2-phenyl-benzooxazole-6-yl)-phenyl}-amine (compound 193).

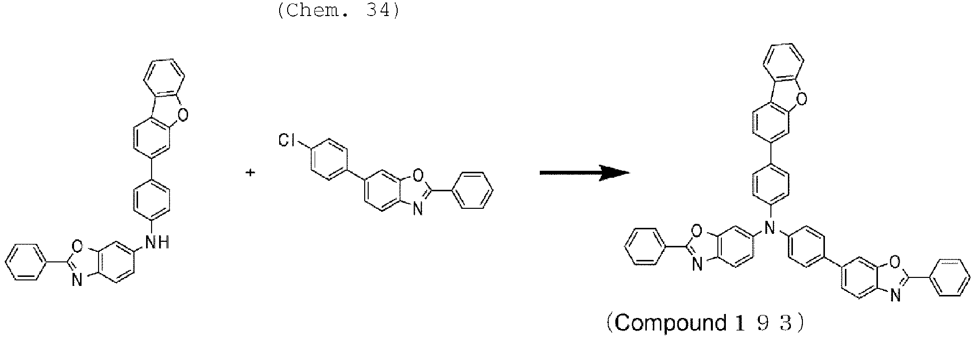

The structure of the obtained yellow powder was identified using NMR. The following 31 hydrogen signals were detected in ¹H-NMR(CDCl₃).

δ (ppm) = 8.31(2H), 8.25(2H), 8.01(1H), 7.99(1H), 7.84 (1H), 7.82(2H), 7.72(1H), 7.69-7.59(7H), 7.59-7.52(6H), 7.49(1H), 7.46(1H), 7.38(1H), 7.31(4H), and 7.28(1H).

### (Example 32)

### <Synthesis of N-(4-naphthalene-2-yl-phenyl)-N,N-bis{4-(2-phenyl-benzooxazole-6-yl)-phenyl}-amine (compound 195)>

4-(naphthalene-2-yl)-aniline: 4.5 g, 6-(4-chlorophenyl)-2-phenyl-benzooxazole: 13.8 g, tris(dibenzylideneacetone)dipalladium(0): 0.8 g, tri-t-butylphosphine: 0.3 g, and t-butoxy sodium: 6.0 g were placed in a reaction vessel purged with nitrogen and refluxed and stirred overnight in toluene solvent. After the mixture is allowed to cool, methanol was added to the system and the precipitated solid was filtered to obtain a crude product. The obtained crude product was purified by crystallization using a mixed solvent of dichloromethane and acetone to obtain a yellow powder: 7.3 g (yield: 46.8%) of N-(4-naphthalene-2-yl-phenyl)-N,N-bis{4-(2-phenyl-benzooxazole-6-yl)-phenyl}-amine (compound 195).

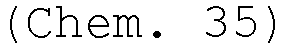

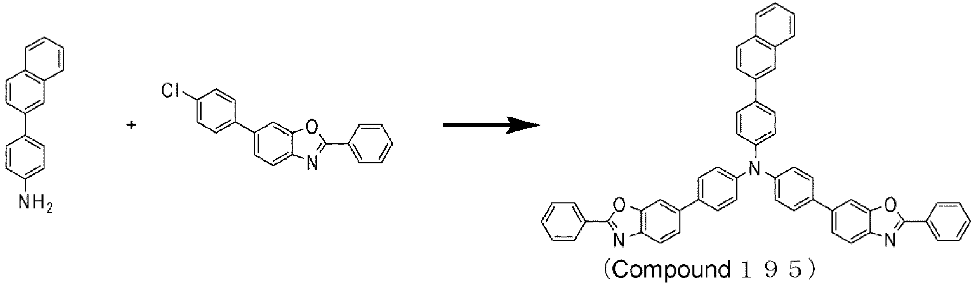

The structure of the obtained yellow powder was identified using NMR. The following 35 hydrogen signals were detected in ¹H-NMR(CDCl₃).

δ (ppm) = 8.32(4H), 8.08 (1H), 7.94 (2H), 7.89(1H), 7.87-7.82(4H), 7.80(1H), 7.72(2H), 7.67-7.61(6H), 7.61-7.47(8H), and 7.38-7.31(6H).

### (Example 33)

### <Synthesis of N-(4-quinoline-3-yl-phenyl)-N,N-bis{4-(2-phenyl-benzooxazole-6-yl)-phenyl}-amine (compound 196)>

4-(quinoline-3-yl)-aniline: 3.9 g, 6-(4-chlorophenyl)-2-phenyl-benzooxazole: 11.9 g, tris(dibenzylideneacetone)dipalladium(0): 0.7 g, tri-t-butylphosphine: 0.3 g, and t-butoxy sodium: 4.3 g were placed in a reaction vessel purged with nitrogen and refluxed and stirred overnight in xylene solvent. After the mixture is allowed to cool, methanol was added to the system and the precipitated solid was filtered to obtain a crude product. The obtained crude product was purified by crystallization using a mixed solution of toluene and acetone to obtain a yellow powder: 4.2 g (yield: 31.3%) of N-(4-quinoline-3-yl-phenyl)-N,N-bis{4-(2-phenyl-benzooxazole-6-yl)-phenyl}-amine (compound 196).

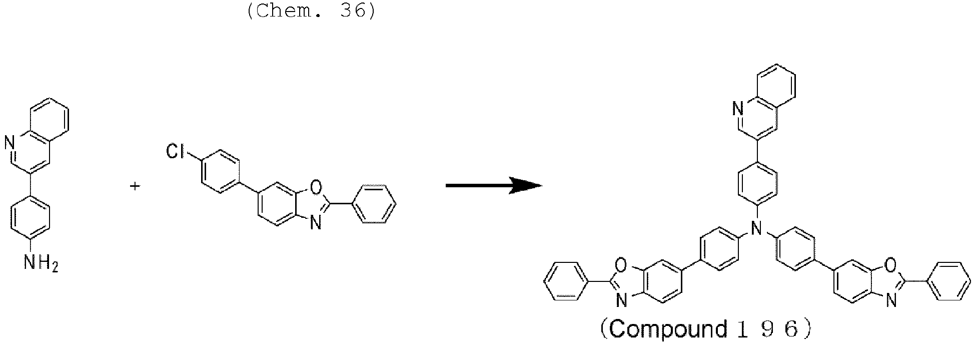

The structure of the obtained yellow powder was identified using NMR. The following 34 hydrogen signals were detected in ¹H-NMR(CDCl₃).

δ(ppm) = 9.25(1H), 8.35-8.27(5H), 8.17(1H), 7.91(1H), 7.85(2H), 7.83(2H), 7.75(1H), 7.71(2H), 7.65(6H), 7.62(1H), 7.60-7.54(6H), 7.38(2H), and 7.35(4H).

### (Example 34)

### <Synthesis of N-(4-thiophene-2-yl-phenyl)-N,N-bis{4-(2-phenyl-benzooxazole-6-yl)-phenyl}-amine (compound 198)>

4-(thiophene-2-yl)-aniline: 2.9 g, 6-(4-chlorophenyl)-2-phenyl-benzooxazole: 11.1 g, tris(dibenzylideneacetone)dipalladium(0): 0.6 g, tri-t-butylphosphine: 0.3 g, and t-butoxy sodium: 4.0 g were placed in a reaction vessel purged with nitrogen and refluxed and stirred overnight in xylene solvent. After the mixture is allowed to cool, methanol was added to the system and the precipitated solid was filtered to obtain a crude product. The obtained crude product was purified by crystallization using a mixed solution of toluene and acetone to obtain a bright yellow powder: 7.4 g (yield: 62.6%) of N-(4-benzofuran-2-yl-phenyl)-N,N-bis{4-(2-phenyl-benzooxazole-6-yl)-phenyl}-amine (compound 198).

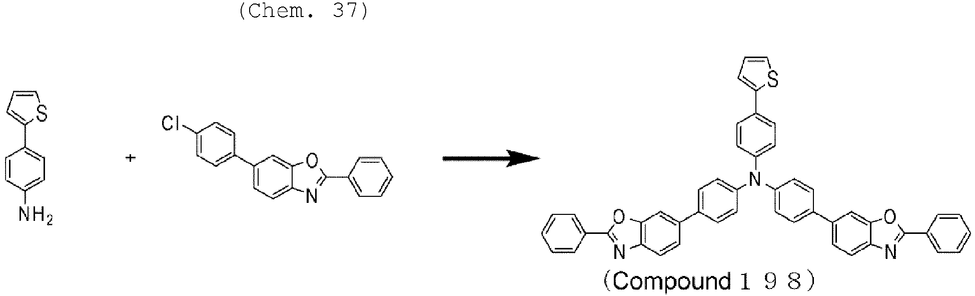

The structure of the obtained bright yellow powder was identified using NMR. The following 31 hydrogen signals were detected in ¹H-NMR(CDCl₃).

δ (ppm) = 8.31(4H), 7.84(2H), 7.82(2H), 7.66-7.52(14H), 7.33-7.26(7H), 7.24(1H), and 7.11(1H).

### (Example 35)

### <Synthesis of N-(dibenzofuran-3-yl)-N,N-bis{4-(2-phenyl-benzooxazole-6-yl)-phenyl}-amine (compound 199)>

N,N-bis{4-(2-phenyl-benzooxazole-6-yl)-phenyl}-amine: 7.0 g, 3-bromo-dibenzofuran: 3.4 g, tris(dibenzylideneacetone)dipalladium(0): 0.2 g, tri-t-butylphosphine: 0.1 g, and t-butoxy sodium: 2.4 g were placed in a reaction vessel purged with nitrogen and refluxed and stirred overnight in toluene solvent. After the mixture is allowed to cool, methanol was added to the system and the precipitated solid was filtered to obtain a crude product. The obtained crude product was purified by crystallization using a mixed solvent of chlorobenzene and acetone to obtain a yellow powder: 6.2 g (yield: 68.1%) of N-(dibenzofuran-3-yl)-N,N-bis{4-(2-phenyl-benzooxazole-6-yl)-phenyl}-amine (compound 199).

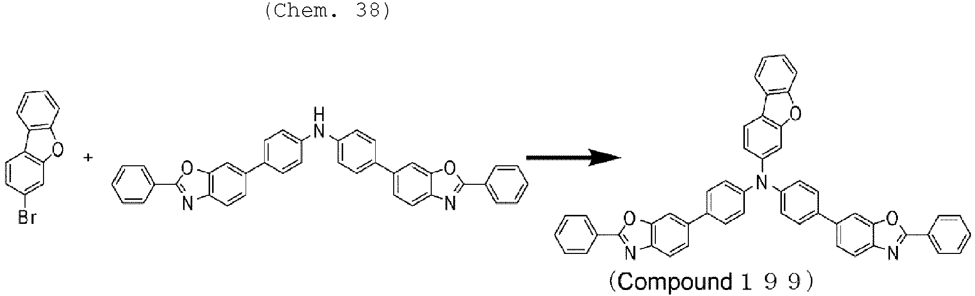

The structure of the obtained yellow powder was identified using NMR. The following 31 hydrogen signals were detected in ¹H-NMR(CDCl₃).

δ (ppm) = 8.31(4H), 7.92(1H), 7.87 (1H), 7.84(2H), 7.81(2H), 7.66-7.59(6H), 7.59-7.52(7H), 7.43(1H), 7.42(1H), 7.36(1H), 7.32(4H), and 7.24(1H).

### (Example 36)

### <Synthesis of N-(dibenzothiophene-2-yl)-N,N-bis{4-(2-phenyl-benzooxazole-6-yl)-phenyl}-amine (compound 200)>

2-amino-dibenzothiophene: 4.5 g, 6-(4-chlorophenyl)-2-phenyl-benzooxazole: 15.2 g, tris(dibenzylideneacetone)dipalladium(0): 0.8 g, tri-t-butylphosphine: 0.4 g, and t-butoxy sodium: 6.5 g were placed in a reaction vessel purged with nitrogen and refluxed and stirred overnight in toluene solvent. After the mixture is allowed to cool, methanol was added to the system and the precipitated solid was filtered to obtain a crude product. The obtained crude product was purified by crystallization using a mixed solution of toluene and acetone to obtain a yellow powder: 11.1 g (yield: 66.5%) of N-(dibenzothiophene-2-yl)-N,N-bis{4-(2-phenyl-benzooxazole-6-yl)-phenyl}-amine (compound 200).

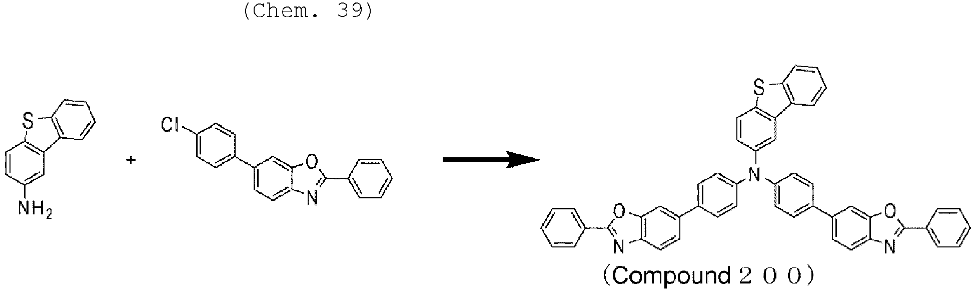

The structure of the obtained yellow powder was identified using NMR. The following 31 hydrogen signals were detected in ¹H-NMR(CDCl₃).

δ(ppm) = 8.31(4H), 8.06(1H), 8.05(1H), 7.89(1H), 7.86-7.81(5H), 7.65(2H), 7.62(4H), 7.59-7.53(6H), 7.48(1H), 7.43(1H), 7.40(1H), and 7.32(4H).

### (Example 37)

### <Synthesis of N-(4-benzooxazole-2-yl-phenyl)-N,N-bis{4-(2-phenyl-benzooxazole-6-yl)-phenyl}-amine (compound 201)>

4-benzooxazole-2-yl-aniline: 4.0 g, 6-(4-chlorophenyl)-2-phenyl-benzooxazole: 12.8 g, tris(dibenzylideneacetone)dipalladium(0): 0.7 g, tri-t-butylphosphine: 0.3 g, and t-butoxy sodium: 5.5 g were placed in a reaction vessel purged with nitrogen and refluxed and stirred overnight in toluene solvent. After the mixture is allowed to cool, methanol was added to the system and the precipitated solid was filtered to obtain a crude product. The obtained crude product was purified by crystallization using a mixed solvent of chlorobenzene and acetone to obtain a yellow powder: 9.4 g (yield: 66.2%) of N-(4-benzooxazole-2-yl-phenyl)-N,N-bis{4-(2-phenyl-benzooxazole-6-yl)-phenyl}-amine (compound 201).

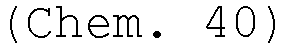

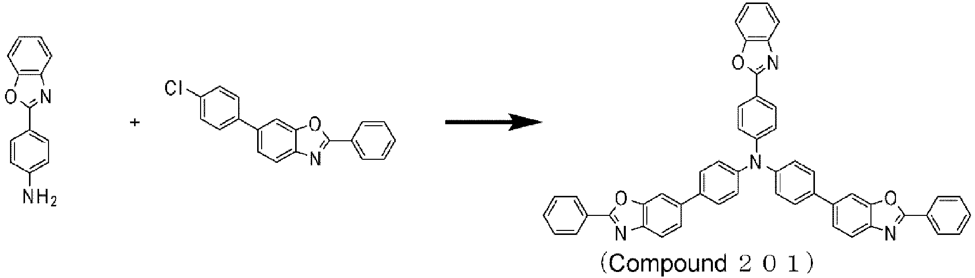

The structure of the obtained yellow powder was identified using NMR. The following 32 hydrogen signals were detected in ¹H-NMR(CDCl₃).

δ (ppm) = 8.32(4H), 8.19(2H), 7.88-7.83(4H), 7.78(1H), 7.71-7.63(6H), 7.62-7.54(7H), 7.41-7.33(6H), and 7.31(2H).

### (Example 38)

### <Synthesis of N-(4-benzofuran-2-yl-phenyl)-N,N-bis{4-(2-phenyl-benzooxazole-6-yl)-phenyl}-amine (compound 203)>

4-(benzofuran-2-yl)-aniline: 3.2 g, 6-(4-chlorophenyl)-2-phenyl-benzooxazole: 10.3 g, tris(dibenzylideneacetone)dipalladium(0): 0.6 g, tri-t-butylphosphine: 0.3 g, and t-butoxy sodium: 3.7 g were placed in a reaction vessel purged with nitrogen and refluxed and stirred overnight in xylene solvent. After the mixture is allowed to cool, methanol was added to the system and the precipitated solid was filtered to obtain a crude product. The obtained crude product was purified by crystallization using a mixed solution of toluene and acetone to obtain a yellow powder: 5.8 g (yield: 50.7%) of N-(4-benzofuran-2-yl-phenyl)-N,N-bis{4-(2-phenyl-benzooxazole-6-yl)-phenyl}-amine (compound 203).

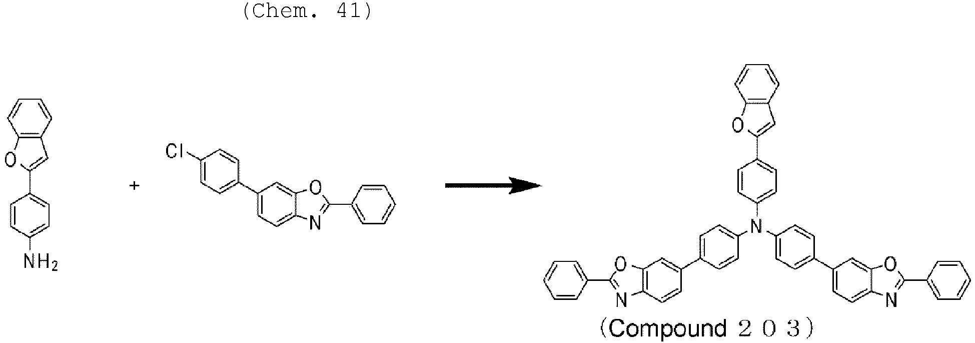

The structure of the obtained yellow powder was identified using NMR. The following 33 hydrogen signals were detected in ¹H-NMR(CDCl₃).

δ(ppm) = 8.32(4H), 7.85(2H), 7.82(4H), 7.67-7.52(14H), 7.32(4H), 7.31-7.22(3H), 7.26(1H), and 6.98(1H).

### (Example 39)

### <Synthesis of N-(4-benzothiophene-2-yl-phenyl)-N,N-bis{4-(2-phenyl-benzooxazole-6-yl)-phenyl}-amine (compound 204)>

4-(benzothiophene-2-yl)-aniline: 4.5 g, 6-(4-chloro-phenyl)-2-phenyl-benzooxazole: 13.4 g, tris(dibenzylideneacetone)dipalladium(0): 0.7 g, tri-t-butylphosphine: 0.3 g, and t-butoxy sodium: 5.8 g were placed in a reaction vessel purged with nitrogen and refluxed and stirred overnight in toluene solvent. After the mixture is allowed to cool, methanol was added to the system and the precipitated solid was filtered to obtain a crude product. The obtained crude product was purified by crystallization using a mixed solvent of chlorobenzene and acetone to obtain a yellow powder: 10.5 g (yield: 68.6%) of N-(4-benzothiophene-2-yl-phenyl)-N,N-bis{4-(2-phenyl-benzooxazole-6-yl)-phenyl}-amine (compound 204).

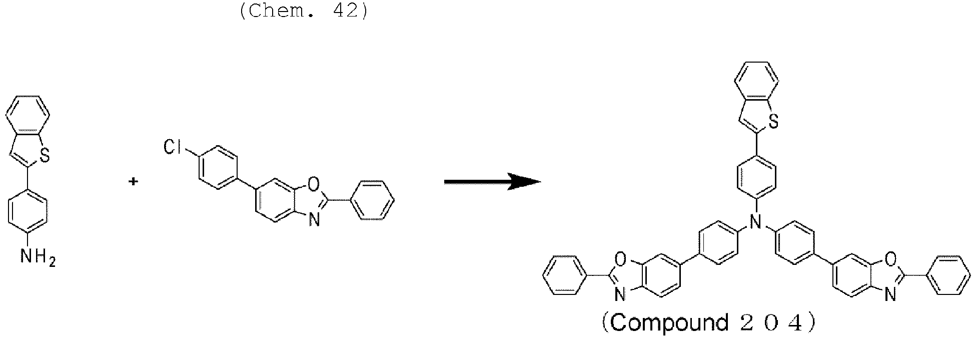

The structure of the obtained yellow powder was identified using NMR. The following 33 hydrogen signals were detected in ¹H-NMR(CDCl₃).

δ(ppm) = 8.32(4H), 7.88-7.81(5H), 7.79(1H), 7.69(2H), 7.67-7.61(6H), 7.58(4H), 7.57(2H), 7.52(1H), 7.38(1H), 7.32(5H), and 7.27(2H).

### (Example 40)

### <Synthesis of N-phenyl-N,N-bis{4-(2-naphthalene-2-yl-benzooxazole-6-yl)-phenyl}-amine (compound 205)>

Aniline: 2.0 g, 6-(4-chloro-phenyl)-2-(naphthalene-2-yl)-benzooxazole: 16.1 g, tris(dibenzylideneacetone)dipalladium(0): 1.0 g, tri-t-butylphosphine: 0.9 g, and t-butoxy sodium: 6.2 g were placed in a reaction vessel purged with nitrogen and refluxed and stirred overnight in toluene solvent. After the mixture is allowed to cool, ethanol was added to the system and the precipitated solid was filtered to obtain a crude product. The obtained crude product was purified by crystallization using a mixed solvent of chlorobenzene and acetone to obtain a yellow powder: 12.7 g (yield: 80.8%) of N-phenyl-N,N-bis{4-(2-naphthalene-2-yl-benzooxazole-6-yl)-phenyl}-amine (compound 205).

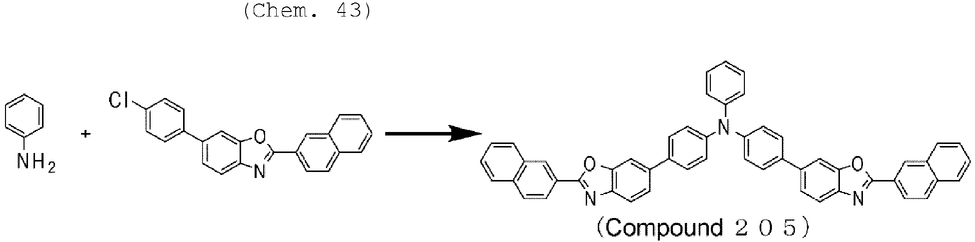

The structure of the obtained yellow powder was identified using NMR. The following 33 hydrogen signals were detected in ¹H-NMR(CDCl₃).

δ(ppm) = 8.81(2H), 8.35(2H), 8.01(4H), 7.92(2H), 7.86(2H), 7.83(2H), 7.64(2H), 7.63-7.56(8H), 7.37(2H), 7.31-7.25(6H), and 7.14(1H).

### (Example 41)

### <Synthesis of N-{4-(2-phenyl-benzooxazole-6-yl)-phenyl}-N-phenyl-N-{4'-(2-phenyl-benzooxazole-6-yl)-[1,1']biphenyl-4-yl}-amine (compound 206)>

N-phenyl-N-{4'-(2-phenyl-benzooxazole-6-yl)-[1,1']biphenyl-4-yl}-amine: 6.4 g, 6-(4-chloro-phenyl)-2-phenyl-benzooxazole: 4.9 g, tris(dibenzylideneacetone)dipalladium(0): 0.3 g, tri-t-butylphosphine: 0.1 g, and t-butoxy sodium: 2.8 g were placed in a reaction vessel purged with nitrogen and refluxed and stirred overnight in toluene solvent. After the mixture is allowed to cool, methanol was added to the system and the precipitated solid was filtered to obtain a crude product. The obtained crude product was purified by crystallization using a mixed solvent of dichloromethane and acetone to obtain a pale yellow powder: 6.2 g (yield: 60.2%) of N-{4-(2-phenyl-benzooxazole-6-yl)-phenyl}-N-phenyl-N-{4'-(2-phenyl-benzooxazole-6-yl)-[1,1']biphenyl-4-yl}-amine (compound 206) .

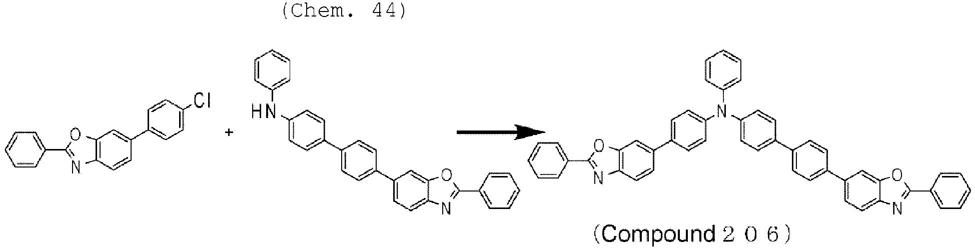

The structure of the obtained pale yellow powder was identified using NMR. The following 33 hydrogen signals were detected in ¹H-NMR(CDCl₃).

δ (ppm) = 8.31(4H), 7.87(1H), 7.86(1H), 7.83(1H), 7.81(1H), 7.74(4H), 7.68(1H), 7.65-7.53(11H), 7.36(2H), 7.28-7.22(6H), and 7.13(1H).

### (Example 42)

The glass transition temperature and melting point of the compounds obtained in the above Examples using a high sensitivity differential scanning calorimeter(DSC3100SA manufactured by Bruker AXS GmbH), and the results are shown in Table 1.

**(Table 1)**

| (Table 1-1) | | | |
|---|---|---|---|
| | Example compound | Glass transition temperature | Melting point |
| Example 1 | Compound 40 | 119°C | Not observed |
| Example 2 | Compound 50 | 115°C | 240°C |
| Example 3 | Compound 69 | 134°C | Not observed |
| Example 4 | Compound 70 | 131°C | Not observed |
| Example 5 | Compound 78 | 125°C | 266°C |
| Example 6 | Compound 119 | 118°C | Not observed |
| Example 7 | Compound 150 | 126°C | Not observed |
| Example 8 | Compound 2 | 115.7 | Not observed |
| Example 9 | Compound 17 | 125.6 | Not observed |
| Example 10 | Compound 38 | 122.7 | Not observed |
| Example 11 | Compound 52 | 114 | 245.3 |
| Example 12 | Compound 61 | 150.5 | Not observed |
| Example 13 | Compound 62 | 122.3 | 265.2 |
| Example 14 | Compound 71 | 132.6 | Not observed |
| Example 15 | Compound 79 | 127.9 | 261.4 |
| Example 16 | Compound 101 | 122.5 | Not observed |
| Example 17 | Compound 137 | 130 | Not observed |
| Example 18 | Compound 138 | 133.7 | Not observed |
| Example 19 | Compound 155 | 130.2 | 237.1 |
| Example 20 | Compound 171 | 106.8 | Not observed |

**(Table 1-2)**

| | | | |
|---|---|---|---|
| Example 21 | Compound 173 | 116.4 | Not observed |
| Example 22 | Compound 177 | 117.5 | Not observed |
| Example 23 | Compound 180 | 106.3 | Not observed |
| Example 24 | Compound 182 | 102.8 | 206.6 |
| Example 25 | Compound 184 | 116.3 | 240.2 |
| Example 26 | Compound 188 | 120.9 | Not observed |
| Example 27 | Compound 189 | 120.6 | Not observed |
| Example 28 | Compound 190 | 123.9 | Not observed |
| Example 29 | Compound 191 | 122.8 | Not observed |
| Example 30 | Compound 192 | 108.8 | Not observed |
| Example 31 | Compound 193 | 118.4 | Not observed |
| Example 32 | Compound 195 | 117.6 | Not observed |
| Example 33 | Compound 196 | 120.8 | Not observed |
| Example 34 | Compound 198 | 116.1 | Not observed |
| Example 35 | Compound 199 | 120.8 | Not observed |
| Example 36 | Compound 200 | 130 | 266.4 |
| Example 37 | Compound 201 | 127.6 | 291.8 |
| Example 38 | Compound 203 | 121.9 | Not observed |
| Example 39 | Compound 204 | 125.6 | Not observed |
| Example 40 | Compound 205 | 121.3 | 220.2 |
| Example 41 | Compound 206 | 111.1 | Not observed |

The above measurement results show that the amine compound according to the present invention has a glass transition temperature of 100°C or more and is stable in a thin-film state.

### (Example 43)

A deposition film having a film thickness of 80 nm was prepared on a silicon substrate using each of the compounds obtained in the Examples, and refractive indices n at wavelengths of 450 nm and 750 nm and extinction coefficients k at wavelengths of 400 nm and 410 nm were measured using a spectrometer (F10-RT-UV manufactured by Filmetrics, INC.). For comparison, measurement was also made on a comparative compound (2-1) and a comparative compound (2-2) having the following structural formulae and Alq₃ (see, for example, Patent Literature 4 and Patent Literature 5). The measurement results of the refractive index n and the extinction coefficient k are collectively shown in Table 2.

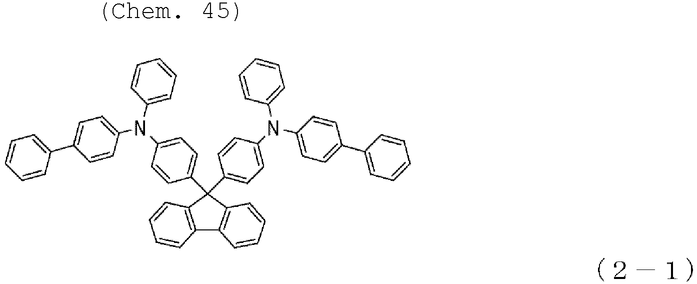

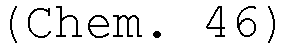

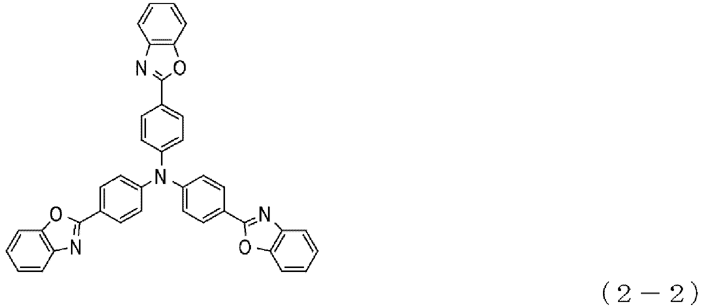

**(Table 2)**

| (Table 2-1) | | | | | |
|---|---|---|---|---|---|
| | Example compound | Refractive index:n (λ :450nm) | Refractive index:n (λ :750nm) | Extinction coefficient:k (λ :400nm) | Extinction coefficient:k (λ:410nm) |
| Example 1 | Compound 40 | 2.36 | 1.93 | 0.86 | 0.56 |
| Example 2 | Compound 50 | 2.37 | 1.91 | 0.98 | 0.70 |
| Example 3 | Compound 69 | 2.50 | 1.95 | 1.11 | 0.94 |
| Example 4 | Compound 70 | 2.45 | 1.94 | 1.05 | 0.78 |
| Example 5 | Compound 78 | 2.43 | 1.95 | 1.02 | 0.77 |
| Example 6 | Compound 119 | 2.43 | 1.93 | 1.06 | 0.82 |
| Example 7 | Compound 150 | 2.38 | 1.92 | 1.00 | 0.70 |
| Example 8 | Compound 2 | 2.34 | 1.90 | 0.92 | 0.61 |
| Example 9 | Compound 17 | 2.34 | 1.90 | 0.94 | 0.65 |
| Example 10 | Compound 38 | 2.53 | 1.93 | 1.18 | 1.02 |
| Example 11 | Compound 52 | 2.35 | 1.92 | 0.64 | 0.36 |
| Example 12 | Compound 61 | 2.50 | 1.95 | 1.01 | 0.91 |
| Example 13 | Compound 62 | 2.40 | 1.94 | 0.98 | 0.72 |
| Example 14 | Compound 71 | 2.46 | 1.95 | 1.06 | 0.81 |
| Example 15 | Compound 79 | 2.46 | 1.95 | 1.04 | 0.82 |

**(Table 2-2)**

| | | | | | |
|---|---|---|---|---|---|
| Example 16 | Compound 101 | 2.43 | 1.93 | 1.07 | 0.79 |
| Example 17 | Compound 137 | 2.36 | 1.91 | 0.99 | 0.68 |
| Example 18 | Compound 138 | 2.38 | 1.92 | 0.99 | 0.70 |
| Example 19 | Compound 155 | 2.40 | 1.93 | 0.99 | 0.73 |
| Example 20 | Compound 171 | 2.30 | 1.88 | 0.63 | 0.42 |
| Example 21 | Compound 173 | 2.31 | 1.91 | 0.67 | 0.44 |
| Example 22 | Compound 177 | 2.30 | 1.89 | 0.69 | 0.50 |
| Example 23 | Compound 180 | 2.32 | 1.89 | 0.75 | 0.54 |
| Example 24 | Compound 182 | 2.32 | 1.89 | 0.72 | 0.43 |
| Example 25 | Compound 184 | 2.32 | 1.91 | 0.74 | 0.57 |
| Example 26 | Compound 188 | 2.41 | 1.91 | 1.08 | 0.76 |
| Example 27 | Compound 189 | 2.44 | 1.93 | 1.05 | 0.78 |
| Example 28 | Compound 190 | 2.43 | 1.92 | 1.08 | 0.80 |
| Example 29 | Compound 191 | 2.43 | 1.94 | 1.04 | 0.80 |
| Example 30 | Compound 192 | 2.31 | 1.91 | 0.62 | 0.39 |
| Example 31 | Compound 193 | 2.33 | 1.91 | 0.76 | 0.49 |
| Example 32 | Compound 195 | 2.30 | 1.92 | 0.74 | 0.43 |
| Example 33 | Compound 196 | 2.31 | 1.93 | 0.79 | 0.50 |
| Example 34 | Compound 198 | 2.33 | 1.92 | 0.76 | 0.47 |
| Example 35 | Compound 199 | 2.32 | 1.92 | 0.66 | 0.37 |
| Example 36 | Compound 200 | 2.31 | 1.91 | 0.65 | 0.32 |
| Example 37 | Compound 201 | 2.35 | 1.91 | 0.94 | 0.65 |
| Example 38 | Compound 203 | 2.35 | 1.92 | 0.88 | 0.58 |
| Example 39 | Compound 204 | 2.33 | 1.93 | 0.85 | 0.59 |
| Example 40 | Compound 205 | 2.35 | 1.94 | 0.61 | 0.45 |
| Example 41 | Compound 206 | 2.31 | 1.89 | 0.49 | 0.28 |
| Comparative Example 1 | Alq3 | 1.88 | 1.73 | 0.16 | 0.14 |
| Comparative Example 2 | Compound (2-1) | 1.84 | 1.77 | 0.07 | 0.03 |
| Comparative Example 3 | Compound (2-2) | 2.29 | 1.89 | 0.85 | 0.56 |

As shown in Table 2, the compound according to the present invention has a refractive index equal to or greater than those of Alq₃, the comparative compound (2-1), and the comparative compound (2-2) in the wavelength range of 450 nm to 750 nm, and has low extinction coefficients at the wavelengths of 400 nm and 410 nm, so that there is little loss of light absorbed in the blue region. This is expected to improve the light extraction efficiency in the organic EL device in which the amine compound according to the present invention is used as the constituent material of the capping layer.

### (Example 44)

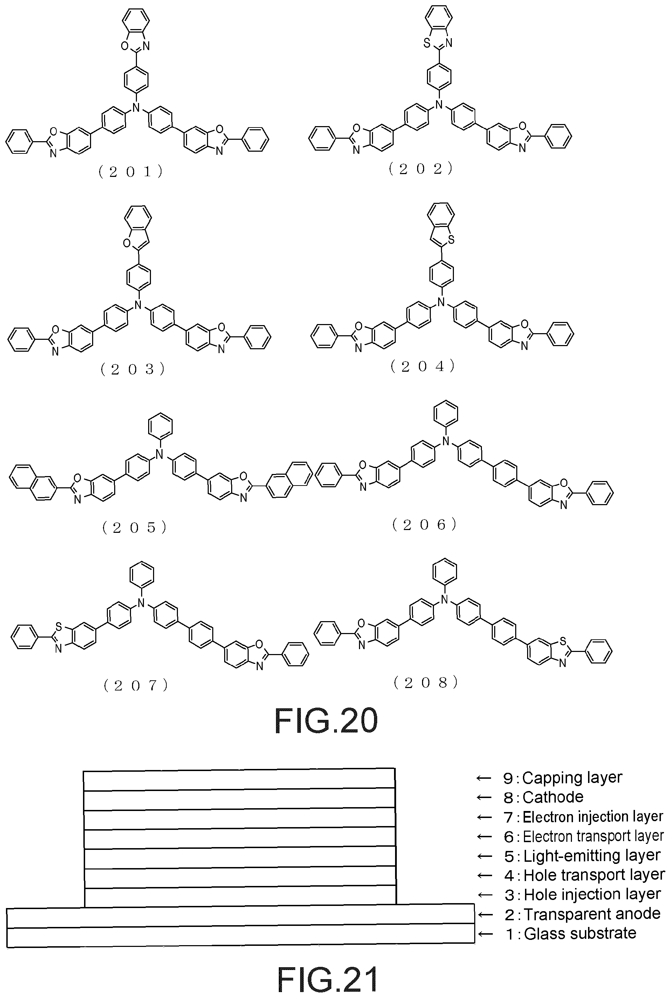

As shown in Fig. 21, the organic EL device was prepared by depositing a hole injection layer 3, a hole transport layer 4, a light-emitting layer 5, an electron transport layer 6, an electron injection layer 7, a cathode 8, and a capping layer 9 in this order on a reflective ITO electrode formed in advance as a transparent anode 2 on a glass substrate 1.

Specifically, the glass substrate 1 on which ITO having a film thickness of 50 nm, a reflective film formed of a silver alloy having a film thickness of 100 nm, and ITO having a film thickness of 5 nm were deposited in this order was subjected to ultrasonic cleaning in isopropyl alcohol for 20 minutes and then dried on a hot plate heated to 250°C for 10 minutes. After that, UV ozone treatment was performed for 2 minutes, this glass substrate with ITO was attached in a vacuum deposition machine, and the pressure was reduced to 0.001 Pa or less. Subsequently, the hole injection layer 3 was formed to have a film thickness of 10 nm and cover the transparent anode 2 by binary deposition of an electron acceptor (Acceptor-1) having the following structural formula and a compound (3-1) having the following structural formula at the deposition rate where the deposition rate ratio satisfies the following relationship of Acceptor-1: compound (3-1) = 3: 97. The hole transport layer 4 was formed using the compound (3-1) having the following structural formula on this hole injection layer 3 to have a film thickness of 140 nm. The light-emitting layer 5 was formed on this hole transport layer 4 to have a film thickness of 20 nm by binary deposition of a compound (3-2) having the following structural formula and a compound (3-3) having the following structural formula at the deposition rate where the deposition rate ratio satisfies the following relationship of (3-2): (3-3) = 5: 95. The electron transport layer 6 was formed on this light-emitting layer 5 to have a film thickness of 30 nm by binary deposition of a compound (3-4) having the following structural formula and a compound (3-5) having the following structural formula at the deposition rate where the deposition rate ratio satisfies the following relationship of (3-4): (3-5) = 50: 50. The electron injection layer 7 was formed using lithium fluoride on this electron transport layer 6 to have a film thickness of 1 nm. The cathode 8 was formed using a magnesium silver alloy on this electron injection layer 7 to have a film thickness of 12 nm. Finally, the capping layer 9 was formed using the compound (40) according to Example 1 to have a film thickness of 60 nm. The properties of the prepared organic EL device were measured in the atmosphere at room temperature.

The measurement results of the light emission properties when a direct current voltage was applied to the prepared organic EL device are collectively shown in Table 3.

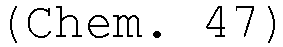

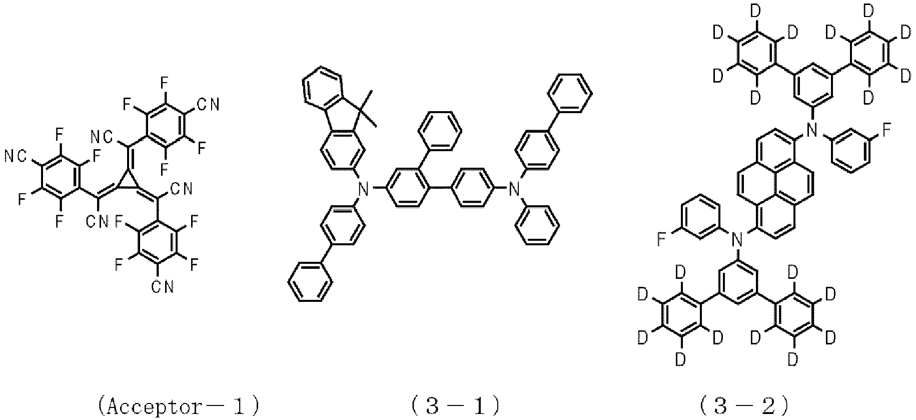

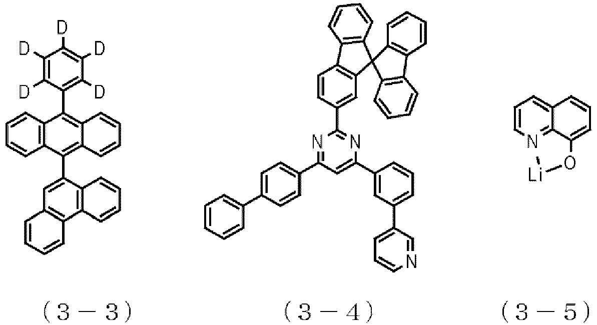

### (Example 45)

An organic EL device was prepared under the same conditions as in Example 44 except that the compound (50) according to Example 2 was formed as the capping layer 9 to have a film thickness of 60 nm instead of the compound (40) according to Example 1. The properties of the prepared organic EL device were measured in the atmosphere at room temperature. The measurement results of the light emission properties 10 when a direct current voltage was applied to the prepared organic EL device are collectively shown in Table 3.

### (Example 46)

An organic EL device was prepared under the same conditions as in Example 44 except that the compound (69) according to Example 3 was formed as the capping layer 9 to have a film thickness of 60 nm instead of the compound (40) according to Example 1. The properties of the prepared organic EL device were measured in the atmosphere at room temperature. The measurement results of the light emission properties when a direct current voltage was applied to the prepared organic EL device are collectively shown in Table 3.

### (Example 47)

An organic EL device was prepared under the same conditions as in Example 44 except that the compound (70) according to Example 4 was formed as the capping layer 9 to have a film thickness of 60 nm instead of the compound (40) according to Example 1. The properties of the prepared organic EL device were measured in the atmosphere at room temperature. The measurement results of the light emission properties when a direct current voltage was applied to the prepared organic EL device are collectively shown in Table 3.

### (Example 48)

An organic EL device was prepared under the same conditions as in Example 44 except that the compound (78) according to Example 5 was formed as the capping layer 9 to have a film thickness of 60 nm instead of the compound (40) according to Example 1. The properties of the prepared organic EL device were measured in the atmosphere at room temperature. The measurement results of the light emission properties when a direct current voltage was applied to the prepared organic EL device are collectively shown in Table 3.

### (Example 49)

An organic EL device was prepared under the same conditions as in Example 44 except that the compound (119) according to Example 6 was formed as the capping layer 9 to have a film thickness of 60 nm instead of the compound (40) according to Example 1. The properties of the prepared organic EL device were measured in the atmosphere at room temperature. The measurement results of the light emission properties when a direct current voltage was applied to the prepared organic EL device are collectively shown in Table 3.

### (Example 50)

An organic EL device was prepared under the same conditions as in Example 44 except that the compound (150) according to Example 7 was formed as the capping layer 9 to have a film thickness of 60 nm instead of the compound (40) according to Example 1. The properties of the prepared organic EL device were measured in the atmosphere at room temperature. The measurement results of the light emission properties when a direct current voltage was applied to the prepared organic EL device are collectively shown in Table 3.

### (Example 51)

An organic EL device was prepared under the same conditions as in Example 44 except that the compound (2) according to Example 8 was formed as the capping layer 9 to have a film thickness of 60 nm instead of the compound (40) according to Example 1. The properties of the prepared organic EL device were measured in the atmosphere at room temperature. The measurement results of the light emission properties when a direct current voltage was applied to the prepared organic EL device are collectively shown in Table 3.

### (Example 52)

An organic EL device was prepared under the same conditions as in Example 44 except that the compound (17) according to Example 9 was formed as the capping layer 9 to have a film thickness of 60 nm instead of the compound (40) according to Example 1. The properties of the prepared organic EL device were measured in the atmosphere at room temperature. The measurement results of the light emission properties when a direct current voltage was applied to the prepared organic EL device are collectively shown in Table 3.

### (Example 53)

An organic EL device was prepared under the same conditions as in Example 44 except that the compound (38) according to Example 10 was formed as the capping layer 9 to have a film thickness of 60 nm instead of the compound (40) according to Example 1. The properties of the prepared organic EL device were measured in the atmosphere at room temperature. The measurement results of the light emission properties when a direct current voltage was applied to the prepared organic EL device are collectively shown in Table 3.

### (Example 54)

An organic EL device was prepared under the same conditions as in Example 44 except that the compound (52) according to Example 11 was formed as the capping layer 9 to have a film thickness of 60 nm instead of the compound (40) according to Example 1. The properties of the prepared organic EL device were measured in the atmosphere at room temperature. The measurement results of the light emission properties when a direct current voltage was applied to the prepared organic EL device are collectively shown in Table 3.

### (Example 55)

An organic EL device was prepared under the same conditions as in Example 44 except that the compound (61) according to Example 12 was formed as the capping layer 9 to have a film thickness of 60 nm instead of the compound (40) according to Example 1. The properties of the prepared organic EL device were measured in the atmosphere at room temperature. The measurement results of the light emission properties when a direct current voltage was applied to the prepared organic EL device are collectively shown in Table 3.

### (Example 56)

An organic EL device was prepared under the same conditions as in Example 44 except that the compound (62) according to Example 13 was formed as the capping layer 9 to have a film thickness of 60 nm instead of the compound (40) according to Example 1. The properties of the prepared organic EL device were measured in the atmosphere at room temperature. The measurement results of the light emission properties when a direct current voltage was applied to the prepared organic EL device are collectively shown in Table 3.

### (Example 57)

An organic EL device was prepared under the same conditions as in Example 44 except that the compound (71) according to Example 14 was formed as the capping layer 9 to have a film thickness of 60 nm instead of the compound (40) according to Example 1. The properties of the prepared organic EL device were measured in the atmosphere at room temperature. The measurement results of the light emission properties when a direct current voltage was applied to the prepared organic EL device are collectively shown in Table 3.

### (Example 58)

An organic EL device was prepared under the same conditions as in Example 44 except that the compound (79) according to Example 15 was formed as the capping layer 9 to have a film thickness of 60 nm instead of the compound (40) according to Example 1. The properties of the prepared organic EL device were measured in the atmosphere at room temperature. The measurement results of the light emission properties when a direct current voltage was applied to the prepared organic EL device are collectively shown in Table 3.

### (Example 59)

An organic EL device was prepared under the same conditions as in Example 44 except that the compound (101) according to Example 16 was formed as the capping layer 9 to have a film thickness of 60 nm instead of the compound (40) according to Example 1. The properties of the prepared organic EL device were measured in the atmosphere at room temperature. The measurement results of the light emission properties when a direct current voltage was applied to the prepared organic EL device are collectively shown in Table 3.

### (Example 60)

An organic EL device was prepared under the same conditions as in Example 44 except that the compound (137) according to Example 17 was formed as the capping layer 9 to have a film thickness of 60 nm instead of the compound (40) according to Example 1. The properties of the prepared organic EL device were measured in the atmosphere at room temperature. The measurement results of the light emission properties when a direct current voltage was applied to the prepared organic EL device are collectively shown in Table 3.

### (Example 61)

An organic EL device was prepared under the same conditions as in Example 44 except that the compound (138) according to Example 18 was formed as the capping layer 9 to have a film thickness of 60 nm instead of the compound (40) according to Example 1. The properties of the prepared organic EL device were measured in the atmosphere at room temperature. The measurement results of the light emission properties when a direct current voltage was applied to the prepared organic EL device are collectively shown in Table 3.

### (Example 62)

An organic EL device was prepared under the same conditions as in Example 44 except that the compound (155) according to Example 19 was formed as the capping layer 9 to have a film thickness of 60 nm instead of the compound (40) according to Example 1. The properties of the prepared organic EL device were measured in the atmosphere at room temperature. The measurement results of the light emission properties when a direct current voltage was applied to the prepared organic EL device are collectively shown in Table 3.

### (Example 63)

An organic EL device was prepared under the same conditions as in Example 44 except that the compound (171) according to Example 20 was formed as the capping layer 9 to have a film thickness of 60 nm instead of the compound (40) according to Example 1. The properties of the prepared organic EL device were measured in the atmosphere at room temperature. The measurement results of the light emission properties when a direct current voltage was applied to the prepared organic EL device are collectively shown in Table 3.

### (Example 64)

An organic EL device was prepared under the same conditions as in Example 44 except that the compound (173) according to Example 21 was formed as the capping layer 9 to have a film thickness of 60 nm instead of the compound (40) according to Example 1. The properties of the prepared organic EL device were measured in the atmosphere at room temperature. The measurement results of the light emission properties when a direct current voltage was applied to the prepared organic EL device are collectively shown in Table 3.

### (Example 65)

An organic EL device was prepared under the same conditions as in Example 44 except that the compound (177) according to Example 22 was formed as the capping layer 9 to have a film thickness of 60 nm instead of the compound (40) according to Example 1. The properties of the prepared organic EL device were measured in the atmosphere at room temperature. The measurement results of the light emission properties when a direct current voltage was applied to the prepared organic EL device are collectively shown in Table 3.

### (Example 66)

An organic EL device was prepared under the same conditions as in Example 44 except that the compound (180) according to Example 23 was formed as the capping layer 9 to have a film thickness of 60 nm instead of the compound (40) according to Example 1. The properties of the prepared organic EL device were measured in the atmosphere at room temperature. The measurement results of the light emission properties when a direct current voltage was applied to the prepared organic EL device are collectively shown in Table 3.

### (Example 67)

An organic EL device was prepared under the same conditions as in Example 44 except that the compound (182) according to Example 24 was formed as the capping layer 9 to have a film thickness of 60 nm instead of the compound (40) according to Example 1. The properties of the prepared organic EL device were measured in the atmosphere at room temperature. The measurement results of the light emission properties when a direct current voltage was applied to the prepared organic EL device are collectively shown in Table 3.

### (Example 68)

An organic EL device was prepared under the same conditions as in Example 44 except that the compound (184) according to Example 25 was formed as the capping layer 9 to have a film thickness of 60 nm instead of the compound (40) according to Example 1. The properties of the prepared organic EL device were measured in the atmosphere at room temperature. The measurement results of the light emission properties when a direct current voltage was applied to the prepared organic EL device are collectively shown in Table 3.

### (Example 69)

An organic EL device was prepared under the same conditions as in Example 44 except that the compound (188) according to Example 26 was formed as the capping layer 9 to have a film thickness of 60 nm instead of the compound (40) according to Example 1. The properties of the prepared organic EL device were measured in the atmosphere at room temperature. The measurement results of the light emission properties when a direct current voltage was applied to the prepared organic EL device are collectively shown in Table 3.

### (Example 70)

An organic EL device was prepared under the same conditions as in Example 44 except that the compound (189) according to Example 27 was formed as the capping layer 9 to have a film thickness of 60 nm instead of the compound (40) according to Example 1. The properties of the prepared organic EL device were measured in the atmosphere at room temperature. The measurement results of the light emission properties when a direct current voltage was applied to the prepared organic EL device are collectively shown in Table 3.

### (Example 71)

An organic EL device was prepared under the same conditions as in Example 44 except that the compound (190) according to Example 28 was formed as the capping layer 9 to have a film thickness of 60 nm instead of the compound (40) according to Example 1. The properties of the prepared organic EL device were measured in the atmosphere at room temperature. The measurement results of the light emission properties when a direct current voltage was applied to the prepared organic EL device are collectively shown in Table 3.

### (Example 72)

An organic EL device was prepared under the same conditions as in Example 44 except that the compound (191) according to Example 29 was formed as the capping layer 9 to have a film thickness of 60 nm instead of the compound (40) according to Example 1. The properties of the prepared organic EL device were measured in the atmosphere at room temperature. The measurement results of the light emission properties when a direct current voltage was applied to the prepared organic EL device are collectively shown in Table 3.

### (Example 73)

An organic EL device was prepared under the same conditions as in Example 44 except that the compound (192) according to Example 30 was formed as the capping layer 9 to have a film thickness of 60 nm instead of the compound (40) according to Example 1. The properties of the prepared organic EL device were measured in the atmosphere at room temperature. The measurement results of the light emission properties when a direct current voltage was applied to the prepared organic EL device are collectively shown in Table 3.

### (Example 74)

An organic EL device was prepared under the same conditions as in Example 44 except that the compound (193) according to Example 31 was formed as the capping layer 9 to have a film thickness of 60 nm instead of the compound (40) according to Example 1. The properties of the prepared organic EL device were measured in the atmosphere at room temperature. The measurement results of the light emission properties when a direct current voltage was applied to the prepared organic EL device are collectively shown in Table 3.

### (Example 75)

An organic EL device was prepared under the same conditions as in Example 44 except that the compound (195) according to Example 32 was formed as the capping layer 9 to have a film thickness of 60 nm instead of the compound (40) according to Example 1. The properties of the prepared organic EL device were measured in the atmosphere at room temperature. The measurement results of the light emission properties when a direct current voltage was applied to the prepared organic EL device are collectively shown in Table 3.

### (Example 76)

An organic EL device was prepared under the same conditions as in Example 44 except that the compound (196) according to Example 33 was formed as the capping layer 9 to have a film thickness of 60 nm instead of the compound (40) according to Example 1. The properties of the prepared organic EL device were measured in the atmosphere at room temperature. The measurement results of the light emission properties when a direct current voltage was applied to the prepared organic EL device are collectively shown in Table 3.

### (Example 77)

An organic EL device was prepared under the same conditions as in Example 44 except that the compound (198) according to Example 34 was formed as the capping layer 9 to have a film thickness of 60 nm instead of the compound (40) according to Example 1. The properties of the prepared organic EL device were measured in the atmosphere at room temperature. The measurement results of the light emission properties when a direct current voltage was applied to the prepared organic EL device are collectively shown in Table 3.

### (Example 78)

An organic EL device was prepared under the same conditions as in Example 44 except that the compound (199) according to Example 35 was formed as the capping layer 9 to have a film thickness of 60 nm instead of the compound (40) according to Example 1. The properties of the prepared organic EL device were measured in the atmosphere at room temperature. The measurement results of the light emission properties when a direct current voltage was applied to the prepared organic EL device are collectively shown in Table 3.

### (Example 79)

An organic EL device was prepared under the same conditions as in Example 44 except that the compound (200) according to Example 36 was formed as the capping layer 9 to have a film thickness of 60 nm instead of the compound (40) according to Example 1. The properties of the prepared organic EL device were measured in the atmosphere at room temperature. The measurement results of the light emission properties when a direct current voltage was applied to the prepared organic EL device are collectively shown in Table 3.

### (Example 80)

An organic EL device was prepared under the same conditions as in Example 44 except that the compound (201) according to Example 37 was formed as the capping layer 9 to have a film thickness of 60 nm instead of the compound (40) according to Example 1. The properties of the prepared organic EL device were measured in the atmosphere at room temperature. The measurement results of the light emission properties when a direct current voltage was applied to the prepared organic EL device are collectively shown in Table 3.

### (Example 81)

An organic EL device was prepared under the same conditions as in Example 44 except that the compound (203) according to Example 38 was formed as the capping layer 9 to have a film thickness of 60 nm instead of the compound (40) according to Example 1. The properties of the prepared organic EL device were measured in the atmosphere at room temperature. The measurement results of the light emission properties when a direct current voltage was applied to the prepared organic EL device are collectively shown in Table 3.

### (Example 82)

An organic EL device was prepared under the same conditions as in Example 44 except that the compound (204) according to Example 39 was formed as the capping layer 9 to have a film thickness of 60 nm instead of the compound (40) according to Example 1. The properties of the prepared organic EL device were measured in the atmosphere at room temperature. The measurement results of the light emission properties when a direct current voltage was applied to the prepared organic EL device are collectively shown in Table 3.

### (Example 83)

An organic EL device was prepared under the same conditions as in Example 44 except that the compound (205) according to Example 40 was formed as the capping layer 9 to have a film thickness of 60 nm instead of the compound (40) according to Example 1. The properties of the prepared organic EL device were measured in the atmosphere at room temperature. The measurement results of the light emission properties when a direct current voltage was applied to the prepared organic EL device are collectively shown in Table 3.

### (Example 84)

An organic EL device was prepared under the same conditions as in Example 44 except that the compound (206) according to Example 41 was formed as the capping layer 9 to have a film thickness of 60 nm instead of the compound (40) according to Example 1. The properties of the prepared organic EL device were measured in the atmosphere at room temperature. The measurement results of the light emission properties when a direct current voltage was applied to the prepared organic EL device are collectively shown in Table 3.

### (Comparative Example 4)

For comparison, an organic EL device was prepared under the same conditions as in Example 44 except that Alq₃ was formed as the capping layer 9 to have a film thickness of 60 nm instead of the compound (40) according to Example 1. The properties of the prepared organic EL device were measured in the atmosphere at room temperature. The measurement results of the light emission properties when a direct current voltage was applied to the prepared organic EL device are collectively shown in Table 3.

### (Comparative Example 5)

For comparison, an organic EL device was prepared under the same conditions as in Example 44 except that the compound(2-1) was formed as the capping layer 9 to have a film thickness of 60 nm instead of the compound (40) according to Example 1. The properties of the prepared organic EL device were measured in the atmosphere at room temperature. The measurement results of the light emission properties when a direct current voltage was applied to the prepared organic EL device are collectively shown in Table 3.

### (Comparative Example 6)

For comparison, an organic EL device was prepared under the same conditions as in Example 44 except that the compound (2-2) was formed as the capping layer 9 to have a film thickness of 60 nm instead of the compound (40) according to Example 1. The properties of the prepared organic EL device were measured in the atmosphere at room temperature. The measurement results of the light emission properties when a direct current voltage was applied to the prepared organic EL device are collectively shown in Table 3.

The results of measuring the device lifetime using the organic EL devices prepared in Examples 44 to Example 84 and Comparative Example 4 to Comparative Example 6 are collectively shown in Table 3. The device lifetime was measured as the time until the luminance was attenuated to 95% with the initial luminance when the constant current driving of 10 mA/cm² was performed being 100%.

**(Table 3)**

| (Table 3-1) | | | | | | |
|---|---|---|---|---|---|---|
| | Capping layer | Voltage[V] (@10mA/cm2) | Luminance [cd/m2] (@10mA/cm2) | Light emission efficiency [cd/A] (@10mA/cm2) | Power efficiency [Im/W] (@10mA/cm2) | Device lifetime 95% attenuation |
| Example 44 | Compound 40 | 3.65 | 819 | 8.19 | 7.06 | 155 hours |
| Example 45 | Compound 50 | 3.66 | 830 | 8.31 | 7.13 | 159 hours |
| Example 46 | Compound 69 | 3.64 | 834 | 8.35 | 7.21 | 150 hours |
| Example 47 | Compound 70 | 3.66 | 833 | 8.33 | 7.16 | 159 hours |
| Example 48 | Compound 78 | 3.65 | 827 | 8.27 | 7.13 | 152 hours |
| Example 49 | Compound 119 | 3.60 | 832 | 8.33 | 7.28 | 158 hours |
| Example 50 | Compound 150 | 3.65 | 817 | 8.17 | 7.03 | 161 hours |
| Example 51 | Compound 2 | 3.64 | 821 | 8.21 | 7.08 | 155 hours |
| Example 52 | Compound 17 | 3.66 | 829 | 8.29 | 7.12 | 162 hours |
| Example 53 | Compound 38 | 3.62 | 839 | 8.39 | 7.28 | 170 hours |
| Example 54 | Compound 52 | 3.64 | 813 | 8.12 | 7.02 | 169 hours |
| Example 55 | Compound 61 | 3.66 | 837 | 8.37 | 7.20 | 148 hours |

**(Table 3-2)**

| | | | | | | |
|---|---|---|---|---|---|---|
| Example 56 | Compound 62 | 3.62 | 833 | 8.33 | 7.22 | 156 hours |
| Example 57 | Compound 71 | 3.62 | 833 | 8.33 | 7.24 | 159 hours |
| Example 58 | Compound 79 | 3.65 | 832 | 8.32 | 7.16 | 168 hours |
| Example 59 | Compound 101 | 3.64 | 833 | 8.34 | 7.20 | 164 hours |
| Example 60 | Compound 137 | 3.63 | 823 | 8.23 | 7.11 | 157 hours |
| Example 61 | Compound 138 | 3.64 | 824 | 8.24 | 7.12 | 166 hours |
| Example 62 | Compound 155 | 3.65 | 831 | 8.30 | 7.15 | 154 hours |
| Example 63 | Compound 171 | 3.65 | 812 | 8.11 | 6.98 | 140 hours |
| Example 64 | Compound 173 | 3.66 | 821 | 8.21 | 7.06 | 167 hours |
| Example 65 | Compound 177 | 3.62 | 814 | 8.14 | 7.07 | 142 hours |
| Example 66 | Compound 180 | 3.65 | 819 | 8.19 | 7.04 | 171 hours |
| Example 67 | Compound 182 | 3.66 | 821 | 8.20 | 7.04 | 148 hours |
| Example 68 | Compound 184 | 3.64 | 822 | 8.22 | 7.09 | 141 hours |
| Example 69 | Compound 188 | 3.63 | 833 | 8.32 | 7.20 | 167 hours |
| Example 70 | Compound 189 | 3.61 | 835 | 8.35 | 7.25 | 171 hours |
| Example 71 | Compound 190 | 3.63 | 832 | 8.31 | 7.19 | 151 hours |
| Example 72 | Compound 191 | 3.64 | 832 | 8.32 | 7.17 | 169 hours |
| Example 73 | Compound 192 | 3.65 | 817 | 8.16 | 7.03 | 154 hours |
| Example 74 | Compound 193 | 3.63 | 834 | 8.33 | 7.20 | 173 hours |
| Example 75 | Compound 195 | 3.65 | 819 | 8.18 | 7.04 | 165 hours |
| Example 76 | Compound 196 | 3.63 | 822 | 8.22 | 7.12 | 158 hours |
| Example 77 | Compound 198 | 3.64 | 827 | 8.27 | 7.14 | 152 hours |
| Example 78 | Compound 199 | 3.64 | 830 | 8.30 | 7.16 | 166 hours |
| Example 79 | Compound 200 | 3.65 | 822 | 8.21 | 7.07 | 149 hours |
| Example 80 | Compound 201 | 3.66 | 824 | 8.24 | 7.07 | 150 hours |
| Example 81 | Compound 203 | 3.64 | 829 | 8.28 | 7.15 | 142 hours |
| Example 82 | Compound 204 | 3.63 | 819 | 8.19 | 7.09 | 158 hours |
| Example 83 | Compound 205 | 3.65 | 825 | 8.25 | 7.11 | 163 hours |
| Example 84 | Compound 206 | 3.64 | 816 | 8.17 | 7.06 | 173 hours |
| Comparative Example 4 | Alq3 | 3.65 | 714 | 7.14 | 6.15 | 114 hours |
| Comparative Example 5 | Compound (2-1) | 3.65 | 709 | 7.08 | 6.10 | 121 hours |
| Comparative Example 6 | Compound (2-2) | 3.63 | 789 | 7.89 | 6.83 | 138 hours |

As shown in Table 3, while the drive voltage at the current density of 10 mA/cm² was substantially the same between the devices according to Comparative Example 4 to Comparative Example 6 and the devices according to Example 44 to Example 84, the luminance, light emission efficiency, power efficiency, and device lifetime of the devices according to Example 10 to Example 16 were significantly improved as compared with the devices according to Comparative Example 4 to Comparative Example 6. This shows that the amine compound according to the present invention is a material suitable for use in a capping layer and the capping layer has a high refractive index and a low extinction coefficient, which makes it possible to significantly improve the light extraction efficiency of the organic EL device.

### Industrial Applicability

The amine compound according to the present invention has a high refractive index and a low extinction coefficient, is capable of significantly improving the light extraction efficiency, is stable in a thin-film state, and thus is excellent as a compound suitable for use in an organic EL device. The organic EL device prepared using the amine compound according to the present invention is capable of achieving high efficiency. Further, the use of the amine compound according to the present invention, which has no absorption in the blue, green, and red wavelength regions, is particularly suitable when a clear and bright image with favorable color purity is desired to be displayed. For example, it has become possible to develop applications for home appliances and lighting.

### Reference Signs List

- 1: glass substrate

- 2: transparent anode
- 3: hole injection layer
- 4: hole transport layer
- 5: light-emitting layer
- 6: electron transport layer
- 7: electron injection layer
- 8: cathode
- 9: capping layer

## Claims

1. An amine compound represented by the following general formula (A). 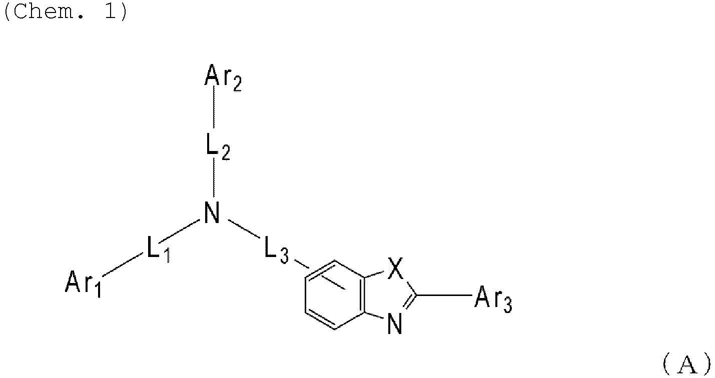 (wherein, Ar₁ represents
a substituted or unsubstituted benzoxazolyl group,
a substituted or unsubstituted benzothiazolyl group,
a substituted or unsubstituted benzofuranyl group,
a substituted or unsubstituted benzothienyl group,
a substituted or unsubstituted dibenzofuranyl group, or
a substituted or unsubstituted dibenzothienyl group,
Ar₂ and Ar₃ may be the same as or different from each other and each represent
a substituted or unsubstituted aromatic hydrocarbon group,
a substituted or unsubstituted aromatic heterocyclic group, or
a substituted or unsubstituted fused polycyclic aromatic group,
L₁ to L₃ may be the same as or different from each other and each represent
a single bond,
a divalent group obtained by removing two hydrogen atoms from unsubstituted benzene,
a divalent group obtained by removing two hydrogen atoms from unsubstituted biphenyl, or
a divalent group obtained by removing two hydrogen atoms from unsubstituted naphthalene, and
X represents an oxygen atom or a sulfur atom.

2. The amine compound according to claim 1, which is represented by the following general formula (B) or (C). 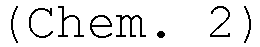 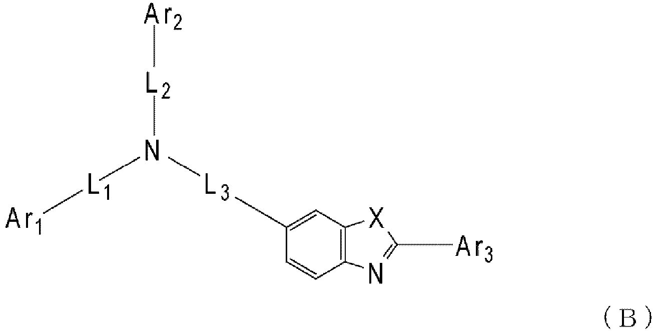 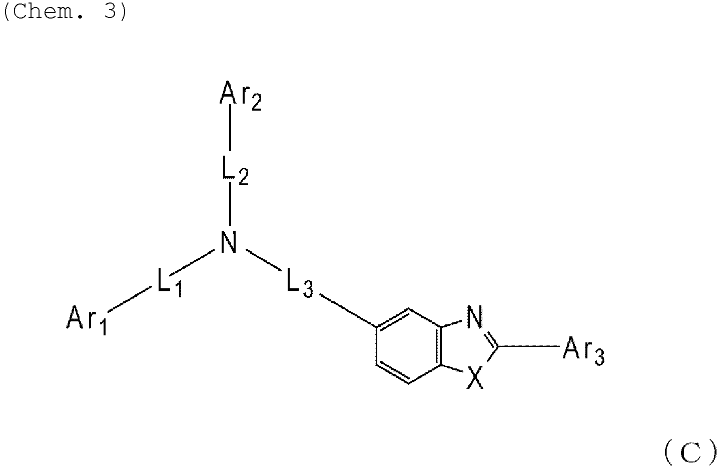
(wherein, Ar₁ to Ar₃, L₁ to L₃, and X are defined to be the same as those in the general formula (A).)

3. The amine compound according to claim 2, wherein
L₁ to L₃ in the general formula (A), (B), or (C) each represent a single bond, an unsubstituted 1,4-phenylene group, an unsubstituted 4,4'-biphenylylene group, an unsubstituted 2,6-naphthylene group, or an unsubstituted 2,7-naphthylene group.

4. The amine compound according to claim 3, wherein
Ar₃ in the general formula (A), (B), or (C) represents a substituted or unsubstituted phenyl group, a substituted or unsubstituted biphenyl group, a substituted or unsubstituted naphthyl group, a substituted or unsubstituted furanyl group, or a substituted or unsubstituted thienyl group.

5. The amine compound according to claim 4, wherein
Ar₃ in the general formula (A), (B), or (C) represents an unsubstituted phenyl group, an unsubstituted biphenyl group, an unsubstituted naphthyl group, an unsubstituted furanyl group, or an unsubstituted thienyl group.

6. The amine compound according to claim 3, wherein
Ar₁ in the general formula (A), (B), or (C) represents a substituted or unsubstituted 2-benzoxazolyl group, a substituted or unsubstituted 2-benzothiazolyl group, a substituted or unsubstituted 2-benzofuranyl group, a substituted or unsubstituted 2-benzothienyl group, a substituted or unsubstituted 2-dibenzofuranyl group, a substituted or unsubstituted 2-dibenzothienyl group, a substituted or unsubstituted 3-dibenzofuranyl group, or a substituted or unsubstituted 3-dibenzothienyl group.

7. The amine compound according to claim 6, wherein
Ar₁ in the general formula (A), (B), or (C) represents an unsubstituted 2-benzoxazolyl group, an unsubstituted 2-benzothiazolyl group, an unsubstituted 2-benzofuranyl group, an unsubstituted 2-benzothienyl group, an unsubstituted 2-dibenzofuranyl group, an unsubstituted 2-dibenzothienyl group, an unsubstituted 3-dibenzofuranyl group, or an unsubstituted 3-dibenzothienyl group.

8. The amine compound according to claim 7, wherein
Ar₃ in the general formula (A), (B), or (C) represents an unsubstituted phenyl group, an unsubstituted biphenyl group, an unsubstituted naphthyl group, an unsubstituted furanyl group, or an unsubstituted thienyl group.

9. An organic EL device, comprising:
at least an anode electrode, a hole transport layer, a light-emitting layer, an electron transport layer, a cathode electrode, and a capping layer in this order,
the capping layer including the amine compound according to claim 1 or 2.

10. The organic EL device according to claim 9, **characterized in that**
the capping layer has an extinction coefficient of 0.2 or more in a wavelength range from 400 nm to 410 nm and absorbance in an absorption spectrum at a concentration of 10⁻⁵ mol/L of 0.2 or more in the wavelength range from 400 nm to 410 nm.

11. The organic EL device according to claim 9, **characterized in that**
the capping layer has a refractive index of 1.85 or more in a wavelength range of 450 nm to 750 nm of light transmitted through the capping layer.

12. An organic EL device, comprising:
at least an anode electrode, a hole transport layer, a light-emitting layer, an electron transport layer, a cathode electrode, and a capping layer in this order,
the capping layer being a stacked layer or a mixed layer formed of two or more types of compounds, at least one of the compounds being the amine compound according to claim 1 or 2.

13. An electronic apparatus or an electronic device, comprising:
a pair of electrodes and at least one organic layer sandwiched between the pair of electrodes,
the organic layer including the amine compound according to claim 1 or 2.
